# EUROPEAN PATENT SPECIFICATION

(11) **EP 2 841 561 B1**
(45) Date of publication and mention of the grant of the patent: **16.01.2019**
(21) Application number: 13720203.2
(22) Date of filing: 24.04.2013
(51) Int. Cl.: C12N 5/00

(54) **CELL CULTURE COMPOSITIONS AND METHODS FOR POLYPEPTIDE PRODUCTION**
ZELLKULTURZUSAMMENSETZUNGEN UND VERFAHREN ZUR POLYPEPTIDHERSTELLUNG
COMPOSITIONS DE CULTURE CELLULAIRE ET PROCÉDÉS DE PRODUCTION DE POLYPEPTIDES

(30) Priority: 24.04.2012 US 201261637778 P; 24.04.2012 US 201261637780 P; 15.03.2013 US 201313841864
(43) Date of publication of application: 04.03.2015
(73) Proprietor: F. Hoffmann-La Roche AG, 4070 Basel (CH)
(72) Inventor: MEIER, Steven, J., South San Francisco, CA 94080-4990 (US); MUN, Melissa, S., South San Francisco, CA 94080-4990 (US); VIJAYASANKARAN, Natarajan, South San Francisco, CA 94080-4990 (US); VARMA, Sharat, South San Francisco, CA 94080-4990 (US); YANG, Yi, South San Francisco, CA 94080-4990 (US); ZHANG, Boyan, South San Francisco, CA 94080-4990 (US); AREVALO, Silvana, R., South San Francisco, CA 94080-4990 (US); GAWLITZEK, Martin, South San Francisco, CA 94080-4990 (US); CARVALHAL, Veronica, South San Francisco, CA 94080-4990 (US)
(74) Representative: Townsend, Carolin
(86) International application number: PCT/US2013/037992
(87) International publication number: WO 2013/163294

(56) References cited:
- EP-A2- 0 591 605
- WO-A1-98/15614
- WO-A1-2006/026445
- WO-A1-2009/014272
- WO-A1-2009/129379
- WO-A1-2010/036767
- WO-A2-2006/050050

## Description

### BACKGROUND OF THE INVENTION

Methods of producing proteins *in vitro* using recombinant cell cultures are well known and are used on an industrial scale to produce protein-based drug products. However, significant challenges remain for the efficient preparation of proteins from recombinant cell cultures. For example, a protein-based drug product has certain quality attributes, such as size distribution, sequence integrity and product color, that may be impacted by the protein's production process.

One quality attribute of particular concern is the color of a protein drug product. Regulatory requirements regarding acceptable color levels for protein-based drug products must also be met. Thus, producing a protein product that has an acceptable color (*e.g.,* to satisfy regulatory requirements for product marketing) is an important aspect of drug production. Establishing product quality comparability with earlier clinical material may be critical as well.

Recent trends towards the subcutaneous delivery of monoclonal antibodies has been accompanied by an increase in concentration of the formulated drug substance (*e.g*., to ≥ 150 mg/mL). At these concentrations, the color of the drug product can be more intense, making it more difficult to produce a protein-based drug product having an acceptable color. Cell culture conditions can impact a protein-based drug products' quality attributes. The media in which cells are cultured can have a particularly significant impact on protein production.

Recombinant DNA techniques for producing proteins *in vitro* have historically employed cell lines cultured in media supplemented with variable and chemically undefined media components such as animal sera and peptone. Chemically undefined nutrients may lead to lot-to-lot variability and animal-derived products may contaminate media with undesirable constituents. Chemically defined cell culture media ("CDM"), which are of a known composition that is consistent lot-to-lot, have been developed to addresses these concerns. Due to the various advantages associated with the use of CDM for protein production, there is an industry wide trend to move away from serum containing and peptone containing processes in favor of processes that utilize CDM. Various CDM have been described in the patent literature, such as in U.S. Patent Nos. 4,767,704; 5,691,202; 6,048,728; 6,900,056; and 7,601,535, and in U.S. Patent Application Publication Nos. 20030087372 and 20110039330. However, chemically undefined media continue to find use in protein production. EP0591605A2 is concerned with methods for suppressing coloring of human serum albumin.

There is a continuing need to provide improved and cost-effective methods of producing proteins (*e.g.,* antibodies) *in vitro* having acceptable product quality attributes. Cell culture media that modulate one or more product quality attributes are desired. Cell culture media, whether chemically undefined or chemically defined, having components that consistently deliver protein products at lower color intensities while maintaining a desired protein concentration (*e.g*., ≥ 150 mg/mL) would find use in the development of protein products, such as antibodies, *e*.*g*., for subcutaneous injection.

### BRIEF SUMMARY OF THE INVENTION

Cell culture media compositions that provide a drug product with an acceptable color are described, as are methods of using the media for cell growth (*i.e.,* cell culture) and/or protein production. In particular, the invention provides a method of producing a polypeptide comprising the step of culturing in a cell culture medium a cell comprising an isolated nucleic acid encoding the polypeptide, wherein:
a) the cell culture medium comprises:
   from about 200 mg/L to about 1200 mg/L cystine;
   from about 0.05 mg/L to about 1.0 mg/L vitamin B2;
   from about 0.05 mg/L to about 10.0 mg/L vitamin B6;
   from about 0.05 mg/L to about 12.0 mg/L vitamin B9;
   from about 0.05 mg/L to about 2.5 mg/L vitamin B12; and
   ferric citrate or ferrous sulfate;
b) the cell expresses the polypeptide; and
c) the polypeptide is isolated from the cell culture medium, wherein the isolated polypeptide is concentrated to provide a composition comprising a polypeptide at a concentration of at least 100 mg/ml and wherein the composition has a color reference standard value selected from any one of B4-B9, BY4-BY7, Y4-Y7, GY4-GY7 and R4-R7 according to the European Pharmacopoeia color standards, European Pharmacopoeia, 2008, 7th Edition, Page 22.
In one particular embodiment, the cell culture medium comprises from about 300 mg/L to about 1200mg/L cystine.
In one particular embodiment, the cell culture medium comprises: from about 0.8 mM to about 2.5 mM cystine; from about 0.11 µM to about 0.72 µM vitamin B2;
from about 4.5 µM to about 30.0 µM vitamin B6; from about 3.4 µM to about 22.0 µM vitamin B9; and from about 0.2 µM to about 1.5 µM vitamin B12.
In one particular embodiment, the cell culture medium further comprises any one or more of vitamin B1, vitamin B3, vitamin B5 and vitamin B7.
In one particular embodiment, the cell culture medium further comprises any one or more of: from about 2.0 µM to about 14.0 µM vitamin B1; from about 11.0 µM to about 72.0 µM vitamin B3; from about 6.8 µM to about 44.0 µM vitamin B5; and from about 0.02 µM to about 0.14 µM vitamin B7.
In one particular embodiment, the cell culture medium comprises ferric citrate or ferrous sulfate at a concentration of from about 2 µM to about 80 µM.
In one particular embodiment, the cell culture medium further comprises hydrocortisone, optionally at a concentration of from about 0.05 µM to about 0.25 µM.
In one particular embodiment, the polypeptide is an antibody, optionally wherein the antibody is an IgG1 antibody.
In one particular embodiment, the antibody is an anti-VEGF, anti-mesothelin, anti-PCSK9 or anti-Beta7 antibody.
In one particular embodiment, the isolated polypeptide is concentrated to provide a composition comprising a polypeptide at a concentration of at least 125 mg/ml and wherein the composition has a color reference standard value selected from any one of B4-B9, BY4-BY7, Y4-Y7, GY4-GY7 and R4-R7 according to the European Pharmacopoeia color standards, European Pharmacopoeia, 2008, 7th Edition, Page 22.
In one particular embodiment, the isolated polypeptide is concentrated to provide a composition comprising a polypeptide at a concentration of at least 150 mg/ml and wherein the composition has a color reference standard value selected from any one of B4-B9, BY4-BY7, Y4-Y7, GY4-GY7 and R4-R7 according to the European Pharmacopoeia color standards, European Pharmacopoeia, 2008, 7th Edition, Page 22.
In one particular embodiment, the color of the isolated polypeptide is determined by an assay selected from the group consisting of the COC assay, the Total Color assay, and the NIFTY assay.
Media that provide protein-based drug products with an acceptable color while maintaining a desired protein-based drug product concentration (*e.g*., ≥ 100 mg/mL or ≥ 150 mg/mL) are also described and may find use in protein production methods, such as for the production of antibodies for subcutaneous injection. The cell culture media as detailed herein may be chemically undefined or CDM. Compositions comprising a medium as described herein and a polypeptide (*e.g.,* a polypeptide secreted by a host cell into the medium) and/or a cell comprising an isolated nucleic acid encoding a polypeptide are also contemplated. Polypeptides prepared by the methods detailed herein are provided, as are formulations comprising the polypeptides and a carrier (*e.g.,* a pharmaceutically acceptable carrier). The polypeptide formulations in one aspect have an acceptable color and maintain a protein-based drug product concentration of at least 100 mg/mL or 150 mg/mL.

The cell culture media detailed herein generally comprise one or more of the following components in an amount to effect a protein product quality attribute such as color: (a) cystine or cysteine; (b) vitamin B2, (c) vitamin B6 (pyridoxine and/or pyridoxal, which may be provided as the HCl salt), (d) vitamin B9, (e) vitamin B12, (f) an iron source such as iron nitrate, ferric citrate or ferrous sulfate and (g) hydrocortisone. In one variation, a cell culture medium comprises 2 or 3 or 4 or 5 or 6 or each of components (a), (b), (c), (d), (e), (f) and (g). It is understood that a cell culture medium described herein may contain any combination of components (a), (b), (c), (d), (e), (f) and (g) the same as if each and every combination were specifically and individually listed. In one aspect, the cell culture media is a CDM. In another aspect of the disclosure, the cell culture media is chemically undefined. In a particular variation, a cell culture medium detailed herein comprises: (a) from about 300 mg/L (in some embodiments, 200 mg/L) to about 1200 mg/L cystine; (b) from about 0.05 mg/L to about 1.0 mg/L vitamin B2; (c) from about 0.05 mg/L to about 10.0 mg/L vitamin B6; (d) from about 0.05 mg/L to about 12.0 mg/L vitamin B9; and (e) from about 0.05 mg/L to about 2.5 mg/L vitamin B12, and where the cell culture medium (a) may in one variation be a CDM and/or (b) may further comprise one or more of the following components: (1) an iron source, such as ferric citrate or ferrous sulfate (which in one aspect is present at a concentration of from about 2 µM to about 80 µM), and (2) hydrocortisone (which in one aspect is present at a concentration from about 0.05 µM to about 0.25 µM). In another variation, a cell culture medium as detailed herein comprises: (a) from about 300 mg/L (in some embodiments, 200 mg/L) to about 1200 mg/L cystine; (b) from about 2 µM to about 80 µM ferric citrate; and (c) from about 0.05 µM to about 0.5 µM hydrocortisone, and where the cell culture medium (1) may in one variation be a CDM and/or (2) may further comprise one or more of the following components: (A) vitamin B2 (which in one aspect is present at a concentration of from about 0.05 mg/L to about 1.0 mg/L); (B) vitamin B6 (which in one aspect is present at a concentration of from about 0.05 mg/L to about 10.0 mg/L); (C) vitamin B9 (which in one aspect is present at a concentration of from about 0.05 mg/L to about 12.0 mg/L); and (D) vitamin B12 (which in one aspect is present at a concentration of from about 0.05 mg/L to about 2.5 mg/L). For any medium described herein, in one variation the medium reduces the presence of charge variants (which in one aspect are acidic charge variants) when used in a method of producing a polypeptide as compared to charge variants (which in one aspect are acidic charge variants) obtained when the polypeptide is produced in a different cell culture medium (e.g., a medium that does not comprise the same medium components or does contains the same medium components but in a different amount). In one variation of the compositions and methods described herein, charge variants (which in one aspect are acidic charge variants) constitute no more than 25% or 20% or 18% or 15% or 10% of the polypeptide product. In another variation of the compositions and methods described herein, at least 75% or 80% or 85% or 90% or 95% or more of the polypeptide product is a main species protein. In some variations, the main species protein is a quantitatively predominant protein as identified by the amino acid sequence, the secondary structure, and/or the tertiary structure of the protein. In some variations, the main species protein is a quantitatively predominant protein that is identified by one or more post-translational modifications. In some variations, the post-translational modification is glycosylation. It is understood that a percentage of a polypeptide product as described herein can be determined before purification of the polypeptide product, after purification of the polypeptide product, or at any step during a polypeptide purification process.

Acidic variants may be evaluated by a variety of methods, but preferably such methods include one, two, three, four, or five of: ion exchange chromatography (IEC) wherein the composition is treated with sialidase before, after, and/or during the IEC (*e.g.* to evaluate sialylated variant), reduced CE-SDS (*e.g.* to evaluate disulfide reduced variant), non-reduced CE-SDS (e.g to evaluate non-reducible variant), boronate chromatography (e.g. to evaluate glycated variant), and peptide mapping (e.g. to evaluate deamidated variant). In one variation, the overall acidic variants are evaluated by ion exchange chromatography, for example using a weak cation exchanger and/or cation exchanger with carboxylate functional group (for example, using a DIONEX PROPAC™ WCX-10 chromatography column).

In another aspect of the disclosure, the cell culture media detailed herein generally comprise one or more of the following components in an amount to effect a protein product quality attribute such as color: (a) cystine; (b) vitamin B1; (c) vitamin B2; (d) vitamin B3; (e) vitamin B5; (f) vitamin B6 (pyridoxine and/or pyridoxal, which may be provided as the HCl salt); (g) vitamin B7; (h) vitamin B9; (i) vitamin B12; and (j) an iron source such as iron nitrate, ferric citrate or ferrous sulfate. In one variation, a cell culture medium comprises 2 or 3 or 4 or 5 or 6 or 7 or 8 or 9 or each of components (a), (b), (c), (d), (e), (f), (g), (h), (i), and (j). It is understood that a cell culture medium described herein may contain any combination of components (a), (b), (c), (d), (e), (f), (g), (h), (i), and (j) the same as if each and every combination were specifically and individually listed. In one aspect, the cell culture media is a CDM. In another aspect, the cell culture media is chemically undefined. In a particular variation, a cell culture medium detailed herein comprises: (a) from about 0.8 mM (in some embodiments, 0.7 mM) to about 2.5 mM cystine; (b) from about 0.11 µM to about 0.72 µM vitamin B2; (c) from about 4.5 µM to about 30.0 µM vitamin B6; (c) from about 3.4 µM to about 22.0 µM vitamin B9; and (d) from about 0.2 µM to about 1.5 µM vitamin B12, and where the cell culture medium (a) may in one variation be a CDM and/or (b) may further comprise one or more of the following components: (1) an iron source, such as ferric citrate or ferrous sulfate (which in one aspect is present at a concentration of from about 11.0 µM to about 36.0 µM), (2) vitamin B1 (which in one aspect is present at a concentration from about 2.0 µM to about 14.0 µM), (3) vitamin B3 (which in one aspect is present at a concentration from about 11.0 µM to about 72.0 µM), (4) vitamin B5 (which in one aspect is present at a concentration from about 6.8 µM to about 44.0 µM), and (5) vitamin B7 (which in one aspect is present at a concentration from about 0.02 µM to about 0.24 µM).

Use of the cell culture media detailed herein may increase the stability (e.g., physical stability and/or chemical stability) of a protein product, such as by reducing oxidation of a main species protein, as compared to protein products produced in a cell culture medium that is of a different composition (e.g., a medium that does not comprise the media components and/or amount of components as detailed herein). Use of the cell culture media detailed herein may also reduce colored forms of a polypeptide product as compared to polypeptide products produced in a medium that is of a different composition (e.g., a medium that does not comprise the media components and/or amount of components as detailed herein). Use of the cell culture media detailed herein may also reduce binding of a polypeptide product to other substances in a cell culture vessel (e.g., adducts) as compared to polypeptide products produced in a medium that is of a different composition (e.g., a medium that does not comprise the media components and/or amount of components as detailed herein). A method of increasing stability of a polypeptide composition is contemplated, as are methods of reducing the presence and/or amount of a colored form of a polypeptide product and reducing the binding of a polypeptide product to other matters in a cell culture vessel such as adducts.

Methods of preparing a formulation comprising a polypeptide (e.g., an antibody) are also provided, comprising the steps of producing a polypeptide as provided by any method detailed herein and combining the polypeptide with one or more formulation components, such as a pharmaceutically acceptable carrier or excipient.

A formulation comprising a polypeptide (*e*.*g*., an antibody) produced by any method detailed herein are also provided. A formulation may comprise a polypeptide and a pharmaceutically acceptable carrier or excipient. A polypeptide formulation, which may be suitable for administration to an individual, may comprise an isolated and/or purified antibody and have one or more desirable product quality attributes, such as an acceptable color. In one aspect, a formulation comprising a polypeptide product obtained by any of the methods provided herein comprises the polypeptide at a concentration of at least 100 mg/mL or at least 150 mg/mL. Formulations comprising at least 100 mg/mL or at least 150 mg/mL polypeptide product (*e.g.,* an antibody, such as an IgG1 antibody) may also be of an acceptable color. Such formulations may be suitable for injection, such as subcutaneous injection into an individual, which in one aspect is a human. In some aspects, a polypeptide drug product suitable for injection is at a concentration greater than at least 100 mg/mL, at least 125 mg/mL, or at least 150 mg/mL and has a color intensity value greater than B3, B4, B5, B6, B7, B8, or B9 as measured by the COC assay. It is understood that the color intensity value as determined by the COC assay can be any one of, but not limited to, brown (B), brownish-yellow (BY), yellow (Y), greenish-yellow (GY), or red (R), wherein higher values indicate a lighter color intensity. In some aspects, a polypeptide drug product suitable for injection is at a concentration greater than at least 100 mg/mL, at least 125 mg/mL, or at least 150 mg/mL and has a color intensity value less than a color intensity value of a reference solution as measured by a color assay (*e.g.,* the Total Color assay or the NIFTY assay). Formulations as provided herein may comprise a polypeptide product where no more than 25% or 20% or 18% or 15% or 10% of the polypeptide product is a polypeptide charge variant (which in one aspect is an acidic charge variant). Formulations as detailed herein may also comprise a polypeptide product where at least 75% or 80% or 85% or 90% or 95% or more of the polypeptide product is a main species protein. In a particular variation, a formulation comprising a polypeptide (*e*.*g*., an antibody) product is provided where the formulation comprises the polypeptide product at a concentration of greater than 100 mg/mL or greater than 125 mg/mL or greater than 150 mg/mL and where the formulations is of an acceptable color and where no more than 25% or 20% or 18% or 15% or 10% of the polypeptide product is a polypeptide charge variant (which in one aspect is an acidic charge variant).

Compositions comprising the cell culture medium and one or more other components, such as a cell and/or a desired polypeptide (*e*.*g*., an antibody), are also described. The compositions encompass any and all phases of cell culture, such as seeding, cell growth, and cell production/maintenance. In one variation is described a composition comprising: (a) a cell comprising an isolated nucleic acid encoding a polypeptide; and (b) a cell culture medium as provided herein. In another variation is described a composition comprising: (a) a polypeptide; and (b) a cell culture medium as provided herein, where in one aspect the polypeptide is secreted into the medium by a cell comprising an isolated nucleic acid encoding the polypeptide or is released into the medium by lysis of a cell comprising an isolated nucleic acid encoding the polypeptide. The cell of the composition may be any cell detailed herein (*e.g.,* a CHO cell) and the cell culture medium of the composition may be any medium detailed herein, the same as if each and every combination of cell and medium where specifically and individually listed. Likewise, the polypeptide of the composition may be any polypeptide detailed herein and the medium of the composition may be any medium detailed herein, the same as if each and every combination of polypeptide and medium where specifically and individually listed.

Methods of growing cells (*i.e.,* culturing cells) by contacting the cells with a cell culture medium as detailed herein are provided. In a particular variation of a method of growing a cell (*i.e.,* culturing a cell), the cell culture medium is a CDM. In one variation, a method of growing a cell (*i.e.,* culturing a cell) comprises the step of contacting the cell with a cell culture medium comprising: (a) from about 300 mg/L (in some embodiments, 200 mg/L) to about 1200 mg/L cystine; (b) from about 0.05 mg/L to about 1.0 mg/L vitamin B2; (c) from about 0.05 mg/L to about 10.0 mg/L vitamin B6; (d) from about 0.05 mg/L to about 12.0 mg/L vitamin B9; and (e) from about 0.05 mg/L to about 2.5 mg/L vitamin B12, where the cell culture medium may further comprise one or more of the following components: (1) an iron source, such as ferric citrate or ferrous sulfate (which in one aspect is present at a concentration of from about 2 µM to about 80 µM), and (2) hydrocortisone (which in one aspect is present at a concentration of from about 0.05 µM to about 0.25 µM). In another variation, a method of growing a cell (*i.e.,* culturing a cell) is provided where the method comprises the step of contacting the cell with a cell culture medium comprising: (a) from about 300 mg/L (in some embodiments, 200 mg/L) to about 1200 mg/L cystine; (b) from about 2 µM to about 80 µM ferric citrate; and (c) from about 0.05 µM to about 0.5 µM hydrocortisone, where the cell culture medium may further comprise one or more of the following components: (1) vitamin B2 (which in one aspect is present at a concentration of from about 0.05 mg/L to about 1.0 mg/L); (2) vitamin B6 (which in one aspect is present at a concentration of from about 0.05 mg/L to about 10.0 mg/L); (3) vitamin B9 (which in one aspect is present at a concentration of from about 0.05 mg/L to about 12.0 mg/L); and (4) vitamin B12 (which in one aspect is present at a concentration of from about 0.05 mg/L to about 2.5 mg/L). In any method of growing cells (*i.e.,* culturing cells) provided herein, the cells may be contacted with the cell culture medium during the cells' growth phase and/or production phase. Contacting the cells with the medium detailed herein at any phase of cell culture is contemplated, such as during cell growth, production and maintenance. As is understood by a skilled artisan, cells are contacted with a medium as detailed herein under conditions (*e*.*g*., temperature, pH, osmolality, etc.) which promote cell maintenance and/or growth, including production of a polypeptide.

In another aspect of the disclosure, a method of growing a cell (*i.e.,* culturing a cell) comprises the step of contacting the cell with a cell culture medium comprising: (a) from about 0.8 mM (in some embodiments, 0.7 mM) to about 2.5 mM cystine; (b) from about 0.11 µM to about 0.72 µM vitamin B2; (c) from about 4.5 µM to about 30.0 µM vitamin B6; (c) from about 3.4 µM to about 22.0 µM vitamin B9; and (d) from about 0.2 µM to about 1.5 µM vitamin B12, and where the cell culture medium may further comprise one or more of the following components: (1) an iron source, such as ferric citrate or ferrous sulfate (which in one aspect is present at a concentration of from about 11.0 µM to about 36.0 µM), (2) vitamin B1 (which in one aspect is present at a concentration from about 2.0 µM to about 14.0 µM), (3) vitamin B3 (which in one aspect is present at a concentration from about 11.0 µM to about 72.0 µM), (4) vitamin B5 (which in one aspect is present at a concentration from about 6.8 µM to about 44.0 µM), and (5) vitamin B7 (which in one aspect is present at a concentration from about 0.02 µM to about 0.24 µM).

In yet another aspect of the disclosure, a method of growing a cell (*i.e.,* culturing a cell) comprises the step of contacting the cell with a cell culture medium comprising from about 0.7 mM to about 2.5 mM cystine; from about 2 µM to about 80 µM ferric citrate; from about 0.05 µM to about 0.5 µM hydrocortisone; from about 0.11 µM to about 0.72 µM vitamin B2; from about 4.5 µM to about 30.0 µM vitamin B6; from about 3.4 µM to about 22.0 µM vitamin B9; and from about 0.2 µM to about 1.5 µM vitamin B12, and where the cell culture medium may further comprise one or more of the following components: (1) vitamin B1 (which in one aspect is present at a concentration from about 2.0 µM to about 14.0 µM), (2) vitamin B3 (which in one aspect is present at a concentration from about 11.0 µM to about 72.0 µM), (3) vitamin B5 (which in one aspect is present at a concentration from about 6.8 µM to about 44.0 µM), and (4) vitamin B7 (which in one aspect is present at a concentration from about 0.02 µM to about 0.24 µM).

Methods of producing a polypeptide by growing in a cell culture medium (*i.e.,* culturing in a cell culture medium) a cell comprising an isolated nucleic acid encoding the polypeptide are also provided, wherein: (a) the cell expresses the polypeptide and (b) the cell culture medium comprises: (1) from about 300 mg/L (in some embodiments, 200 mg/L) to about 1200 mg/L cystine; (2) from about 0.05 mg/L to about 1.0 mg/L vitamin B2; (3) from about 0.05 mg/L to about 10.0 mg/L vitamin B6; (4) from about 0.05 mg/L to about 12.0 mg/L vitamin B9; and (5) from about 0.05 mg/L to about 2.5 mg/L vitamin B12, and where the cell culture medium may further comprise one or more of the following components: (A) an iron source, such as ferric citrate or ferrous sulfate (which in one aspect is present at a concentration of from about 2 µM to about 80 µM) and (B) hydrocortisone (which in one aspect is present at a concentration from about 0.05 µM to about 0.25 µM). In another variation, a method of producing a polypeptide by growing in a cell culture medium (*i.e.,* culturing in a cell culture medium) a cell comprising an isolated nucleic acid encoding the polypeptide is provided, wherein: (a) the cell expresses the polypeptide and (b) the cell culture medium comprises: (1) from about 300 mg/L (in some embodiments, 200 mg/L) to about 1200 mg/L cystine; (2) from about 2 µM to about 80 µM ferric citrate; and (3) from about 0.05 µM to about 0.5 µM hydrocortisone, where the cell culture medium may further comprise one or more of the following components: (A) vitamin B2 (which in one aspect is present at a concentration of from about 0.05 mg/L to about 1.0 mg/L); (B) vitamin B6 (which in one aspect is present at a concentration of from about 0.05 mg/L to about 10.0 mg/L); (C) vitamin B9 (which in one aspect is present at a concentration of from about 0.05 mg/L to about 12.0 mg/L); and (D) vitamin B12 (which in one aspect is present at a concentration of from about 0.05 mg/L to about 2.5 mg/L).

In another aspect of the disclosure, a method of producing a polypeptide by growing in a cell culture medium (*i.e.,* culturing in a cell culture medium) a cell comprising an isolated nucleic acid encoding the polypeptide are also provided, wherein: (a) the cell expresses the polypeptide and (b) the cell culture medium comprises: (a) from about 0.8 mM (in some embodiments, 0.7 mM) to about 2.5 mM cystine; (b) from about 0.11 µM to about 0.72 µM vitamin B2; (c) from about 4.5 µM to about 30.0 µM vitamin B6; (c) from about 3.4 µM to about 22.0 µM vitamin B9; and (d) from about 0.2 µM to about 1.5 µM vitamin B12, and where the cell culture medium may further comprise one or more of the following components: (1) an iron source, such as ferric citrate or ferrous sulfate (which in one aspect is present at a concentration of from about 11.0 µM to about 36.0 µM), (2) vitamin B1 (which in one aspect is present at a concentration from about 2.0 µM to about 14.0 µM), (3) vitamin B3 (which in one aspect is present at a concentration from about 11.0 µM to about 72.0 µM), (4) vitamin B5 (which in one aspect is present at a concentration from about 6.8 µM to about 44.0 µM), and (5) vitamin B7 (which in one aspect is present at a concentration from about 0.02 µM to about 0.24 µM).

In yet another aspect of the disclosure, a method of producing a polypeptide by growing in a cell culture medium (*i.e.,* culturing in a cell culture medium) a cell comprising an isolated nucleic acid encoding the polypeptide are also provided, wherein: (a) the cell expresses the polypeptide and (b) the cell culture medium comprises: from about 0.7 mM to about 2.5 mM cystine; from about 2 µM to about 80 µM ferric citrate; from about 0.05 µM to about 0.5 µM hydrocortisone; from about 0.11 µM to about 0.72 µM vitamin B2; from about 4.5 µM to about 30.0 µM vitamin B6; from about 3.4 µM to about 22.0 µM vitamin B9; and from about 0.2 µM to about 1.5 µM vitamin B12, and where the cell culture medium may further comprise one or more of the following components: (1) vitamin B1 (which in one aspect is present at a concentration from about 2.0 µM to about 14.0 µM), (2) vitamin B3 (which in one aspect is present at a concentration from about 11.0 µM to about 72.0 µM), (3) vitamin B5 (which in one aspect is present at a concentration from about 6.8 µM to about 44.0 µM), and (4) vitamin B7 (which in one aspect is present at a concentration from about 0.02 µM to about 0.24 µM).

The polypeptide produced by a method or present in a composition described herein may in one variation be an antibody, such as an IgG1 antibody. In one aspect, the polypeptide produced by a method or present in a composition described herein is an anti-VEGF, anti-mesothelin, anti-PCSK9 or anti-Beta7 antibody. Polypeptides produced according to the methods provided herein or present in the described compositions may be isolated from the cell culture medium and may be further purified. Polypeptides (such as antibodies) may also be concentrated to achieve a desirable concentration. Methods of concentrating polypeptides are known in the field, for example, the concentration of a polypeptide or protein may be increased by using ultrafiltration. In a particular variation, the polypeptide is isolated at a concentration of at least 100 mg/mL or 150 mg/mL and/or appears as a colorless or slightly colored liquid. In a particular variation, a composition comprises the isolated polypeptide at a concentration of at least 100 mg/mL and/or appears as a colorless or slightly colored liquid. In another variation, a composition comprises the isolated polypeptide at a concentration of at least 1 mg/mL or 10 mg/mL or 50 mg/mL or 75 mg/mL and/or appears as a colorless or slightly colored liquid. In another variation, a composition comprises the isolated polypeptide at a concentration of at least about any one of 1 mg/mL or 10 mg/mL or 50 mg/mL or 75 mg/mL and/or appears as a colorless or slightly colored liquid. In another variation, a composition comprises the isolated polypeptide at a concentration of at least about any one of 1 mg/mL or 10 mg/mL or 50 mg/mL or 75 mg/mL to about 125 mg/mL or to about 150 mg/mL and/or appears as a colorless or slightly colored liquid. Also described is a composition comprising a polypeptide obtained by a method detailed herein and a pharmaceutically acceptable carrier.

A kit for supplementing a cell culture medium with chemically defined constituents is also described, the kit comprising: (a) cystine in an amount to provide from about 300 mg/L (in some embodiments, 200 mg/L) to about 1200 mg/L cystine in the cell culture medium; (b) vitamin B2 in an amount to provide from about 0.05 mg/L to about 1.0 mg/L vitamin B2 in the cell culture medium; (c) vitamin B6 in an amount to provide from about 0.05 mg/L to about 10.0 mg/L vitamin B6 in the cell culture medium; (d) vitamin B9 in an amount to provide from about 0.05 mg/L to about 12.0 mg/L vitamin B9 in the cell culture medium; and (e) vitamin B12 in an amount to provide from about 0.05 mg/L to about 2.5 mg/L vitamin B12 in the cell culture medium, where the kit may further comprise one or more of the following components (1) an iron source, such as ferric citrate or ferrous sulfate (which in one aspect is in an amount to provide the iron source at a concentration of from about 2 µM to about 80 µM) and (2) hydrocortisone (which in one aspect is in an amount to provide hydrocortisone at a concentration from about 0.05 µM to about 0.25 µM). In another variation, a kit for supplementing a cell culture medium with chemically defined constituents is described, the kit comprising: (a) cystine in an amount to provide from about 300 mg/L (in some embodiments, 200 mg/L) to about 1200 mg/L cystine in the cell culture medium; (b) ferric citrate in an amount to provide a concentration of from about 2 µM to about 80 µM ferric citrate in the cell culture medium; and (c) hydrocortisone in an amount to provide a concentration of from about 0.05 µM to about 0.5 µM hydrocortisone in the cell growth (*i.e.,* cell culture) medium, where the kit may further comprise one or more of the following components: (1) vitamin B2 (which in one aspect is in an amount to provide from about 0.05 mg/L to about 1.0 mg/L of vitamin B2 in the cell culture medium); (2) vitamin B6 (which in one aspect is in an amount to provide from about 0.05 mg/L to about 10.0 mg/L of vitamin B6 in the cell culture medium); (3) vitamin B9 (which in one aspect is in an amount to provide from about 0.05 mg/L to about 12.0 mg/L of vitamin B9 in the cell culture medium); and (4) vitamin B12 (which in one aspect is in an amount to provide from about 0.05 mg/L to about 2.5 mg/L of vitamin B12 in the cell culture medium). Also provide herein is a kit for supplementing a cell culture medium with chemically defined constituents, wherein the kit comprises: (a) cystine in an amount to provide from about 0.8 mM (in some embodiments, 0.7 mM) to about 2.5 mM cystine in the cell culture medium; (b) vitamin B2 in an amount to provide from about 0.11 µM to about 0.72 µM vitamin B2 in the cell culture medium; (c) vitamin B6 in an amount to provide from about 4.5 µM to about 30.0 µM vitamin B6 in the cell culture medium; (d) vitamin B9 in an amount to provide from about 3.4 µM to about 22.0 µM vitamin B9 in the cell culture medium and (e) vitamin B12 in an amount to provide from about 0.2 µM to about 1.5 µM vitamin B12 in the cell culture medium, and where the kit may further comprise one or more of the following components: (1) an iron source, such as ferric citrate or ferrous sulfate (which in one aspect is in an amount to provide from about 11.0 µM to about 36.0 µM in the cell culture medium), (2) vitamin B1 (which in one aspect is in an amount to provide from about 2.0 µM to about 14.0 µM in a cell culture medium), (3) vitamin B3 (which in one aspect is in an amount to provide from about 11.0 µM to about 72.0 µM in a cell culture medium), (4) vitamin B5 (which in one aspect is in an amount to provide from about 6.8 µM to about 44.0 µM in a cell culture medium), and (5) vitamin B7 (which in one aspect is in an amount to provide from about 0.02 µM to about 0.24 µM in a cell culture medium). Kits may also comprise instructions for use, such as instructions for preparing a medium (e.g., a CDM) and/or for producing polypeptides (including antibodies) from a cell culture system.

### BRIEF DESCRIPTION OF THE DRAWINGS

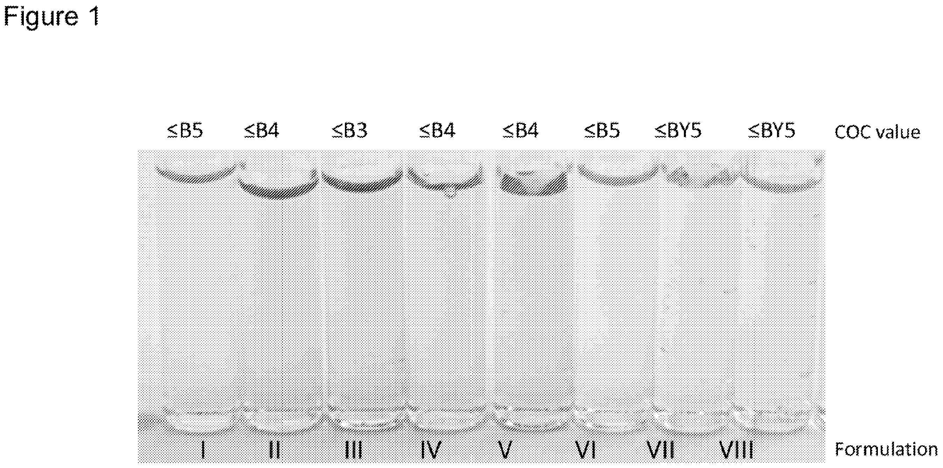

**Figure** 1 is a picture demonstrating the COC assay for antibody samples isolated from cell lines cultured under various cell culture conditions. From left to right the vials contain Formulations with antibodies isolated from cells cultured in: I) chemically undefined media; II) basal Media 1 and feed Media 2; III) basal Media 1 and feed Media 2; IV) basal Media 5 and feed Media 4; V) basal Media 5 and feed Media 2; VI) modified basal Media 3 containing cysteine instead of cystine and feed Media 4; VII) basal Media 3 and feed Media 4; and VIII) modified basal Media 3 containing cysteine instead of cystine and feed Media 4. COC values are depicted above the vials. All vials contain approximately 150 g/L protein. Formulation III, IV, and VI had a color intensity value of 1.59, 1.47, and 0.71, respectively, as measured by the NIFTY assay. Formulation III, IV, and VI had a color intensity value of 2.62, 2.04, and 1.00, respectively, as measured by the Total Color assay.

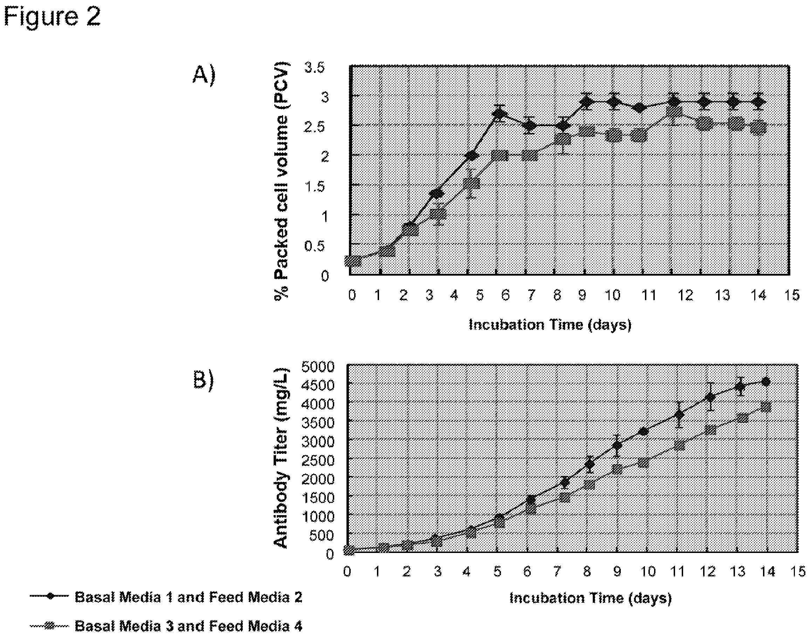

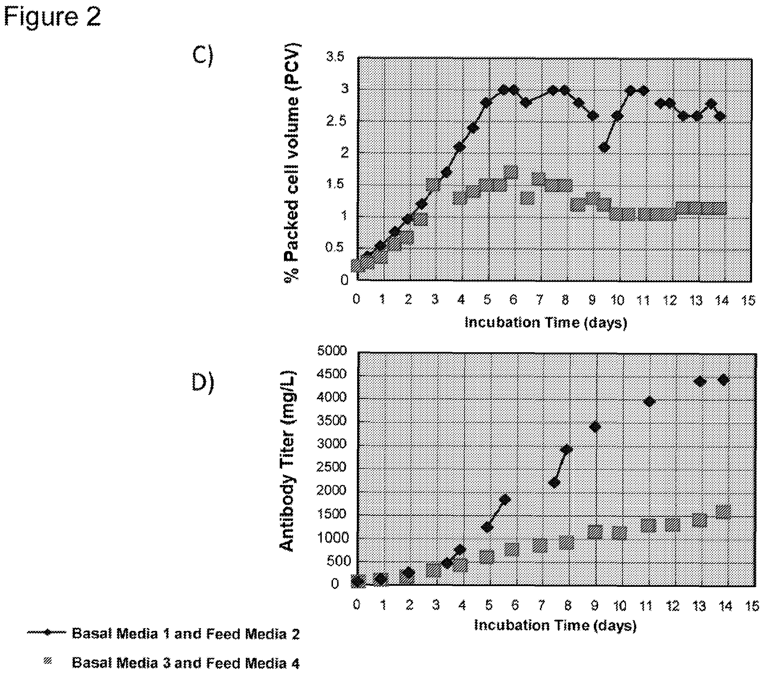

**Figure 2** is a series of graphs showing slight reduction of cell number and antibody production in cell cultures incubated with basal Media 3 and feed Media 4 as compared to basal Media 1 and feed Media 2. **A** and **C)** cell number in culture over the duration of incubation as measured by packed cell volume (PCV) expressed as percent of the total culture volume. **B** and **D)** antibody production in cell culture over the duration of incubation as measured by high performance liquid chromatography and expressed as antibody titer.

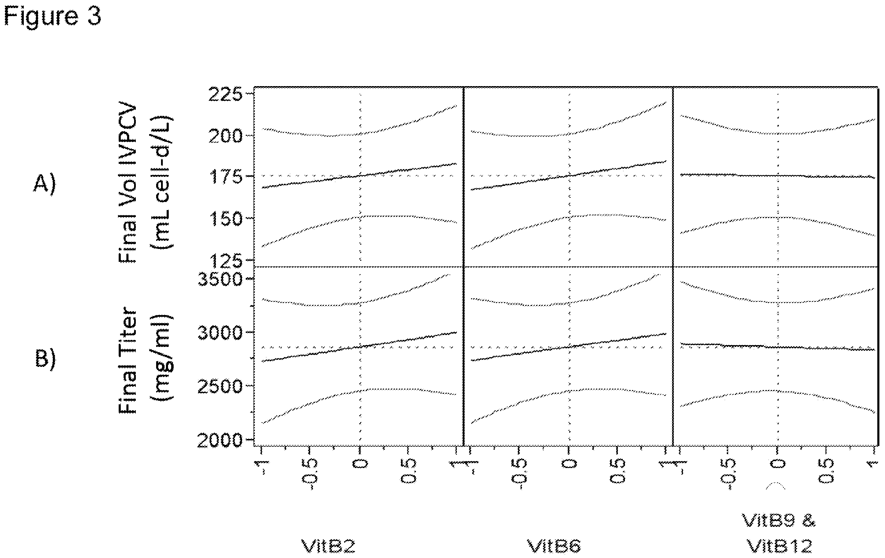

**Figure 3** is a series of graphs demonstrating the effect on productive cell biomass and antibody production by cell cultures grown in chemically defined media (CDM) containing varying levels of vitamin B2, vitamin B6, and vitamin B9 together with vitamin B12. A) productive biomass in culture as measured by packed cell volume (PCV) expressed as percent of the total culture volume. **B)** antibody production from cells as measured by high performance liquid chromatography and expressed as antibody titer. For vitamin B2, -1 indicates 0.25 mg/L basal with 0 mg/L feed and 1 indicates 1.41 mg/L with 10 mg/L feed; for vitamin B6, -1 indicates 5.35 mg/L basal (pyridoxine) with 0 mg/L feed and 1 indicates pyridoxine at 15.42 mg/L basal with 7 mg/L feed in combination with pyridoxal at 0 mg/L basal with 60 mg/L feed; for vitamin B9, -1 indicates 8.61 mg/L basal with 0 mg/L feed and 1 indicates 9.93 mg/L basal with 197 mg/L feed; for vitamin B12, -1 indicates 1.76 mg/L basal with 0 mg/L feed and 1 indicates 3.05 mg/L basal with 48 mg/L feed. Middle line indicates the predicted value calculated from a linear model that is derived from the data. Upper and lower lines indicate 95% confidence interval of the prediction.

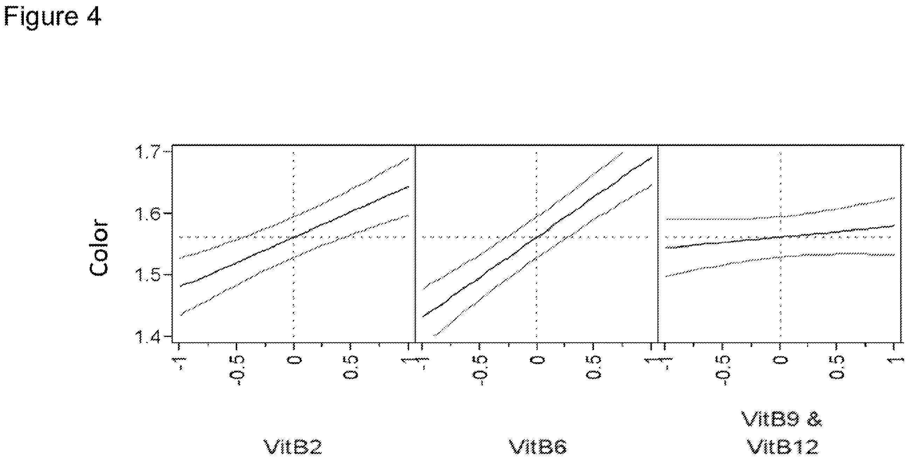

**Figure 4** is a series of graphs showing color intensity of antibodies isolated from cell cultures grown in CDM containing varying levels of vitamin B2, vitamin B6, and vitamin B9 together with vitamin B12. Color intensity was determined with a color assay wherein higher numerical values indicate higher color intensity and lower numerical values indicate lower color intensity. For vitamin B2, -1 indicates 0.25 mg/L basal with 0 mg/L feed and 1 indicates 1.41 mg/L basal with 10 mg/L feed; for vitamin B6, -1 indicates 5.35 mg/L basal (pyridoxine) with 0 mg/L feed and 1 indicates pyridoxine at 15.42 mg/L basal with 7 mg/L feed in combination with pyridoxal at 0 mg/L basal with 60 mg/L feed; for vitamin B9, -1 indicates 8.61 mg/L basal with 0 mg/L feed and 1 indicates 9.93 mg/L basal with 197 mg/L feed; for vitamin B12, -1 indicates 1.76 mg/L basal with 0 mg/L feed and 1 indicates 3.05 mg/L basal with 48 mg/L feed.

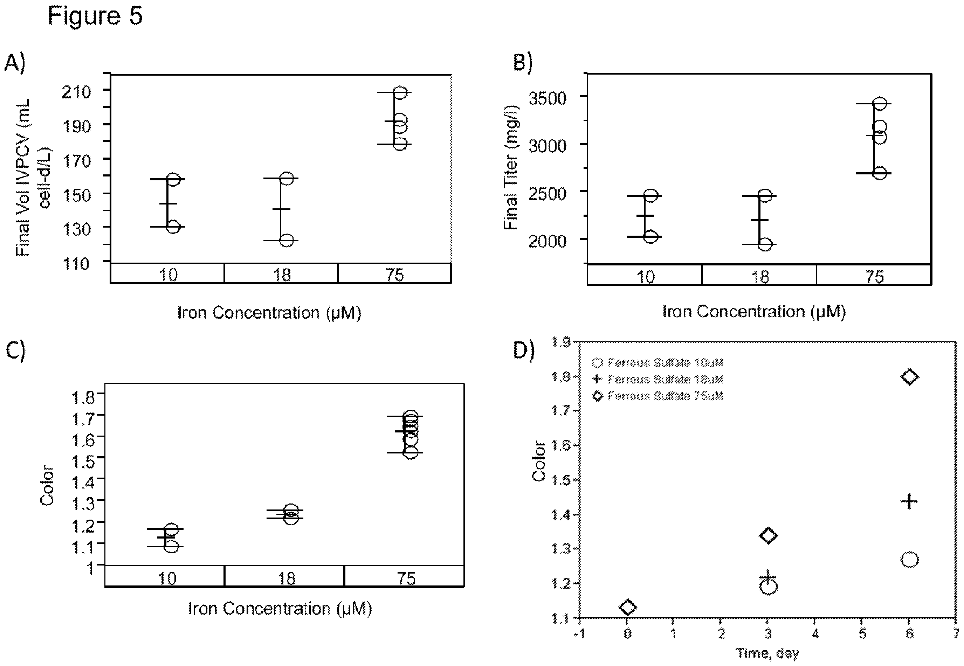

**Figure 5** A-C) is a series of graphs showing productive cell biomass, antibody production, and color intensity of antibodies isolated from cell cultures grown in CDM containing increasing concentrations of ferrous sulfate. **A)** productive biomass in culture as measured by packed cell volume over time (IVPCV). **B)** antibody production from cells as measured by high performance liquid chromatography. **C)** color intensity of antibodies isolated from cells as measured by a color assay wherein higher numerical values indicate higher color intensity and lower numerical values indicate lower color intensity. Bars indicate upper and lower extent of data and the mean. **D)** is a graph showing color intensity of antibodies incubated in media containing increasing concentrations of ferrous sulfate in an *in vitro* experiment.

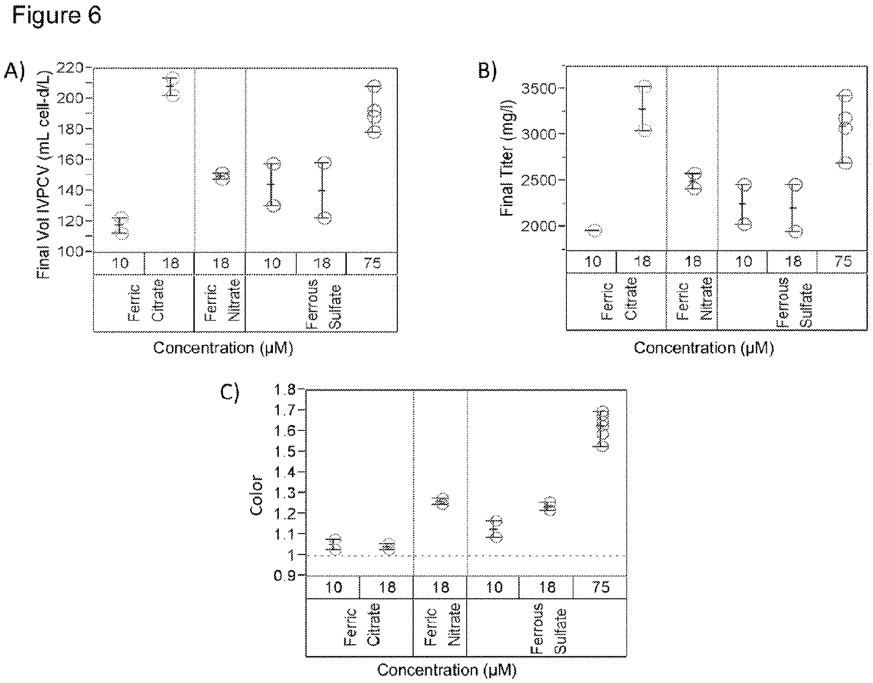

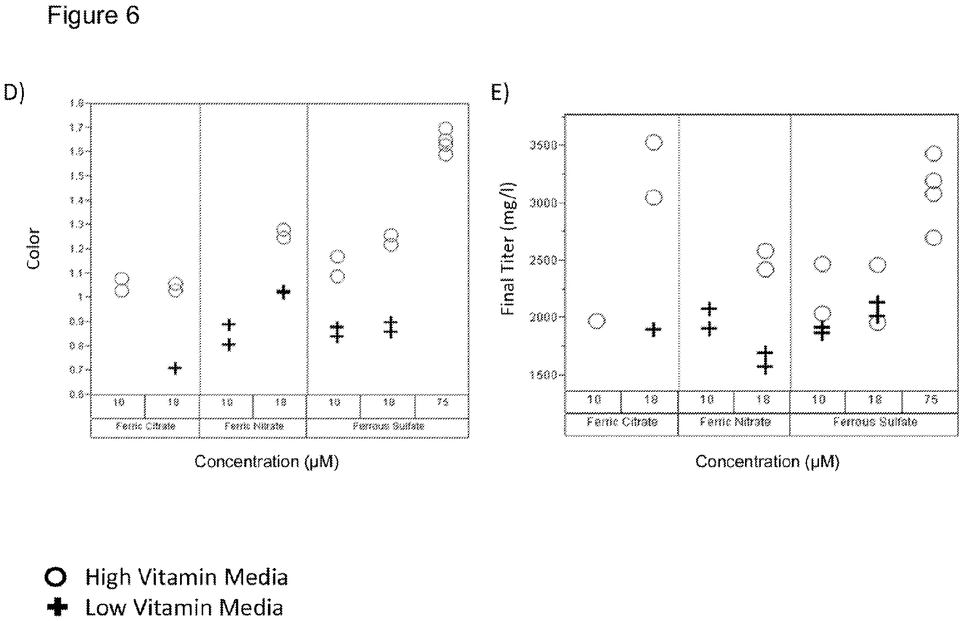

**Figure 6** is a series of graphs showing productive cell biomass, antibody production, and color intensity of antibodies isolated from cell cultures grown in CDM containing increasing concentrations of iron and with varying iron sources. **A)** productive biomass in culture as measured by packed cell volume over time (IVPCV). **B)** antibody production from cells as measured by high performance liquid chromatography. **C)** color intensity of antibodies isolated from cells as measured by a color assay wherein higher numerical values indicate higher color intensity and lower numerical values indicate lower color intensity. Bars indicate upper and lower extent of data and the mean. **D-E)** shows antibody production and color intensity of antibodies isolated from cell cultures grown in CDM containing varying concentrations of different iron sources and reduced vitamin B levels. Plus signs indicate low vitamin conditions and empty spheres indicate high vitamin conditions. **D)** antibody production from cells as measured by high performance liquid chromatography. **E)** color intensity of antibodies isolated from cells as measured by the NIFTY assay wherein higher numerical values indicate higher color intensity and lower numerical values indicate lower color intensity.

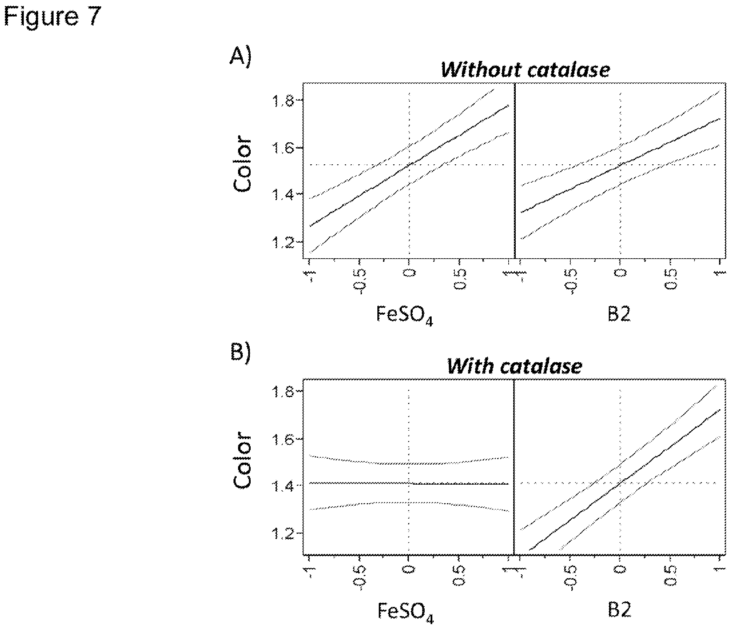

**Figure** 7 is a series of graphs showing color intensity of antibodies incubated in CDM containing varying concentrations of ferrous sulfate or vitamin B2 in the absence or presence of catalase in an *in vitro* experiment. **A)** color intensity of antibodies in the absence of catalase. **B)** color intensity of antibodies in the presence of catalase. Color intensity as measured by a color assay wherein higher numerical values indicate higher color intensity and lower numerical values indicate lower color intensity. For ferrous sulfate, -1 indicates 18 µM basal with 0 µM feed and 1 indicates 75 µM basal with 0 µM feed; for vitamin B2, -1 indicates 0.25 mg/L basal with 0 mg/L feed and 1 indicates 1.41 mg/L with 10 mg/L feed. Middle line indicates the predicted value calculated from a linear model that is derived from the data. Upper and lower lines indicate 95% confidence interval of the prediction.

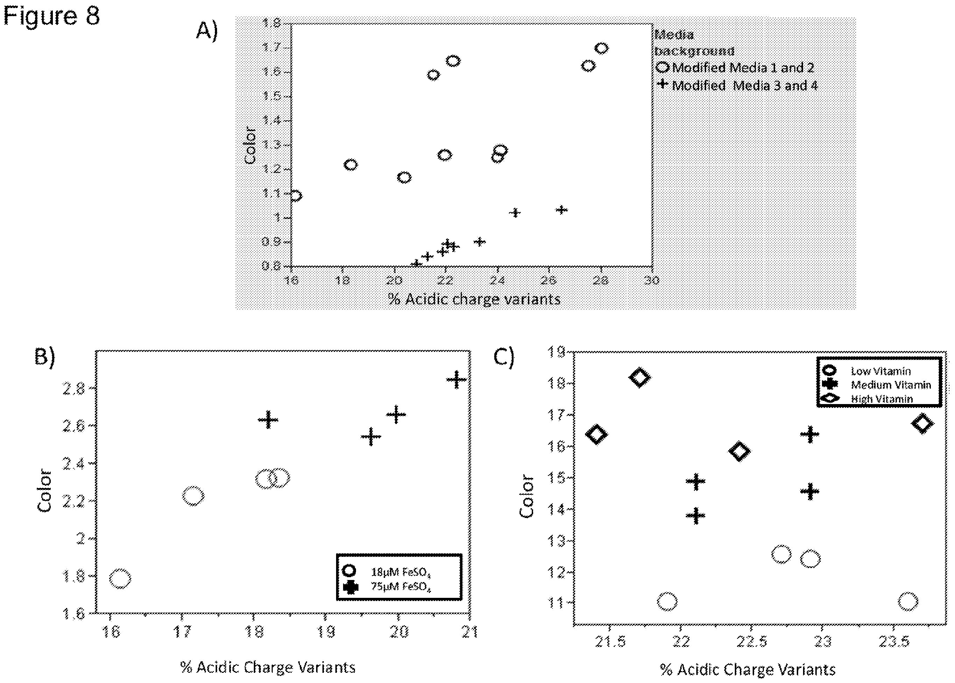

**Figure 8** is **A)** a graph showing a correlation between reduced color intensity and reduced presence of acidic charge variants in antibody solutions obtained from cell cultures grown in modified basal Media 3 and feed Media 4 (plus sign) as compared to modified basal Media 1 and feed Media 2 (empty sphere). Modified Media 1 contained 10 µM, 18 µM or 75 µM ferrous sulfate. Modified Media 3 contained 10 µM or 18 µM ferric citrate. **B)** a graph showing a correlation between reduced color intensity and reduced presence of acidic charge variants in antibody solutions obtained from cell cultures grown in media containing 18 µM ferrous sulfate (empty sphere) as compared to 75 µM ferrous sulfate (plus sign). **C)** a graph showing no correlation between reduced color intensity and reduced presence of acidic charge variants in antibody solutions obtained from cell cultures grown in media containing varying levels of Vitamin B2, B6, B9, and B12. Vitamin B levels were varied simultaneously to a low concentration (empty sphere), medium concentration (plus sign), or a high concentration (empty diamond). Color intensity as measured by a color assay wherein higher numerical values indicate higher color intensity and lower numerical values indicate lower color intensity. Percent of acidic charge variants in antibody solutions was determined by ion exchange chromatography.

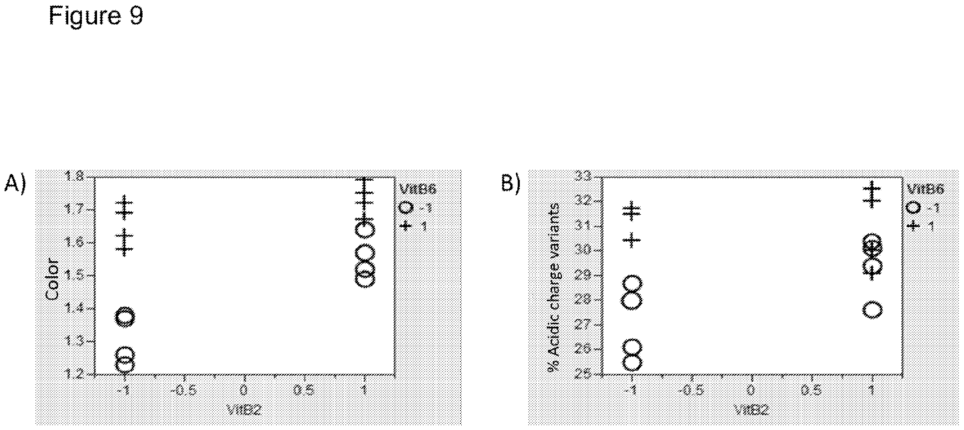

**Figure 9** is a series of graphs showing a correlation between reduced color intensity and reduced presence of acidic charge variants in antibody solutions obtained from cell cultures grown in media containing reduced concentrations of vitamin B2 and vitamin B6. **A)** color intensity of antibodies isolated from cells as measured by a color assay wherein higher numerical values indicate higher color intensity and lower numerical values indicate lower color intensity. **B)** percent acidic charge variants in antibody solutions as determined by ion exchange chromatography. For vitamin B2, -1 indicates 0.25 mg/L basal with 0 mg/L feed and 1 indicates 1.41 mg/L basal with 10 mg/L feed; for vitamin B6, -1 indicates 5.35 mg/L basal (pyridoxine) with 0 mg/L feed and 1 indicates pyridoxine at 15.42 mg/L basal with 7 mg/L feed in combination with pyridoxal at 0 mg/L basal with 60 mg/L feed.

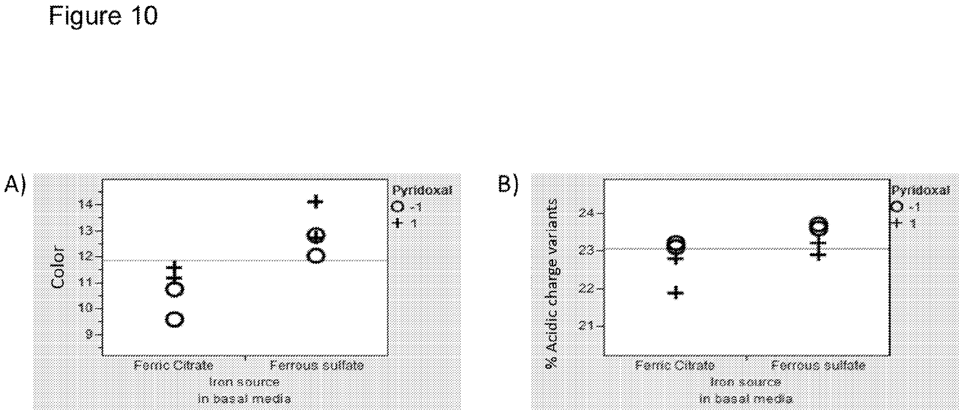

**Figure 10** is a series of graphs showing reduced color intensity and reduced presence of acidic charge variants in antibody solutions obtained from cell cultures grown in basal media containing ferric citrate versus ferrous sulfate with varying concentrations of pyridoxal. **A)** color intensity of antibodies isolated from cells as measured by a color assay wherein higher numerical values indicate higher color intensity and lower numerical values indicate lower color intensity. **B)** percent acidic charge variants in antibody solutions as measured by ion exchange chromatography. For pyridoxal, -1 indicates 0 mg/L basal with 0 mg/L feed and 1 indicates 0 mg/L basal with 60 mg/L feed. Ferric citrate or ferrous sulfate was present as 18 µM in basal media.

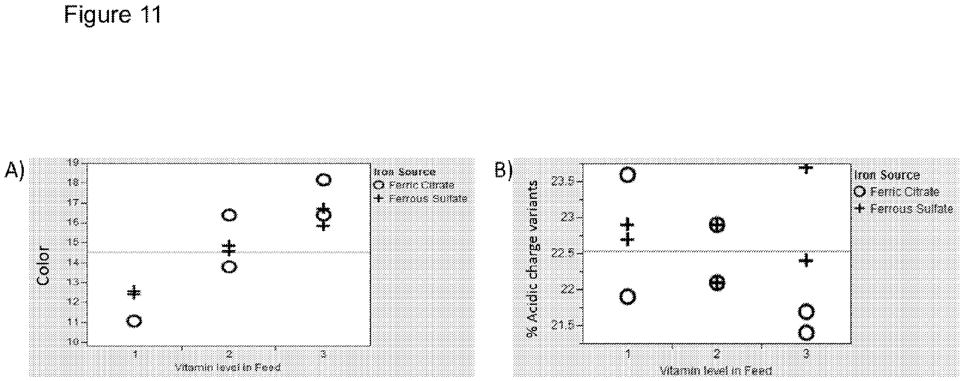

**Figure 11** is a series of graphs showing reduced color intensity and levels of acidic charge variants in antibody solutions obtained from cell cultures grown in basal media containing ferric citrate versus ferrous sulfate with varying concentrations of vitamin B2, B6, B9, and B12 in feed media. **A)** color intensity of antibodies isolated from cells as measured by a color assay wherein higher numerical values indicate higher color intensity and lower numerical values indicate lower color intensity. **B)** percent acidic charge variants in antibody solutions as measured by ion exchange chromatography. Level 1 indicates fed media free of vitamin B2, B6, B9 and B12. Level 2 indicates feed media containing 10 mg/L vitamin B2, 7 mg/L pyridoxine, 60 mg/L pyridoxal, 197 mg/L vitamin B9, and 48 mg/L vitamin B12. Level 3 indicates feed media containing 5 mg/L vitamin B2, 3.5 mg/L pyridoxine, 30 mg/L pyridoxal, 98.5 mg/L vitamin B9, and 24 mg/L vitamin B12. Ferric citrate or ferrous sulfate was present as 18 µM in basal media.

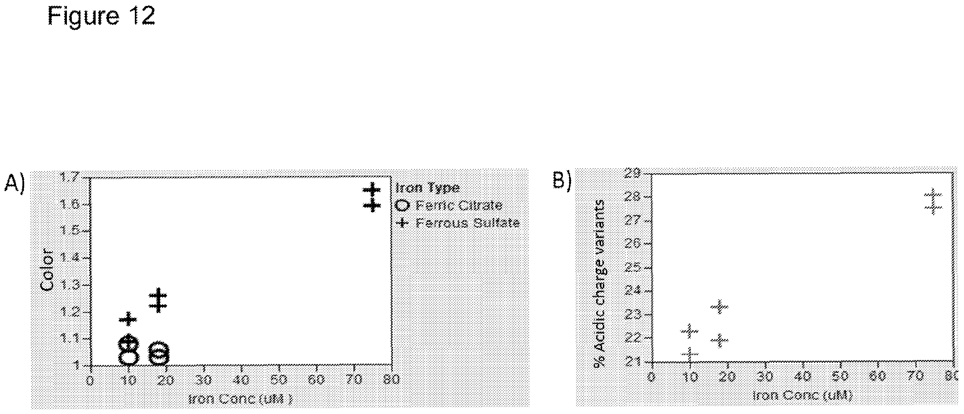

**Figure 12** is a series of graphs showing reduced color intensity and reduced levels of acidic charge variants in antibody solutions obtained from cell cultures grown in basal media containing reduced concentration of iron and preferably with ferric citrate instead of ferrous sulfate as the iron source. **A)** color intensity of antibodies isolated from cells as measured by a color assay wherein higher numerical values indicate higher color intensity and lower numerical values indicate lower color intensity. **B)** percent acidic charge variants in antibody solutions as measured by ion exchange chromatography.

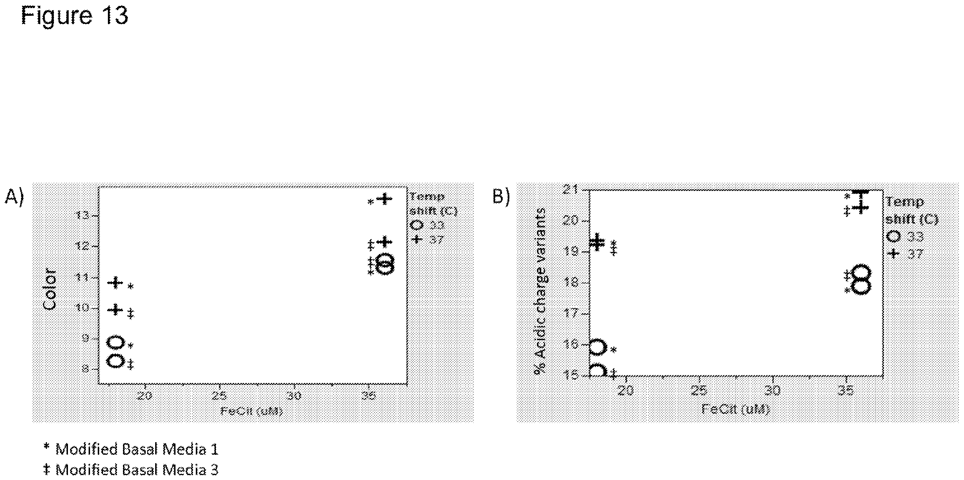

**Figure 13** is a series of graphs showing reduced color intensity and reduced levels of acidic charge variants in antibody solutions obtained from cell cultures grown in basal media containing reduced concentrations of ferric citrate. **A)** color intensity of antibodies isolated from cells as measured by a color assay wherein higher numerical values indicate higher color intensity and lower numerical values indicate lower color intensity. **B)** percent acidic charge variants in antibody solutions as measured by ion exchange chromatography. * indicates basal Media 1 modified to contain ferric citrate at the indicated concentrations. ‡ indicates basal Media 3 modified to contain ferric citrate at the indicated concentrations. _{○} indicates antibody solutions isolated from cells cultured at 33°C. + indicates antibody solutions isolated from cells cultured at 37°C.

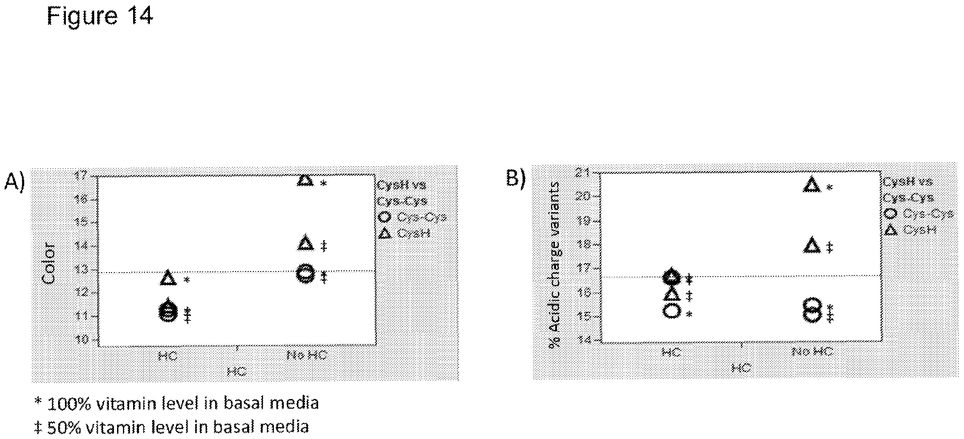

**Figure 14** is a series of graphs showing reduced color intensity and reduced levels of acidic charge variants in antibody solutions obtained from cell cultures grown in basal media containing reduced concentrations of vitamin B2, B6, B9, and B12 with addition of cystine instead of cysteine and in the presence of hydrocortisone. **A)** color intensity of antibodies isolated from cells as measured by a color assay wherein higher numerical values indicate higher color intensity and lower numerical values indicate lower color intensity. **B)** percent acidic charge variants in antibody solutions as measured by ion exchange chromatography. * indicates basal media containing 1.41 mg/L vitamin B2, 15.42 mg/L pyridoxine, 0 mg/L pyridoxal, 9.93 mg/L vitamin B9, and 3.05 mg/L vitamin B12. ‡ indicates basal media containing 0.7 mg/L vitamin B2, 7.7 mg/L pyridoxine, 0 mg/L pyridoxal, 4.9 mg/L vitamin B9, and 1.5 mg/L vitamin B12. ○ indicates 480 mg/L cystine. Δ indicates 525 mg/L cysteine. Hydrocortisone is present at 150 nM in indicated solutions.

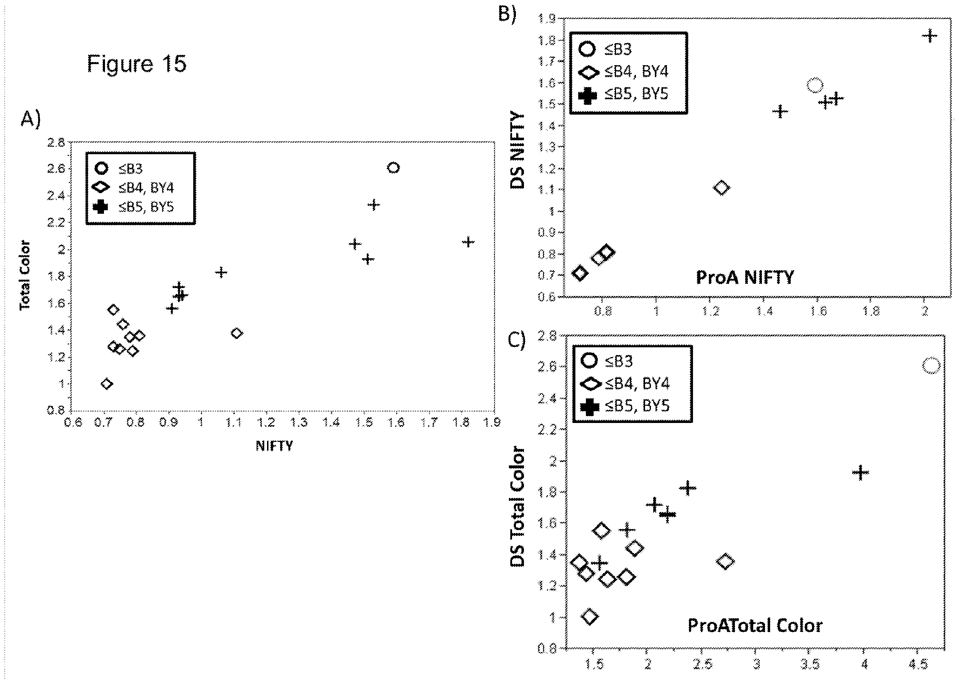

**Figure 15** is a series of graphs showing the correlation between the Total Color assay and NIFTY assay measurements as compared to COC assay measurements for color intensity. **A)** Total Color and NIFTY values were plotted with the symbols representing the COC values. **B)** The color measurements by the NIFTY assay in the protein A pool and in the corresponding drug substance formulation were plotted with symbols representing the COC values. **C)** The color measurements by the Total Color assay in the protein A pool and in the corresponding drug substance formulation were plotted with symbols representing the COC values. Empty spheres represent a COC value of ≤B3; empty diamonds represent a COC value of ≤B4 or BY4; plus signs represent a COC value of ≤B5 or BY5; DS NIFTY and DS Total Color indicate the corresponding assay values in the final drug substance formulation; and ProA NIFTY and ProA Total Color indicate the corresponding assay values in the protein A pool.

### DETAILED DESCRIPTION OF THE INVENTION

Cell culture media and methods of using the media for cell growth (*i.e.,* cell culture) and polypeptide production are described. Polypeptides, including antibodies, produced by the described methods are also provided. Kits for preparing the media and compositions comprising the media are also described, as are compositions comprising a cell and/or polypeptide produced by the described methods. Pharmaceutical compositions comprising a polypeptide at a concentration greater than at least 100 mg/mL, at least 125 mg/mL, or at least 150 mg/mL and having a color intensity value greater than B3, B4, B5, B6, B7, B8, or B9 as measured by the Color, Opalescence and Coloration (COC) assay are described. Pharmaceutical compositions comprising a polypeptide at a concentration greater than at least 1 mg/mL, at least 10 mg/mL, at least 25 mg/mL, at least 50 mg/mL, or at least 75 mg/mL and having a color intensity value greater than B3, B4, B5, B6, B7, B8, or B9 as measured by the COC assay are also described. It is understood that the reference standards for the COC assay can be any one of, but not limited to, B, BY, Y, GY, or R, wherein higher values indicate a lighter color intensity. Also provided are pharmaceutical compositions comprising a polypeptide at a concentration greater than at least 100 mg/mL, at least 125 mg/mL, or at least 150 mg/mL and having a color intensity value less than a color intensity value of a reference solution as measured by a color assay (*e.g.,* the Total Color assay or the NIFTY assay). Provided also herein are pharmaceutical compositions comprising a polypeptide at a concentration greater than at least 1 mg/mL, at least 10 mg/mL, at least 25 mg/mL, at least 50 mg/mL, or at least 75 mg/mL and having a color intensity value less than a color intensity value of a reference solution as measured by a color assay (*e.g.,* the Total Color assay or the NIFTY assay). Also provided are methods of assessing color in a polypeptide-containing solution (*e*.*g*., an antibody-containing solution). In particular variations, the media, method, kits and compositions comprise a CDM.

Certain cell culture media (*e.g.,* CDM) used in polypeptide production have been found to provide a polypeptide drug product with acceptable quality attributes. For example, certain CDM have been found to modulate the color intensity of a polypeptide drug product, with polypeptides produced in the CDM providing acceptable color (*e.g.,* for use as an injectable drug product) while maintaining the benefits associated with the use of CDM. These CDM, when used in a method of polypeptide production, have been found to decrease the color intensity of a polypeptide drug product as compared to the polypeptide produced in a different medium (*e*.*g*., a medium that does not comprise the media components and/or amount of components as detailed herein). The cell culture media may be chemically defined or chemically undefined.

Without wishing to be bound by theory, it is believed that the use of particular medium components (such as cystine and/or cysteine, certain B vitamins, hydrocortisone and/or an iron source) at certain concentrations produce a polypeptide drug product with acceptable quality attributes, and with an acceptable color in particular. Although it is believed that use of these medium components in the basal medium used for cell growth is particularly influential on a polypeptide drug product's quality attributes, it is contemplated that the media as provided herein may be employed at any stage of the cell growth, maintenance and polypeptide production process, including in the basal and the feed media. The media provided herein can be used in methods of growing a cell (*i.e.,* culturing a cell) and in producing a polypeptide, and may find use in the growth, maintenance and/or production phase of a cell comprising an isolated nucleic acid encoding a desired polypeptide. The media described herein may be employed to yield improvements in one or more properties of a polypeptide drug product (*e.g.,* color, composition, purity, etc.) or one or more aspects of a method of producing the polypeptide (*e*.*g*., batch-to-batch reproducibility, ease of manufacture, cost of manufacture, etc.). Polypeptides (*e*.*g*., an antibody) produced by a cell culture process employing the medium provided herein are described. In one aspect, the polypeptide is at a concentration greater than at least 100 mg/mL, at least 125 mg/mL, or at least 150 mg/mL and with a color intensity value greater than B3, B4, B5, B6, B7, B8, or B9 as measured by the COC assay. In another aspect, the polypeptide is at a concentration greater than at least 1 mg/mL, at least 10 mg/mL, at least 25 mg/mL, at least 50 mg/mL, or at least 75 mg/mL and with a color intensity value greater than B3, B4, B5, B6, B7, B8, or B9 as measured by the COC assay. In some aspects, the color intensity value as determined by the COC assay can be any one of, but not limited to, B, BY, Y, GY, or R, wherein higher values indicate a lighter color intensity. Methods of administering polypeptide products detailed herein are also described, as are articles of manufacture comprising the polypeptide products as produced herein.

### Definitions

For use herein, unless clearly indicated otherwise, use of the terms "a", "an," and the like refers to one or more.

Reference to "about" a value or parameter herein includes (and describes) embodiments that are directed to that value or parameter per se. For example, description referring to "about X" includes description of "X." Numeric ranges are inclusive of the numbers defining the range.

A "charge variant" is a variant of the main species protein (*e*.*g*., antibody) which has a different charge than that of the main species protein.

An "acidic charge variant" is a variant of the main species protein (*e*.*g*., antibody) which is more acidic than the main species protein (*e*.*g*., antibody). An acidic variant has gained negative charge or lost positive charge relative to the main species protein (*e.g.,* antibody). Such acidic charge variants can be resolved using a separation methodology, such as ion exchange chromatography, that separates proteins according to charge.

The term "main species protein" herein refers to the protein (*e.g.,* antibody) amino acid sequence structure in a composition which is the quantitatively predominant protein (*e*.*g*., antibody) molecule in the composition.

"Culturing" a cell refers to contacting a cell with a cell culture medium under conditions suitable to the survival and/or growth of the cell.

"Batch culture" refers to a culture in which all components for cell culturing (including the cells and all culture nutrients) are supplied to the culturing vessel at the start of the culturing process.

The phrase "fed batch cell culture," as used herein refers to a batch culture wherein the cells and culture medium are supplied to the culturing vessel initially, and additional culture nutrients are fed, continuously or in discrete increments, to the culture during the culturing process, with or without periodic cell and/or product harvest before termination of culture.

"Perfusion culture" is a culture by which the cells are restrained in the culture by, *e*.*g*., filtration, encapsulation, anchoring to microcarriers, etc., and the culture medium is continuously or intermittently introduced and removed from the culturing vessel.

"Culturing vessel" refers to a container used for culturing a cell. The culturing vessel can be of any size so long as it is useful for the culturing of cells.

"Titer": The term "titer" as used herein refers to the total amount of recombinantly expressed polypeptide produced by a cell culture divided by a given amount of medium volume. Titer is typically expressed in units of milligrams of polypeptide per milliliter of medium.

The terms "medium" and "cell culture medium" refer to a nutrient source used for growing or maintaining cells. As is understood by a person of skill in the art, the nutrient source may contain components required by the cell for growth and/or survival or may contain components that aid in cell growth and/or survival. Vitamins, essential or non-essential amino acids, and trace elements are examples of medium components.

A "chemically defined cell culture medium" or "CDM" is a medium with a specified composition that is free of animal-derived products such as animal serum and peptone. The terms also encompass a medium with a specified composition that is free of undefined or partially defined components, for example, components such as an animal serum, an animal peptone, and a plant peptone. As would be understood by a person of skill in the art, a CDM may be used in a process of polypeptide production whereby a cell is in contact with, and secretes a polypeptide into, the CDM. Thus, it is understood that a composition may contain a CDM and a polypeptide product and that the presence of the polypeptide product does not render the CDM chemically undefined.

A "chemically undefined cell culture medium" refers to a medium whose chemical composition cannot be specified and which may contain one or more animal-derived products such as animal serum and peptone. As would be understood by a person of skill in the art, a chemically undefined cell culture medium may contain an animal-derived product as a nutrient source. The term can also encompass a cell culture medium comprising undefined or partially undefined components, for example, components such as an animal serum, an animal peptone, and a plant peptone.

The terms "polypeptide" and "protein" are used interchangeably herein to refer to polymers of amino acids of any length. The polymer may be linear or branched, it may comprise modified amino acids, and it may be interrupted by non-amino acids. The terms also encompass an amino acid polymer that has been modified naturally or by intervention; for example, disulfide bond formation, glycosylation, lipidation, acetylation, phosphorylation, or any other manipulation or modification, such as conjugation with a labeling component. Also included within the definition are, for example, polypeptides containing one or more analogs of an amino acid (including, for example, unnatural amino acids, etc.), as well as other modifications known in the art. Examples of polypeptides encompassed within the definition herein include mammalian proteins, such as, *e.g.,* renin; a growth hormone, including human growth hormone and bovine growth hormone; growth hormone releasing factor; parathyroid hormone; thyroid stimulating hormone; lipoproteins; alpha-1-antitrypsin; insulin A-chain; insulin B-chain; proinsulin; follicle stimulating hormone; calcitonin; luteinizing hormone; glucagon; clotting factors such as factor VIIIC, factor IX, tissue factor, and von Willebrands factor; anti-clotting factors such as Protein C; atrial natriuretic factor; lung surfactant; a plasminogen activator, such as urokinase or human urine or tissue-type plasminogen activator (t-PA); bombesin; thrombin; hemopoietic growth factor; tumor necrosis factor-alpha and -beta; enkephalinase; RANTES (regulated on activation normally T-cell expressed and secreted); human macrophage inflammatory protein (MIP-1-alpha); a serum albumin such as human serum albumin; Muellerian-inhibiting substance; relaxin A-chain; relaxin B-chain; prorelaxin; mouse gonadotropin-associated peptide; a microbial protein, such as beta-lactamase; DNase; IgE; a cytotoxic T-lymphocyte associated antigen (CTLA), such as CTLA-4; inhibin; activin; vascular endothelial growth factor (VEGF); receptors for hormones or growth factors; protein A or D; rheumatoid factors; a neurotrophic factor such as bone-derived neurotrophic factor (BDNF), neurotrophin-3, -4, -5, or -6 (NT-3, NT-4, NT-5, or NT-6), or a nerve growth factor such as NGF-b; platelet-derived growth factor (PDGF); fibroblast growth factor such as aFGF and bFGF; epidermal growth factor (EGF); transforming growth factor (TGF) such as TGF-alpha and TGF-beta, including TGF-β1, TGF-β2, TGF-β3, TGF-β4, or TGF-β5; insulin-like growth factor-I and -II (IGF-I and IGF-II); des(1-3)-IGF-I (brain IGF-I), insulin-like growth factor binding proteins (IGFBPs); CD proteins such as CD3, CD4, CD8, CD19 and CD20; erythropoietin; osteoinductive factors; immunotoxins; a bone morphogenetic protein (BMP); an interferon such as interferon-alpha, -beta, and -gamma; colony stimulating factors (CSFs), *e.g.,* M-CSF, GM-CSF, and G-CSF; interleukins (ILs), *e.g.,* IL-1 to IL-10; superoxide dismutase; T-cell receptors; surface membrane proteins; decay accelerating factor; viral antigen such as, for example, a portion of the AIDS envelope; transport proteins; homing receptors; addressins; regulatory proteins; integrins such as CD11a, CD11b, CD11c, CD18, an ICAM, VLA-4 and VCAM; a tumor associated antigen such as CA125 (ovarian cancer antigen) or HER2, HER3 or HER4 receptor; immunoadhesins; and fragments and/or variants of any of the above-listed proteins as well as antibodies, including antibody fragments, binding to a protein, including, for example, any of the above-listed proteins.

An "isolated polypeptide" means a polypeptide that has been recovered from a cell or cell culture from which it was expressed.

"nucleic acid," as used interchangeably herein, refer to polymers of nucleotides of any length, and include DNA and RNA. The nucleotides can be deoxyribonucleotides, ribonucleotides, modified nucleotides or bases, and/or their analogs, or any substrate that can be incorporated into a polymer by DNA or RNA polymerase, or by a synthetic reaction. A polynucleotide may comprise modified nucleotides, such as methylated nucleotides and their analogs. If present, modification to the nucleotide structure may be imparted before or after assembly of the polymer.

An "isolated nucleic acid" means and encompasses a non-naturally occurring, recombinant or a naturally occurring sequence outside of or separated from its usual context..

A "purified" polypeptide means that the polypeptide has been increased in purity, such that it exists in a form that is more pure than it exists in its natural environment and/or when initially produced and/or synthesized and/or amplified under laboratory conditions. Purity is a relative term and does not necessarily mean absolute purity.

The term "antibody" is used in the broadest sense and specifically covers monoclonal antibodies (including full length monoclonal antibodies), polyclonal antibodies, multispecific antibodies (*e*.*g*., bispecific antibodies), and antibody fragments.

"Antibody fragments" comprise a portion of a full length antibody, generally the antigen binding or variable region thereof. Examples of antibody fragments include Fab, Fab', F(ab')2, and Fv fragments; single-chain antibody molecules; diabodies; linear antibodies; and multispecific antibodies formed from antibody fragments.

The term "monoclonal antibody" as used herein refers to an antibody obtained from a population of substantially homogeneous antibodies, *i.e.,* the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present disclosure may be made by the hybridoma method first described by Kohleret al, Nature 256:495 (1975), or may be made by recombinant DNA methods (see, *e.g.,* U.S. Pat. No. 4,816,567). The "monoclonal antibodies" may also be isolated from phage antibody libraries using the techniques described in Clackson et al., Nature 352:624-628 (1991) and Marks et al., J. Mol. Biol. 222:581-597 (1991), for example.

"Humanized" antibodies are forms of non-human (*e*.*g*., rodent) antibodies that are chimeric antibodies that contain minimal sequence derived from the non-human antibody. For the most part, humanized antibodies are human immunoglobulins (recipient antibody) in which residues from a hypervariable region of the recipient are replaced by residues from a hypervariable region of a non-human species (donor antibody) such as mouse, rat, rabbit or non-human primate having the desired antibody specificity, affinity, and capability. In some instances, framework region (FR) residues of the human immunoglobulin are replaced by corresponding non-human residues. Furthermore, humanized antibodies can comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin sequence. The humanized antibody optionally also will comprise at least a portion of an immunoglobulin constant region (Fc), typically that of a human immunoglobulin. For further details, see Jones et al., Nature 321:522-525 (1986); Riechmann et al., Nature 332:323-329 (1988); and Presta, Curr. Op. Struct. Biol. 2:593-596 (1992).

A "species-dependent antibody" is an antibody which has a stronger binding affinity for an antigen from a first mammalian species than it has for a homologue of that antigen from a second mammalian species. Normally, the species-dependent antibody "bind specifically" to a human antigen (*i.e.,* has a binding affinity (Kd) value of no more than about 1.times. 10.sup.-7 M, no more than about 1.times. 10.sup.-8 or no more than about 1.times. 10.sup.-9 M) but has a binding affinity for a homologue of the antigen from a second non-human mammalian species which is at least about 50 fold, or at least about 500 fold, or at least about 1000 fold, weaker than its binding affinity for the human antigen. The species-dependent antibody can be of any of the various types of antibodies as defined above, but preferably is a humanized or human antibody.

"Contaminants" refer to materials that are different from the desired polypeptide product. The contaminant includes, without limitation: host cell materials, such as CHOP; leached Protein A; nucleic acid; a variant, fragment, aggregate or derivative of the desired polypeptide; another polypeptide; endotoxin; viral contaminant; cell culture media component, etc.

The term "pharmaceutical formulation" refers to a preparation which is in such form as to permit the biological activity of the active ingredient to be effective, and which contains no additional components which are unacceptably toxic to a subject to which the formulation would be administered. Such formulations are sterile.

A "sterile" formulation is aseptic or free or essentially free from all living microorganisms and their spores.

A "colorless or slightly colored" liquid refers to a liquid composition comprising a polypeptide that is measured by quantitative and/or qualitative analysis. Qualitative analysis includes visual inspection such as comparison of the composition comprising the polypeptide to a reference standard.

It is understood that wherever embodiments are described herein with the language "comprising," otherwise analogous embodiments described in terms of "consisting of" and/or "consisting essentially of" are also provided.

Where aspects or embodiments of the disclosure are described in terms of a Markush group or other grouping of alternatives, the present disclosure encompasses not only the entire group listed as a whole, but each member of the group individually and all possible subgroups of the main group, but also the main group absent one or more of the group members. The present disclosure also envisages the explicit exclusion of one or more of any of the group members in the claimed disclosure.

### Cell Culture Medium

Cell culture media provided herein may find use in methods (*e.g.,* of growing cells (*i.e.,* of culturing cells) and producing polypeptides) and in compositions as detailed herein. Media components have been identified as capable of providing a polypeptide drug product with acceptable quality attributes, such as an acceptable color (*e.g.,* for use as an injectable drug product). Certain media components reduce the color intensity of a polypeptide drug product as compared to the polypeptide produced in different media, which may be particularly significant for polypeptide products that are formulated at concentrations of greater than any of 100 mg/mL or 125 mg/mL or 150 mg/mL. In some aspects, a polypeptide drug product is at a concentration greater than at least 100 mg/mL, at least 125 mg/mL, or at least 150 mg/mL and has a color intensity value greater than B3, B4, B5, B6, B7, B8, or B9 as measured by the COC assay. In some aspects, a polypeptide drug product is at a concentration greater than at least 1 mg/mL, at least 10 mg/mL, at least 25 mg/mL, at least 50 mg/mL, or at least 75 mg/mL and has a color intensity value greater than B3, B4, B5, B6, B7, B8, or B9 as measured by the COC assay. In some aspects, the color intensity value as determined by the COC assay can be any one of, but not limited to, B, BY, Y, GY, or R, wherein higher values indicate a lighter color intensity. In some aspects, a polypeptide drug product is at a concentration greater than at least 100 mg/mL, at least 125 mg/mL, or at least 150 mg/mL and has a color intensity value less than a color intensity value of a reference solution as measured by a color assay (*e.g.,* the Total Color assay or the NIFTY assay). In some aspects, a polypeptide drug product is at a concentration greater than at least 1 mg/mL, at least 10 mg/mL, at least 25 mg/mL, at least 50 mg/mL, or at least 75 mg/mL and has a color intensity value less than a color intensity value of a reference solution as measured by a color assay (*e.g.,* the Total Color assay or the NIFTY assay). Suitable cell culture media are detailed throughout, including in the Brief Summary of the Invention and elsewhere. Any medium detailed herein may be employed at any stage of cell growth, maintenance and polypeptide production and may be used in the basal medium and/or in the feed medium. Media as described herein in one variation result in acceptable cell viability and antibody titer levels, and in acceptable color intensity of a polypeptide (*e*.*g*., antibody) isolated from cell culture grown in the media.

A cell culture medium comprising one or more of the following components is provided: (a) cystine and/or cysteine; (b) vitamin B2, (c) vitamin B6 (pyridoxine and/or pyridoxal), (d) vitamin B9, (e) vitamin B12, (f) an iron source such as ferric citrate and (g) hydrocortisone. In one variation, a cell culture medium comprises 2 or 3 or 4 or 5 or 6 or each of components (a), (b), (c), (d), (e), (f) and (g). It is understood that a cell culture medium provided herein may contain any combination of components (a), (b), (c), (d), (e), (f) and (g) the same as if each and every combination were specifically and individually listed. For example, it is understood that a cell culture medium comprising four of components (a), (b), (c), (d), (e), (f) and (g) may comprise any combination of the components so long as at least four of the components are present. In one aspect, the cell culture medium is a CDM. In another aspect, the cell culture medium is a chemically undefined cell culture medium.

Media components may be added to a composition in forms that are known in the art. For example, vitamin B2 may be provided as riboflavin powder, vitamin B6 may be provided as pyridoxine HCl or as pyridoxal HCl, vitamin B9 may be provided as folic acid powder, vitamin B12 may be provided as cyanocobalamin powder, cysteine may be provided as L-Cysteine monohydrochloride monohydrate powder, cystine may be provided as disodium salt monohydrate powder. In some embodiments, vitamin B6 is not provided as pyridoxal HCl. In an additional non-limiting example, vitamin B1 may be provided as thiamine monohydrochloride, vitamin B3 may be provided as niacinamide, vitamin B5 may be provided as D-calcium pantothenate, and vitamin B7 may be provided as biotin. As another non-limiting example, iron may be added in different iron forms or iron sources. In some embodiments, an iron source is ferric citrate or ferrous sulfate. Media components described herein can be provided in the form of a salt, a hydrate, a salt hydrate, or as a solution, an extract, or in solid form.

In one variation, the medium comprises cystine and each of vitamins B2, B6, B9 and B12. In one variation, the medium comprises cystine, vitamins B2, B6, B9, B12, and an iron source such as ferric citrate. In another variation, the medium comprises each of cystine, vitamins B2, B6, B9, B12, an iron source such as ferric citrate, and hydrocortisone. In a further variation, the medium comprises cystine, hydrocortisone and an iron source such as ferric citrate. In still another variation, the medium comprises cystine, hydrocortisone, an iron source such as ferric citrate, and at least one of vitamins B2, B6, B9 and B12. In still another variation, the medium comprises cystine, hydrocortisone, an iron source such as ferric citrate, and at least two of vitamins B2, B6, B9 and B12. In still another variation, the medium comprises cystine, hydrocortisone, an iron source such as ferric citrate, and at least three of vitamins B2, B6, B9 and B12. In any medium described herein, in one aspect the medium is a CDM. In one aspect, a cell culture medium comprises cysteine. In another variation, a cell culture medium comprises cystine and is free of cysteine. In another variation, a cell culture medium comprises both cystine and cysteine.

In one variation, the media is a cell culture medium comprising from about 300 mg/L (in some embodiments, 200 mg/L) to about 1200 mg/L cystine, from about 0.05 mg/L to about 1.0 mg/L vitamin B2, from about 0.05 mg/L to about 10.0 mg/L vitamin B6, from about 0.05 mg/L to about 12.0 mg/L vitamin B9 and from about 0.05 mg/L to about 2.5 mg/L vitamin B12. In a variation, vitamin B2 is at a concentration of from about 0.05 mg/L to about 0.50 mg/L. In another variation, vitamin B2 is at a concentration of from about 0.05 mg/L to about 0.40 mg/L. In another variation, vitamin B2 is at a concentration of from about 0.05 mg/L to about 0.30 mg/L. In a variation, vitamin B6 is at a concentration of from about 0.05 mg/L to about 8.0 mg/L. In another variation, vitamin B6 is at a concentration of from about 0.05 mg/L to about 7.0 mg/L. In another variation, vitamin B6 is at a concentration of from about 0.05 mg/L to about 6.0 mg/L. In a variation, the cell culture medium further comprises an iron source. In a variation the iron source is ferric citrate or ferrous sulfate. In one variation, the cell culture medium comprises ferric citrate at a concentration of from about 2 µM to about 80 µM. In any of the variations herein the cell culture medium further comprises hydrocortisone. In a variation the hydrocortisone is at a concentration of from about 0.05 µM to about 0.25 µM.

In another variation, the cell culture medium comprises one or more of the following: (a) from about 300 mg/L (in some embodiments, 200 mg/L) to about 1200 mg/L cystine and/or cysteine (and which in one aspect is cystine); (b) from about 0.05 mg/L to about 1.0 mg/L vitamin B2; (c) from about 0.05 mg/L to about 10.0 mg/L vitamin B6 (which in one aspect is pyridoxine); (d) from about 0.05 mg/L to about 12.0 mg/L vitamin B9; (e) from about 0.05 mg/L to about 2.5 mg/L vitamin B12; (f) from about 2 µM to about 80 µM of an iron source, such as iron nitrate, citrate or sulfate (which in one aspect is ferric citrate and/or ferrous sulfate); and (g) from about 0.05 µM to about 0.25 µM hydrocortisone. In another variation, the cell culture medium comprises one or more of the following: (a) from about 300 mg/L (in some embodiments, 200 mg/L) to about 600 mg/L cystine and/or cysteine (and which in one aspect is cystine); (b) from about 0.05 mg/L to about 0.5 mg/L vitamin B2; (c) from about 2.0 mg/L to about 8.0 mg/L vitamin B6 (which in one aspect is pyridoxine); (d) from about 4.0 mg/L to about 12.0 mg/L vitamin B9; (e) from about 1.0 to about 2.0 mg/L vitamin B12; (f) from about 5 µM to about 25 µM of an iron source, such as iron nitrate, citrate or sulfate (which in one aspect is ferric citrate and/or sulfate); and (g) from about 0.1 µM to about 0.2 µM hydrocortisone. In yet another variation, the cell culture medium comprises one or more of the following: (a) from about 400 mg/L to about 500 mg/L cystine and/or cysteine (and which in one aspect is cystine); (b) from about 0.1 mg/L to about 0.3 mg/L vitamin B2; (c) from about 4.0 mg/L to about 6.0 mg/L vitamin B6 (which in one aspect is pyridoxine); (d) from about 7.0 mg/L to about 10.0 mg/L vitamin B9; (e) from about 1.5 to about 2.0 mg/L vitamin B12; (f) from about 12 µM to about 20 µM of an iron source, such as iron nitrate, citrate or sulfate (which in one aspect is ferric citrate and/or sulfate); and (g) from about 0.125 µM to about 0.175 µM hydrocortisone. In one variation, a cell culture medium comprises 2 or 3 or 4 or 5 or 6 or each of components (a), (b), (c), (d), (e), (f) and (g) in the concentrations recited herein. It is understood that a cell culture medium may contain any combination of components (a), (b), (c), (d), (e), (f) and (g) in the concentration ranges provided herein the same as if each and every combination were specifically and individually listed. For example, it is understood that the medium in one variation comprises components (a), (b), (c), (d) and (e) and may optionally comprise components (f) and/or (g).. It is also understood that in another variation, the medium comprises components (a), (f) and (g) and may optionally comprise any one or more of components (b), (c), (d) and (e). In any variation in which the medium comprises cystine or cysteine, in one aspect the medium comprises cystine (and in a further variation is free of cysteine). In any variation in which the medium comprises vitamin B6, in one aspect the medium comprises pyridoxane In any variation in which the medium comprises an iron source, in one aspect the iron source is ferric citrate. Thus, it is understood that in certain variations, a medium comprises cystine, pyridoxane and ferric citrate. In one aspect, the cell culture medium is a CDM.

In one variation, the medium comprises (a) from about 300 mg/L (in some embodiments, 200 mg/L) to about 1200 mg/L cystine; (b) from about 0.05 mg/L to about 1.0 mg/L vitamin B2; (c) from about 0.05 mg/L to about 10.0 mg/L vitamin B6; (d) from about 0.05 mg/L to about 12.0 mg/L vitamin B9; and (e) from about 0.05 mg/L to about 2.5 mg/L vitamin B12. In one variation, the medium comprises (a) from about 300 mg/L (in some embodiments, 200 mg/L) to about 1200 mg/L cystine; (b) from about 0.05 mg/L to about 1.0 mg/L vitamin B2; (c) from about 0.05 mg/L to about 10.0 mg/L vitamin B6; (d) from about 0.05 mg/L to about 12.0 mg/L vitamin B9; (e) from about 0.05 mg/L to about 2.5 mg/L vitamin B12; and (f) from about 2 µM to about 80 µM of an iron source, such as ferric citrate or ferrous sulfate. In another variation, the medium comprises each of (a) from about 300 mg/L (in some embodiments, 200 mg/L) to about 1200 mg/L cystine; (b) from about 0.05 mg/L to about 1.0 mg/L vitamin B2; (c) from about 0.05 mg/L to about 10.0 mg/L vitamin B6; (d) from about 0.05 mg/L to about 12.0 mg/L vitamin B9; (e) from about 0.05 mg/L to about 2.5 mg/L vitamin B12; (f) from about 2 µM to about 80 µM of an iron source, such as ferric citrate or ferrous sulfate; and (g) from about 0.05 µM to about 0.25 µM hydrocortisone. In a further variation, the medium comprises: from about 300 mg/L (in some embodiments, 200 mg/L) to about 1200 mg/L cystine; from about 2 µM to about 80 µM of an iron source, such as ferric citrate or ferrous sulfate; and from about 0.05 µM to about 0.25 µM hydrocortisone. In still another variation, the medium comprises: from about 300 mg/L (in some embodiments, 200 mg/L) to about 1200 mg/L cystine; from about 2 µM to about 80 µM of an iron source, such as ferric citrate or ferrous sulfate; from about 0.05 µM to about 0.25 µM hydrocortisone, and at least one or two or three of: from about 0.05 mg/L to about 1.0 mg/L vitamin B2; from about 0.05 mg/L to about 10.0 mg/L vitamin B6; from about 0.05 mg/L to about 12.0 mg/L vitamin B9; and from about 0.05 mg/L to about 2.5 mg/L vitamin B12. In any medium described herein, in one aspect the medium is a CDM. In any medium described herein, in one aspect the medium is a chemically undefined cell culture medium.

In some further variations, the cell culture medium further comprises cysteine in amounts as described in Table 1. For example, it is understood that a cell culture medium comprising (a) from about 300 mg/L (in some embodiments, 200 mg/L) to about 1200 mg/L cystine; (b) from about 0.05 mg/L to about 1.0 mg/L vitamin B2; (c) from about 0.05 mg/L to about 10.0 mg/L vitamin B6; (d) from about 0.05 mg/L to about 12.0 mg/L vitamin B9; and (e) from about 0.05 mg/L to about 2.5 mg/L vitamin B12 can further comprise from about 80 mg/L to about 1500 mg/L cysteine. In a variation, the a cell culture medium comprising (a) from about 300 mg/L (in some embodiments, 200 mg/L) to about 1200 mg/L cystine; (b) from about 0.05 mg/L to about 1.0 mg/L vitamin B2; (c) from about 0.05 mg/L to about 10.0 mg/L vitamin B6; (d) from about 0.05 mg/L to about 12.0 mg/L vitamin B9; (e) from about 0.05 mg/L to about 2.5 mg/L vitamin B12; and (f) from about 2 µM to about 80 µM of an iron source, such as ferric citrate or ferrous sulfate can further comprise from about 80 mg/L to about 1500 mg/L cysteine. In some variations, a cell culture medium comprising (a) from about 300 mg/L (in some embodiments, 200 mg/L) to about 1200 mg/L cystine; (b) from about 0.05 mg/L to about 1.0 mg/L vitamin B2; (c) from about 0.05 mg/L to about 10.0 mg/L vitamin B6; (d) from about 0.05 mg/L to about 12.0 mg/L vitamin B9; (e) from about 0.05 mg/L to about 2.5 mg/L vitamin B12; (f) from about 2 µM to about 80 µM of an iron source, such as ferric citrate or ferrous sulfate; and (g) from about 0.05 µM to about 0.25 µM hydrocortisone can further comprise from about 80 mg/L to about 1500 mg/L cysteine. In yet another variation, a cell culture medium comprising from about 300 mg/L (in some embodiments, 200 mg/L) to about 1200 mg/L cystine; from about 2 µM to about 80 µM of an iron source, such as ferric citrate or ferrous sulfate; from about 0.05 µM to about 0.25 µM hydrocortisone, and at least one or two or three of: from about 0.05 mg/L to about 1.0 mg/L vitamin B2; from about 0.05 mg/L to about 10.0 mg/L vitamin B6; from about 0.05 mg/L to about 12.0 mg/L vitamin B9; and from about 0.05 mg/L to about 2.5 mg/L vitamin B12 can further comprise from about 80 mg/L to about 1500 mg/L cysteine.

Individual media components may be present in amounts that result in one or more advantageous properties (such as one or more acceptable product quality attribute). In one variation, a cell culture medium as provided herein contains media components in amounts as described in Table 1. It is understood that a medium may comprise any one or more of the medium components of Table 1 *(e.g.,* any one or more of components (a)-(g), such as a medium comprising components (a), (b), (c), (d) and (e) or a medium comprising components (a), (f) and (g) or a medium comprising each of components (a)-(g)) in any of the amounts listed in Table 1, the same as if each and every combination of components and amounts were specifically and individually listed. In a particular variation, the medium is a CDM. In another particular variation, the medium is a chemically undefined cell culture medium. A medium provided herein (*e.g.,* a CDM) in one variation comprises pyridoxine and is free of pyridoxal. A pyridoxal-free medium may be employed in the basal medium and/or in the feed medium. In one variation, the basal medium is free of pyridoxal and comprises pyridoxine.

**Table 1. Exemplary Amounts of Media Components**

| **Media Component** | **Amount of Component in Medium** |
|---|---|
| (a) Cystine and/or cysteine (which in one variation is cystine) | from about 80 mg/L to about 1500 mg/L; from about 100 mg/L to about 1500 mg/L; from about 200 mg/L to about 1500 mg/L; from about 200 mg/L to about 1400 mg/L; from about 200 mg/L to about 1300 mg/L; from about 200 mg/L to about 1200 mg/L; from about 300 mg/L to about 1200 mg/L; from about 300 mg/L to about 1100 mg/L; from about 300 mg/L to about 1000 mg/L; from about 300 mg/L to about 900 mg/L; from about 300 mg/L to about 800 mg/L; from about 300 mg/L to about 700 mg/L; from about 300 mg/L to about 600 mg/L; from about 300 mg/L to about 500 mg/L; from about 300 mg/L to about 400 mg/L; from about 400 mg/L to about 1500 mg/L; from about 500 mg/L to about 1500 mg/L; from about 600 mg/L to about 1500 mg/L; from about 700 mg/L to about 1500 mg/L; from about 400 mg/L to about 1200 mg/L; from about 500 mg/L to about 1200 mg/L; from about 600 mg/L to about 1200 mg/L; from about 700 mg/L to about 1200 mg/L; from about 800 mg/L to about 1200 mg/L; from about 900 mg/L to about 1200 mg/L; from about 1000 mg/L to about 1200 mg/L; from about 1100 mg/L to about 1200 mg/L; from about 350 mg/L to about 850 mg/L; from about 400 mg/L to about 800 mg/L; from about 450 mg/L to about 550 mg/L; from about 450 mg/L to about 500 mg/L; about any of 80 or 81 or 82 or 83 or 84 or 85 or 86 or 87 or 88 or 90 or 100 or 120 or 140 or 160 or 180 or 200 or 210 or 220 or 230 or 240 or 250 or 260 or 270 or 280 or 290 or 300 or 350 or 375 or 400 or 425 or 450 or 475 or 500 or 525 or 550 or 575 or 600 or 625 or 650 or 675 or 700 or 725 or 750 or 765 or 775 or 785 or 795 or 800 or 825 or 850 or 875 or 900 or 925 or 950 or 975 or 1000 or 1050 or 1100 or 1150 or 1200 or 1250 or 1300 or 1350 or 1400 or 1450 or 1500 mg/L; at least about any of 80 or 81 or 82 or 83 or 84 or 85 or 86 or 87 or 88 or 90 or 100 or 120 or 140 or 160 or 180 or 200 or 210 or 220 or 230 or 240 or 250 or 260 or 270 or 280 or 290 or 300 or 350 or 375 or 400 or 425 or 450 or 475 or 500 or 525 or 550 or 575 or 600 or 625 or 650 or 675 or 700 or 725 or 750 or 765 or 775 or 785 or 795 or 800 or 825 or 850 or 875 or 900 or 925 or 950 or 975 or 1000 or 1050 or 1100 or 1150 or 1200 or 1250 or 1300 or 1350 or 1400 or 1450 or 1500 mg/L. |
| (b) vitamin B2 | from about 0.05 mg/L to about 1.0 mg/L; from about 0.05 mg/L to about 0.9 mg/L; from about 0.05 mg/L to about 0.8 mg/L; from about 0.05 mg/L to about 0.7 mg/L; from about 0.05 mg/L to about 0.6 mg/L; from about 0.05 mg/L to about 0.5 mg/L; from about 0.05 mg/L to about 0.4 mg/L; from about 0.05 mg/L to about 0.3 mg/L; from about 0.05 mg/L to about 0.2 mg/L; from about 0.05 mg/L to about 0.1 mg/L; from about 0.1 mg/L to about 1.0 mg/L; from about 0.2 mg/L to about 1.0 mg/L; from about 0.3 mg/L to about 1.0 mg/L; from about 0.4 mg/L to about 1.0 mg/L; from about 0.5 mg/L to about 1.0 mg/L; from about 0.6 mg/L to about 1.0 mg/L; from about 0.7 mg/L to about 1.0 mg/L; from about 0.8 mg/L to about 1.0 mg/L; from about 0.9 mg/L to about 1.0 mg/L; from about 0.1 mg/L to about 0.6 mg/L; from about 0.2 mg/L to about 0.4 mg/L; from about 0.2 mg/L to about 0.3 mg/L; about any of 0.05 or 0.1 or 0.15 or 0.2 or 0.25 or 0.3 or 0.35 or 0.4 or 0.45 or 0.5 or 0.55 or 0.6 or 0.65 or 0.7 or 0.75 or 0.8 or 0.85 or 0.9 or 0.95 or 1.0 mg/L; at least about any of 0.05 or 0.1 or 0.15 or 0.2 or 0.25 or 0.3 or 0.35 or 0.4 or 0.45 mg/L and no more than 0.7 or 0.6 mg/L. |
| (c) vitamin B6 (which in one aspect is pyridoxine) | from about 0.05 mg/L to about 10.0 mg/L; from about 0.05 mg/L to about 9.5 mg/L; from about 0.05 mg/L to about 9.0 mg/L; from about 0.05 mg/L to about 8.5 mg/L; from about 0.05 mg/L to about 8.0 mg/L; from about 0.05 mg/L to about 7.5 mg/L; from about 0.05 mg/L to about 7.0 mg/L; from about 0.05 mg/L to about 6.5 mg/L; from about 0.05 mg/L to about 6.0 mg/L; from about 0.05 mg/L to about 5.5 mg/L; from about 0.05 mg/L to about 5.0 mg/L; from about 0.05 mg/L to about 4.5 mg/L; from about 0.05 mg/L to about 4.0 mg/L; from about 0.05 mg/L to about 3.5 mg/L; from about 0.05 mg/L to about 3.0 mg/L; from about 0.05 mg/L to about 2.5 mg/L; from about 0.05 mg/L to about 2.0 mg/L; from about 0.05 mg/L to about 1.5 mg/L; from about 0.05 mg/L to about 1.0 mg/L; from about 1.0 mg/L to about 10.0 mg/L; from about 1.5 mg/L to about 10.0 mg/L; from about 2.0 mg/L to about 10.0 mg/L; from about 2.5 mg/L to about 10.0 mg/L; from about 3.0 mg/L to about 10.0 mg/L; from about 3.5 mg/L to about 10.0 mg/L; from about 4.0 mg/L to about 10.0 mg/L; from about 4.5 mg/L to about 10.0 mg/L; from about 5.0 mg/L to about 10.0 mg/L; from about 5.5 mg/L to about 10.0 mg/L; from about 6.0 mg/L to about 10.0 mg/L; from about 7.0 mg/L to about 10.0 mg/L; from about 8.0 mg/L to about 10.0 mg/L; from about 2.5 mg/L to about 8.0 mg/L; from about 3.0 mg/L to about 7.0 mg/L; from about 4.5 mg/L to about 6.5 mg/L; about any of 1.0 or 2.0 or 3.0 or 3.5 or 4.0 or 4.5 or 5.0 or 5.5 or 6.0 or 6.5 or 7.0 or 8 mg/L; at least about any of 2.0 or 3.0 or 4.0 or 5.0 mg/L and no more than 7.0 or 8.0 mg/L. |
| (d) vitamin B9 | from about 0.05 mg/L to about 12.0 mg/L; from about 0.05 mg/L to about 11.0 mg/L; from about 0.05 mg/L to about 10.0 mg/L; from about 0.05 mg/L to about 9.5 mg/L; from about 0.05 mg/L to about 9.0 mg/L; from about 0.05 mg/L to about 8.5 mg/L; from about 0.05 mg/L to about 8.0 mg/L; from about 0.05 mg/L to about 7.5 mg/L; from about 0.05 mg/L to about 7.0 mg/L; from about 0.05 mg/L to about 6.5 mg/L; from about 0.05 mg/L to about 6.0 mg/L; from about 0.05 mg/L to about 5.5 mg/L; from about 0.05 mg/L to about 5.0 mg/L; from about 0.05 mg/L to about 4.5 mg/L; from about 0.05 mg/L to about 4.0 mg/L; from about 0.05 mg/L to about 3.5 mg/L; from about 0.05 mg/L to about 3.0 mg/L; from about 0.05 mg/L to about 2.5 mg/L; from about 0.05 mg/L to about 2.0 mg/L; from about 0.05 mg/L to about 1.5 mg/L; from about 0.05 mg/L to about 1.0 mg/L; from about 1.0 mg/L to about 12.0 mg/L; from about 1.5 mg/L to about 12.0 mg/L; from about 2.0 mg/L to about 12.0 mg/L; from about 2.5 mg/L to about 12.0 mg/L; from about 3.0 mg/L to about 12.0 mg/L; from about 3.5 mg/L to about 12.0 mg/L; from about 4.0 mg/L to about 12.0 mg/L; from about 4.5 mg/L to about 12.0 mg/L; from about 5.0 mg/L to about 12.0 mg/L; from about 5.5 mg/L to about 12.0 mg/L; from about 6.0 mg/L to about 12.0 mg/L; from about 7.0 mg/L to about 12.0 mg/L; from about 8.0 mg/L to about 10.0 mg/L; from about 3.0 mg/L to about 10.0 mg/L; from about 4.0 mg/L to about 9.0 mg/L; from about 7.0 mg/L to about 10.0 mg/L; about any of 1.0 or 2.0 or 3.0 or 4.0 or 4.5 or 5.0 or 5.5 or 6.0 or 6.5 or 7.0 or 7.5 or 8 or 8.5 or 9.0 or 9.5 or 10 mg/L; at least about any of 2.0 or 3.0 or 4.0 or 5.0 or 6.0 or 7.0 or 8.0 mg/L and no more than 12.0 or 10.0 or 9.0 mg/L. |
| (e) vitamin B12 | from about 0.05 to about 2.5 mg/L; from about 0.05 to about 2.25 mg/L; from about 0.05 to about 2.0 mg/L; from about 0.05 to about 1.75 mg/L; from about 0.05 to about 1.5 mg/L; from about 0.05 to about 1.25 mg/L; from about 0.05 to about 1.0 mg/L; from about 0.05 to about 0.75 mg/L; from about 0.05 to about 0.5 mg/L; from about 0.05 to about 0.25 mg/L; from about 0.5 to about 2.5 mg/L; from about 0.75 to about 2.5 mg/L; from about 1.0 to about 2.5 mg/L; from about 1.25 to about 2.5 mg/L; from about 1.5 to about 2.5 mg/L; from about 1.75 to about 2.5 mg/L; from about 2.0 to about 2.5 mg/L; from about 2.25 to about 2.5 mg/L; from about 0.5 to about 2.0 mg/L; from about 1.0 to about 2.0 mg/L; from about 1.25 to about 2.0 mg/L; from about 1.5 to about 2.25 mg/L; from about 075 to about 2.0 mg/L; about any of 0.5 or 1.0 or 1.25 or 1.5 or 1.75 or 2.0 or 2.25 to about 2.5 mg/L; at least about any of 0.5 or 1.0 or 1.25 or 1.5 and no more than 2.25 or 2.0 or 1.75 mg/L. |
| (f) an iron source such as ferric citrate | from about 2 µM to about 80 µM; from about 2 µM to about 40 µM; from about 2 µM to about 30 µM; from about 2 µM to about 25 µM; from about 2 µM to about 20 µM; from about 2 µM to about 15 µM; from about 2 µM to about 10 µM; from about 10 µM to about 50 µM; from about 15 µM to about 50 µM; from about 20 µM to about 50 µM; from about 25 µM to about 50 µM; from about 30 µM to about 50 µM; from about 40 µM to about 50 µM; from about 10 µM to about 40 µM; from about 10 µM to about 30 µM; from about 10 µM to about 25 µM; from about 15 µM to about 25 µM; from about 15 µM to about 20 µM; about any of 5 or 10 or 15 or 20 or 25 or 30 or 35 or 40 µM; at least about any of 2 or 5 or 10 or 12.5 or 15 or 17.5 and no more than about 30 or 25 or 20 µM. |
| (g) hydrocortisone | from about 0.05 µM to about 0.25 µM; from about 0.05 µM to about 0.2 µM; from about 0.05 µM to about 0.15 µM; from about 0.05 µM to about 0.1 µM; from about 0.05 µM to about 0.075 µM; from about 0.075 µM to about 0.25 µM; from about 0.1 µM to about 0.25 µM; from about 0.15 µM to about 0.25 µM; from about 0.2 µM to about 0.25 µM; from about 0.1 µM to about 0.2 µM; from about 0.125 µM to about 0.225 µM; from about 0.125 µM to about 0.2 µM; from about 0.15 µM to about 0.175 µM; about any of 0.05or 0.1 or 0.125 or 0.15 or 0.175 or 0.2 or 0.22 µM; at least about any of 0.05or 0.1 or 0.125 and no more than 0.2 or 0.175 µM. |

In some aspects, the disclosure herein provides a cell culture medium comprising one or more of the following components selected from the group consisting of: (a) vitamin B1; (b) vitamin B2; (c) vitamin B3; (d) vitamin B5; (e) vitamin B6; (f) vitamin B7; (g) vitamin B9; (h) vitamin B12; (i) an iron source such as ferric citrate; and (j) cystine. In some embodiments, the cell culture medium comprises 2 or 3 or 4 or 5 or 6 or 7 or 8 or 9 or each of components (a), (b), (c), (d), (e), (f), (g), (h), (i), and (j). It is understood that the cell culture medium provided herein may contain any combination of components (a), (b), (c), (d), (e), (f), (g), (h), (i), and (j) the same as if each and every combination were specifically and individually listed. For example, it is understood that a cell culture medium comprising eight of components (a), (b), (c), (d), (e), (f), (g) and (h) may comprise any combination of the components so long as at least eight of the components are present. In some embodiments, a cell culture provided herein comprises components (b), (e), (g), (h) and (j). In some embodiments herein, a cell culture provided herein comprising components (b), (e), (g), (h) and (j) further comprises (a), (c), (d), and (f). In some embodiments herein, a cell culture provided herein comprising components (a), (b), (c), (d), (e), (f), (g), (h) and (j) further comprises (i).

In some aspects, a cell culture medium as provided herein contains one or more media components selected from the group consisting of (a) vitamin B1; (b) vitamin B2; (c) vitamin B3; (d) vitamin B5; (e) vitamin B6; (f) vitamin B7; (g) vitamin B9; (h) vitamin B12; (i) an iron source such as ferric citrate; and (j) cystine in amounts as described in Table 1A. It is understood that a medium may comprise any one or more of the medium components of Table 1A (*e.g.,* any one or more of components (a)-(j), such as a medium comprising components (a), (b), (c), (d), (e), (f), (g), (h), and (i), or a medium comprising components (b), (e), (g), (h) and (j) or a medium comprising only one of components (a)-(j)) in any of the amounts listed in Table 1A, the same as if each and every combination of components and amounts were specifically and individually listed. In some aspects, a cell culture medium comprises components (a), (b), (c), (d), (e), (f), (g), (h), and (i), wherein (a) is from about 2 µM to about 14 µM vitamin B1, (b) is from about 0.11 µM to about 0.72 µM vitamin B2, (c) is from about 11 µM to about 72 µM vitamin B3, (d) is from about 6.8 µM to about 44 µM vitamin B5, (e) is from about 4.5 µM to about 30 µM vitamin B6, (f) is from about 0.02 µM to about 0.14 µM vitamin B7, (g) is from about 3.4 µM to about 22 µM vitamin B9, (h) is from about 0.2 µM to about 1.5 µM vitamin B12, (i) is from about 11 µM to about 36 µM ferric citrate, and (j) is from about 0.9 mM to about 1.5 mM cystine.

In some further aspects, the cell culture medium further comprises cysteine in amounts as described in Table 1A. For example, it is understood that a cell culture medium comprising (a) from about 2 µM to about 14 µM vitamin B1, (b) from about 0.11 µM to about 0.72 µM vitamin B2, (c) from about 11 µM to about 72 µM vitamin B3, (d) from about 6.8 µM to about 44 µM vitamin B5, (e) from about 4.5 µM to about 30 µM vitamin B6, (f) from about 0.02 µM to about 0.14 µM vitamin B7, (g) from about 3.4 µM to about 22 µM vitamin B9, (h) from about 0.2 µM to about 1.5 µM vitamin B12, (i) from about 11 µM to about 36 µM ferric citrate, and (j) from about 0.7 mM to about 2.0 mM cystine can further comprise (k) from about 0.5 mM to about 2.0 mM cysteine.

**Table 1A. Exemplary Amounts of Media Components**

| **Component** | **Amount of Component in Medium** |
|---|---|
| (a) Vitamin B1 | from about 1 µM to about 18 µM; from about 1 µM to about 16 µM; from about 1 µM to about 14 µM; from about 1 µM to about 12 µM; from about 1 µM to about 10 µM; from about 1 µM to about 8 µM; from about 1 µM to about 6 µM; from about 1 µM to about 4 µM; from about 1 µM to about 2 µM; from about 2 µM to about 18 µM; from about 4 µM to about 18 µM; from about 6 µM to about 18 µM; from about 8 µM to about 18 µM; from about 10 µM to about 18 µM; from about 12 µM to about 18 µM; from about 14 µM to about 18 µM; from about 16 µM to about 18 µM; from about 1.5 µM to about 16 µM; from about 2 µM to about 14 µM; from about 2.5 µM to about 12 µM; from about 3 µM to about 10 µM; from about 3.5 µM to about 8 µM; from about 4 µM to about 6 µM; about any of 1 or 2 or 4 or 6 or 8 or 10 or 12 or 14 µM; at least about any of 1 or 2 or 4 or 6 µM and no more than about 14 or 12 or 10 µM. |
| (b) Vitamin B2 | from about 0.09 µM to about 0.8 µM; from about 0.09 µM to about 0.6 µM; from about 0.09 µM to about 0.4 µM; from about 0.09 µM to about 0.2 µM; from about 0.09 µM to about 0.1 µM; from about 0.1 µM to about 0.8 µM; from about 0.2 µM to about 0.8 µM; from about 0.4 µM to about 0.8 µM; from about 0.6 µM to about 0.8 µM; from about 0.1 µM to about 0.7 µM; from about 0.2 µM to about 0.6 µM; from about 0.3 µM to about 0.5 µM; from about 0.1 µM to about 0.76 µM; from about 0.11 µM to about 0.72 µM; from about 0.12 µM to about 0.68 µM; from about 0.13 µM to about 0.64 µM; from about 0.14 µM to about 0.6 µM; about any of 0.1 or 0.2 or 0.3 or 0.4 or 0.5 or 0.6 or 0.7 µM; at least about any of 0.1 or 0.2 or 0.3 µM and no more than about 0.8 or 0.7 or 0.6 µM. |
| (c) Vitamin B3 | from about 8.5 µM to about 86 µM; from about 8.5 µM to about 80 µM; from about 8.5 µM to about 70 µM; from about 8.5 µM to about 60 µM; from about 8.5 µM to about 50 µM; from about 8.5 µM to about 40 µM; from about 8.5 µM to about 30 µM; from about 8.5 µM to about 20 µM; from about 8.5 µM to about 10 µM; from about 10 µM to about 86 µM; from about 20 µM to about 86 µM; from about 30 µM to about 86 µM; from about 40 µM to about 86 µM; from about 50 µM to about 86 µM; from about 60 µM to about 86 µM; from about 70 µM to about 86 µM; from about 80 µM to about 86 µM; from about 10 µM to about 80 µM; from about 20 µM to about 70 µM; from about 30 µM to about 60 µM; from about 40 µM to about 50 µM; from about 9.5 µM to about 80 µM; from about 11 µM to about 72 µM; from about 12.5 µM to about 64 µM; from about 14 µM to about 56 µM; about any of 10 or 11 or 15 or 20 or 30 or 40 or 50 or 60 or 70 or 72 µM; at least about any of 9 or 10 or 11 or 12 or 13 µM and no more than about 80 or 75 or 72 or 65 µM. |
| (d) Vitamin B5 | from about 5.4 µM to about 54 µM; from about 5.4 µM to about 50 µM; from about 5.4 µM to about 40 µM; from about 5.4 µM to about 30 µM; from about 5.4 µM to about 20 µM; from about 5.4 µM to about 10 µM; from about 10 µM to about 54 µM; from about 20 µM to about 54 µM; from about 30 µM to about 54 µM; from about 40 µM to about 54 µM; from about 50 µM to about 54 µM; from about 6 µM to about 50 µM; from about 7 µM to about 40 µM; from about 8 µM to about 30 µM; from about 9 µM to about 20 µM; from about 6.1 µM to about 50 µM; from about 6.2 µM to about 49 µM; from about 6.3 µM to about 48 µM; from about 6.4 µM to about 47 µM; from about 6.5 µM to about 46 µM; from about 6.6 µM to about 45 µM; from about 6.8 µM to about 44 µM; about any of 6 or 6.8 or 10 or 20 or 30 or 40 or 44 or 50 µM; at least about any of 6 or 6.8 or 7 or 8 or 9 or 10 µM and no more than about 50 or 44 or 40 or 35 µM. |
| (e) Vitamin B6 (which in one aspect is pyridoxine) | from about 4.0 µM to about 32 µM; from about 4.0 µM to about 30 µM; from about 4.0 µM to about 25 µM; from about 4.0 µM to about 20 µM; from about 4.0 µM to about 15 µM; from about 4.0 µM to about 10 µM; from about 10 µM to about 32 µM; from about 15 µM to about 32 µM; from about 20 µM to about 32 µM; from about 25 µM to about 32 µM; from about 30 µM to about 32 µM; from about 5.0 µM to about 30 µM; from about 10 µM to about 25 µM; from about 15 µM to about 20 µM; from about 4.5 µM to about 30 µM; from about 5.0 µM to about 28 µM; from about 5.5 µM to about 26 µM; about any of 4.0 or 4.5 or 5.0 or 10 or 15 or 20 or 30 or 32 µM; at least about any of 4.0 or 4.5 or 5.0 or 6.0 µM and no more than about 32 or 30 or 28 µM. |
| (f) Vitamin B7 | from about 0.016 µM to about 0.18 µM; from about 0.016 µM to about 0.15 µM; from about 0.016 µM to about 0.10 µM; from about 0.016 µM to about 0.05 µM; from about 0.05 µM to about 0.18 µM; from about 0.10 µM to about 0.18 µM; from about 0.15 µM to about 0.18 µM; from about 0.018 µM to about 0.16 µM; from about 0.02 µM to about 0.14 µM; from about 0.022 µM to about 0.12 µM; from about 0.024 µM to about 0.10 µM; from about 0.026 µM to about 0.08 µM; from about 0.028 µM to about 0.06 µM; from about 0.030 µM to about 0.04 µM; about any of 0.016 or 0.018 or 0.02 or 0.05 or 0.10 or 0.12 or 0.14 or 0.16 or 0.18 µM; at least about any of 0.016 or 0.018 or 0.02 or 0.025 µM and no more than about 0.18 or 0.16 or 0.14 or 0.12 µM. |
| (g) Vitamin B9 | from about 3.0 µM to about 25 µM; from about 3.0 µM to about 20 µM; from about 3.0 µM to about 15 µM; from about 3.0 µM to about 10 µM; from about 3.0 µM to about 5.0 µM; from about 5.0 µM to about 25 µM; from about 10 µM to about 25 µM; from about 15 µM to about 25 µM; from about 20 µM to about 25 µM; from about 5.0 µM to about 20 µM; from about 10 µM to about 15 µM; from about 3.4 µM to about 22 µM; from about 3.8 µM to about 19 µM; from about 4.2 µM to about 16 µM; about any of 3.0 or 3.4 or 4.0 or 5.0 or 10 or 15 or 20 or 22 or 25 µM; at least about any of 3 or 3.4 or 4.0 or 5.0 µM and no more than about 25 or 22 or 22 µM. |
| (h) Vitamin B12 | from about 0.18 µM to about 2.0 µM; from about 0.18 µM to about 1.75 µM; from about 0.18 µM to about 1.5 µM; from about 0.18 µM to about 1.25 µM; from about 0.18 µM to about 1.0 µM; from about 0.18 µM to about 0.5 µM; from about 0.25 µM to about 2.0 µM; from about 0.5 µM to about 2.0 µM; from about 0.75 µM to about 2.0 µM; from about 1.0 to about 2.0 µM; from about 1.25 µM to about 2.0 µM; from about 1.5 µM to about 2.0 µM; from about 1.75 µM to about 2.0 µM; from about 0.25 µM to about 1.75 µM; from about 0.5 µM to about 1.5 µM; from about 0.75 µM to about 1.25 µM; from about 0.2 µM to about 1.5 µM; from about 0.4 µM to about 1.0 µM; about any of 0.18 or 0.2 or 0.5 or 1.0 or 1.5 or 2.0 µM; at least about any of 0.18 or 0.2 or 0.4 µM and no more than about 2.0 or 1.75 or 1.5 or 1.25 µM. |
| (i) an iron source such as ferric citrate | from about 2 µM to about 80 µM; from about 2 µM to about 40 µM; from about 2 µM to about 30 µM; from about 2 µM to about 25 µM; from about 2 µM to about 20 µM; from about 2 µM to about 15 µM; from about 2 µM to about 10 µM; from about 10 µM to about 50 µM; from about 15 µM to about 50 µM; from about 20 µM to about 50 µM; from about 25 µM to about 50 µM; from about 30 µM to about 50 µM; from about 40 µM to about 50 µM; from about 10 µM to about 40 µM; from about 10 µM to about 30 µM; from about 10 µM to about 25 µM; from about 15 µM to about 25 µM; from about 15 µM to about 20 µM; about any of 5 or 10 or 15 or 20 or 25 or 30 or 35 or 40 µM; at least about any of 2 or 5 or 10 or 12.5 or 15 or 17.5 and no more than about 30 or 25 or 20 µM. |
| (j) Cystine | from about 0.7 mM to about 2.5 mM; from about 0.7 mM to about 2.0 mM; from about 0.8 mM to about 2.5 mM; from about 0.8 mM to about 2.25 mM; from about 0.8 mM to about 2.0 mM; from about 0.8 mM to about 1.75 mM; from about 0.8 mM to about 1.5 mM; from about 0.8 mM to about 1.25 mM; from about 0.8 mM to about 1.0 mM; from about 1.0 mM to about 2.5 mM; from about 1.0 mM to about 1.6 mM; from about 1.2 mM to about 1.4 mM; from about 1.25 mM to about 2.5 mM; from about 1.5 mM to about 2.5 mM; from about 1.75 mM to about 2.5 mM; from about 2.0 mM to about 2.5 mM; from about 2.25 mM to about 2.5 mM; from about 0.9 mM to about 2.0 mM; from about 0.8 mM to about 1.75 mM; from about 0.9 mM to about 1.5 mM; from about 1.0 mM to about 1.25 mM; about any of 0.7 or 0.8 or 0.9 or 1.0 or 1.1 or 1.2 or 1.3 or 1.4 or 1.5 or 1.6 mM; any of 0.7 mM, 0.75 mM, 0.8 mM, 0.85 mM, 0.9 mM, 0.95 mM, 1.0 mM, 1.05 mM, 1.1 mM, 1.15 mM, 1.2 mM, 1.25 mM, 1.3 mM, 1.35 mM, 1.4 mM, 1.5 mM, 1.55 mM, 1.6 mM, 1.65 mM, 1.7 mM, 1.75 mM, 1.8 mM, 1.85 mM, 1.9 mM or 1.95 mM or 2.0 mM; at least about any of 0.7 or 0.8 or 0.9 or 1.0 or 1.1 mM and no more than about 2.0 or 1.75 or 1.6 or 1.5 or 1.4 mM. |
| (k) Cysteine | from about 0.5 mM to about 2.0 mM; from about 0.5 mM to about 1.75 mM; from about 0.5 mM to about 1.5 mM; from about 0.5 mM to about 1.25 mM; from about 0.5 mM to about 1.0 mM; from about 0.5 mM to about 0.75 mM; from about 0.6 mM to about 2.0 mM; from about 0.8 mM to about 2.0 mM; from about 1.0 mM to about 2.0 mM; from about 1.25 mM to about 2.0 mM; from about 1.5 mM to about 2.0 mM; from about 1.75 mM to about 2.0 mM; about any of 0.5 or 0.6 or 0.7 or 0.8 or 0.9 or 1.0 or 1.1 or 1.2 or 1.3 or 1.4 or 1.5 or 1.6 or 1.7 or 1.8 or 1.9 or 2.0 mM; any of 0.5 mM, 0.55 mM, 0.6 mM, 0.65 mM, 0.7 mM, 0.75 mM, 0.8 mM, 0.85 mM, 0.9 mM, 0.95 mM, 1.0 mM, 1.05 mM, 1.1 mM, 1.15 mM, 1.2 mM, 1.25 mM, 1.3 mM, 1.35 mM, 1.4 mM, 1.5 mM, 1.55 mM, 1.6 mM, 1.65 mM, 1.7 mM, 1.75 mM, 1.8 mM, 1.85 mM, 1.9 mM, 1.95 mM, or 2.0 mM; at least about any of 0.5 or 0.6 or 0.7 or 0.8 or 0.9 or 1.0 or 1.1 mM and no more than about 2.0 or 1.8 or 1.75 or 1.6 or 1.5 or 1.4 mM. |

A medium provided herein (*e*.*g*., a CDM or a chemically undefined medium) in one variation comprises cysteine and is free of cystine. A cystine-free medium may be employed in the basal medium or in the feed medium. In one variation, the feed medium is free of cystine and comprises cysteine. In one variation, a feed medium comprises from about 80 mg/L to about 1500 mg/L cysteine. In another variation, a feed medium comprises from about 0.5 mM to about 2.0 mM cysteine. For example, a basal medium comprising from about 300 mg/L (in some aspects, 200 mg/L) to about 1200 mg/L cystine; from about 0.05 mg/L to about 1.0 mg/L vitamin B2; from about 0.05 mg/L to about 10.0 mg/L vitamin B6 (which in one aspect is pyridoxine); from about 0.05 mg/L to about 12.0 mg/L vitamin B9; and from about 0.05 mg/L to about 2.5 mg/L vitamin B12, which in one aspect can further comprise any one or more of: (1) vitamin B1 (which in one aspect is present at a concentration from about 2.0 µM to about 14.0 µM), (2) vitamin B3 (which in one aspect is present at a concentration from about 11.0 µM to about 72.0 µM), (3) vitamin B5 (which in one aspect is present at a concentration from about 6.8 µM to about 44.0 µM), and (4) vitamin B7 (which in one aspect is present at a concentration from about 0.02 µM to about 0.24 µM) can be supplemented with a feed medium comprising from about 80 mg/L to about 1500 mg/L cysteine (in some aspects, 0.5 mM to about 2.0 mM cysteine). In another variation, a basal medium comprising from about 0.8 mM (in some aspects, 0.7 mM) to about 2.5 mM cystine; from about 0.11 µM to about 0.72 µM vitamin B2; from about 4.5 µM to about 30 µM vitamin B6 (which in one aspect is pyridoxine); from about 3.4 µM to about 22 µM vitamin B9; and from about 0.2 µM to about 1.5 µM vitamin B12 , which in one aspect can further comprise any one or more of: (1) vitamin B1 (which in one aspect is present at a concentration from about 2.0 µM to about 14.0 µM), (2) vitamin B3 (which in one aspect is present at a concentration from about 11.0 µM to about 72.0 µM), (3) vitamin B5 (which in one aspect is present at a concentration from about 6.8 µM to about 44.0 µM), and (4) vitamin B7 (which in one aspect is present at a concentration from about 0.02 µM to about 0.24 µM) can be supplemented with a feed medium comprising from about 80 mg/L to about 1500 mg/L cysteine (in some aspects, 0.5 mM to about 2.0 mM cysteine). In any variation herein, the basal medium can further comprise an iron source, such as ferric citrate or ferrous sulfate (which in one aspect is present at a concentration of from about 11.0 µM to about 36.0 µM). In any variation herein, the basal media can further comprise hydrocortisone (which in one aspect is present at a concentration from about 0.05 µM to about 0.5 µM).

A medium provided herein (*e*.*g*., a CDM or a chemically undefined medium) in one variation comprises ferric citrate and is free of ferrous sulfate. A ferrous sulfate-free medium may be employed in the basal medium or in the feed medium. In one variation, the basal medium is free of ferrous sulfate and comprises ferric citrate.

In a particular variation, a medium provided herein is free from cysteine and ferrous sulfate. In one such variation, the medium is free from cysteine and ferrous sulfate and comprises cystine and/or ferric citrate.

A medium provided herein in one variation is free of hydrocortisone. In another variation, a medium comprises hydrocortisone. In one aspect, the medium comprises hydrocortisone and is free of cysteine and/or ferrous sulfate. In another aspect, the medium comprises hydrocortisone and cystine and ferric citrate. In a particular variation, the medium comprising hydrocortisone is the basal medium. In one variation, a basal medium comprises from about 0.8 mM (in some aspects, 0.7 mM) to about 2.5 mM cystine; from about 2 µM to about 80 µM ferric citrate; from about 0.05 µM to about 0.5 µM hydrocortisone, and where the basal medium may further comprise one or more of the following components: (1) vitamin B2 (which in one aspect is present at a concentration of from about 0.05 mg/L to about 1.0 mg/L); (2) vitamin B6 (which in one aspect is present at a concentration of from about 0.05 mg/L to about 10.0 mg/L); (3) vitamin B9 (which in one aspect is present at a concentration of from about 0.05 mg/L to about 12.0 mg/L); and (4) vitamin B12 (which in one aspect is present at a concentration of from about 0.05 mg/L to about 2.5 mg/L). In another variation, a basal medium comprises from about 300 mg/L (in some aspects, 200 mg/L) to about 1200 mg/L cystine; from about 2 µM to about 80 µM ferric citrate; and from about 0.05 µM to about 0.5 µM hydrocortisone, and where the basal medium may further comprise one or more of the following components: (1) vitamin B2 (which in one aspect is present at a concentration of from about 0.05 mg/L to about 1.0 mg/L); (2) vitamin B6 (which in one aspect is present at a concentration of from about 0.05 mg/L to about 10.0 mg/L); (3) vitamin B9 (which in one aspect is present at a concentration of from about 0.05 mg/L to about 12.0 mg/L); and (4) vitamin B12 (which in one aspect is present at a concentration of from about 0.05 mg/L to about 2.5 mg/L). In any of the variations herein, the basal medium may further comprise any one or more of: (1) vitamin B1 (which in one aspect is present at a concentration from about 2.0 µM to about 14.0 µM), (2) vitamin B3 (which in one aspect is present at a concentration from about 11.0 µM to about 72.0 µM), (3) vitamin B5 (which in one aspect is present at a concentration from about 6.8 µM to about 44.0 µM), and (4) vitamin B7 (which in one aspect is present at a concentration from about 0.02 µM to about 0.24 µM).

A method of preparing a cell culture medium for use in culturing a cell is also provided, wherein the method comprises combining any one or more media components selected from the group consisting of (a) cystine and/or cysteine; (b) vitamin B2, (c) vitamin B6 (pyridoxine and/or pyridoxal), (d) vitamin B9, (e) vitamin B12, (f) an iron source such as ferric citrate and (g) hydrocortisone, wherein each of (a)-(g) are provided in amounts as described in Table 1. Also provided herein, is a method of preparing a cell culture medium for use in culturing a cell, wherein the method comprises combining any one or more media components selected from the group consisting of (a) vitamin B1; (b) vitamin B2; (c) vitamin B3; (d) vitamin B5; (e) vitamin B6; (f) vitamin B7; (g) vitamin B9; (h) vitamin B12; (i) an iron source such as ferric citrate; (j) cystine; and (k) cysteine in amounts as described in Table 1A. In a variation, the method comprises adding any one or more media components as described herein (*e*.*g*., Table 1 or Table 1A) to a composition suitable for cell culture, wherein the one or more media components may be added to the composition sequentially or simultaneously. In a further variation, the method comprises combining any one or more media components as described herein (*e*.*g*., Table 1 or Table 1A) in a composition suitable for cell culture at a first period of time and wherein the method further comprises adding an amount of one or more media components at a second period of time, such as at least once, at least twice, at least three times, at least four time, at least five times, at least six times, at least seven times, etc. during a cell culture cycle. In some embodiments, a cell culture cycle is at least 1 day, 2 days, 3 days, 4 days, 5 days, 6 days, 7 days, 8 days, 9 days, 10 days, 11 days, 12 days, 13 days, 14 days, 15 days, 16 days, 17 days, 18, days, 19 days, 20 days, or any amount of days wherein the cells may remain in cell culture while still remaining viable. In one variation of a method of preparing a cell culture medium for use in culturing a cell, cystine is added in an amount to provide from about 300 mg/L (in some aspects, 200 mg/L) to about 1200 mg/L cystine, vitamin B2 is added in an amount to provide from about 0.05 mg/L to about 1.0 mg/L vitamin B2, vitamin B6 is added in an amount to provide from about 0.05 mg/L to about 10.0 mg/L vitamin B6, vitamin B9 is added in an amount to provide from about 0.05 mg/L to about 12.0 mg/L vitamin B9, and vitamin B12 is added in an amount to provide from about 0.05 mg/L to about 2.5 mg/L vitamin B12 in the cell culture medium. In another variation of a method of preparing a cell culture medium for use in culturing a cell, cystine is added in an amount to provide from about 0.8 mM (in some aspects, 0.7 mM) to about 2.5 mM cystine, vitamin B2 is added in an amount to provide from about 0.11 µM to about 0.72 µM vitamin B2, vitamin B6 is added in an amount to provide from about 4.5 µM to about 30.0 µM vitamin B6, vitamin B9 is added in an amount to provide from about 3.4 µM to about 22.0 µM vitamin B9, and vitamin B12 is added in an amount to provide from about 0.2 µM to about 1.5 µM vitamin B12 in the cell culture medium.

In some variations herein, the cell culture medium is a basal cell culture medium. In other variations herein, the cell culture medium is a feed cell culture medium. In some variations herein, the cell culture medium is a basal cell culture medium comprising any one or more media components selected from the group consisting of (a) cystine; (b) vitamin B2, (c) vitamin B6 (pyridoxine and/or pyridoxal), (d) vitamin B9, (e) vitamin B12, (f) an iron source such as ferric citrate and (g) hydrocortisone in amounts as described in Table 1, and where the basal cell culture medium is supplemented (*e*.*g*., at a period of time following initiation of a cell culture cycle, such as any one of at least two times, at least three times, at least four times, at least five times, at least six times, at least seven times, etc. of a cell culture cycle) with a feed cell culture medium comprising (a) cysteine in amounts as described in Table 1. In some variations herein, the cell culture medium is a basal cell culture medium comprising any one or more media components selected from the group consisting of (a) vitamin B1; (b) vitamin B2; (c) vitamin B3; (d) vitamin B5; (e) vitamin B6; (f) vitamin B7; (g) vitamin B9; (h) vitamin B12; (i) an iron source such as ferric citrate; and (j) cystine in amounts as described in Table 1A, and where the basal cell culture medium is supplemented (*e*.*g*., at a period of time following initiation of a cell culture cycle, such as any one of at least two times, at least three times, at least four times, at least five times, at least six times, at least seven times, etc. of a cell culture cycle) with a feed cell culture medium comprising (k) cysteine in amounts as described in Table 1A.

As would be understood by the skilled artisan, the cell culture media detailed herein may comprise other components (*e*.*g*., besides one or more of cystine, vitamin B1, vitamin B2, vitamin B3, vitamin B5, vitamin B6, vitamin B7, vitamin B9, and vitamin B12, iron, and optionally hydrocortisone) that are useful for cell culture. For example, it is understood that the cell culture media may comprise additional components such as amino acids (e.g., glutamine, arginine, or asparagine), vitamins (including but not limited to ascorbic acid), trace elements, transition metals (including but not limited to nickel, copper, or zinc), and other media components such as, but not limited to, hydrolysate derived from an animal and/or plant. Any media provided herein may also be supplemented with hormones and/or other growth factors (such as insulin, transferrin, or epidermal growth factor), ions (such as sodium, chloride, calcium, magnesium, and phosphate), buffers (such as HEPES), nucleosides (such as adenosine and thymidine), and glucose or an equivalent energy source. Additional cell culture media components, such as those listed herein, may be included in the cell culture medium at appropriate concentrations at different times during a cell culture cycle that would be known to those skilled in the art.

A medium provided herein in one aspect results in one or more favorable product quality attributes when used in a method of producing a polypeptide as compared to quality attributes of the polypeptide when produced in a different medium. Production of a protein product (*e*.*g*., an antibody product) with an altered charge variant distribution may impact the quality attributes of a protein product, such as the protein products' color. In addition, reactive oxygen species (ROS) formed through the use of certain media components may oxidize specific amino acids and produce oxidation products. The presence of such product variants may also alter the product quality attributes of a protein product, such as color. The color of a composition comprising a polypeptide produced with a media detailed herein (including a composition comprising at least 100 mg/mL or 125 mg/mL or 150 mg/mL of polypeptide, such as an antibody) in one aspect has a color reference standard value as described in Table 2. In particular variations, the color of a composition comprising a polypeptide produced with a media detailed herein (including a composition comprising at least 100 mg/mL or 125 mg/mL or 150 mg/mL of polypeptide, such as an antibody) in one aspect has a color reference standard value selected from the group consisting of B3, B4, B5, B6, B7, B8, B9, BY3, BY4, BY5, BY6, BY7, Y3, Y4, Y5, Y6, Y7, GY3, GY4, GY5, GY6, GY7, R3, R4, R5, R6 and R7. In another aspect, the color of a composition comprising a polypeptide produced with a media detailed herein (including a composition comprising at least 1 mg/mL or 25 mg/mL or 50 mg/mL or 75 mg/mL of polypeptide, such as an antibody) in one aspect has a color reference standard value as described in Table 2. In some variations, the color of a composition comprising a polypeptide produced with a media detailed herein (including a composition comprising at least 1 mg/mL or 25 mg/mL or 50 mg/mL or 75 mg/mL of polypeptide, such as an antibody) in one aspect has a color reference standard value selected from the group consisting of B3, B4, B5, B6, B7, B8, B9, BY3, BY4, BY5, BY6, BY7, Y3, Y4, Y5, Y6, Y7, GY3, GY4, GY5, GY6, GY7, R3, R4, R5, R6 and R7. See USP-24 Monograph 631 Color and Achromaticity. *United States Pharmacopoeia Inc*., 2000, p. 1926-1927 and Council of Europe. European Pharmacopoeia, 2008, 7th Ed. P.22 for a description of color reference values brown (B), brownish-yellow (BY), yellow (Y), greenish-yellow (GY), or red (R). In one variation, a medium as provided herein reduces the presence of charge variants (*e*.*g*., acidic charge variants) when used in a method of producing a polypeptide as compared to charge variants (*e*.*g*., acidic charge variants) obtained when the polypeptide is produced in a different medium. In another variation, a medium reduces the presence of reactive oxygen species when used in a method of producing a polypeptide as compared to reactive oxygen species obtained when the polypeptide is produced in a different medium. In another variation, a medium reduces the presence of contaminants when used in a method of producing a polypeptide as compared to contaminants obtained when the polypeptide is produced in a different medium.

As described herein, various methods (such as methods of culturing cells and methods of producing polypeptides) are provided which employ cell culture media described in this section and elsewhere.

### Methods

The cell culture media detailed herein (including any CDM or chemically undefined media detailed herein) can be used in a method of culturing cells to produce polypeptides, including particular antibodies. The medium may be used in a method of culturing cells, whether by batch culture, fed batch culture or perfusion culture, and can be used in a method of producing an antibody including any aspects or variations or embodiments of antibody as described herein.

Methods of growing cells (*i*.*e*., culturing cells) by contacting the cells with a cell culture medium as detailed herein are provided. In one variation, the method comprises contacting a cell with a cell culture medium comprising one or more medium components as described in Table 1 (*e*.*g*., a medium comprising components (a), (b), (c), (d) and (e) or a medium comprising components (a), (f) and (g) or a medium comprising each of components (a)-(g) in any of the amounts listed in Table 1). In one variation, the method comprises contacting a cell with a cell culture medium comprising one or more medium components as described in Table 1A (*e*.*g*., a medium comprising components (a)-(j), or a medium comprising components (a), (b), (c), (d), (e), (f), (g), (h), and (i), or a medium comprising components (b), (e), (g), (h) and (j) or a medium comprising only one of components (a)-(j) in any of the amounts listed in Table 1A). In a particular variation of a method of growing a cell (*i*.*e*., culturing a cell), the cell culture medium is a CDM. In one aspect of the methods, the cells are grown (*i*.*e*., cultured) in a CDM basal medium. In another particular variation of a method of growing a cell (*i*.*e*., culturing a cell), the cell culture medium is a chemically undefined cell culture medium. In one aspect of the methods, the cells are grown (*i*.*e*., cultured) in a chemically undefined cell culture basal medium. In some aspects, the cells are contacted with the cell culture medium during the cells' growth phase. In some aspects, the cells are contacted with the cell culture medium during the cells' production phase. In some aspects, the method further comprises a step of adding cysteine to the cell culture medium. In a further aspect, the cysteine is added in an amount to provide from about 80 mg/L to about 1500 mg/L cysteine in the cell culture medium. In another further aspect, the cysteine is added in an amount to provide about 1500 mg/L cysteine in the cell culture medium. In yet another further aspect, the cysteine is added in an amount to provide about 140 mg/L cysteine in the cell culture medium. In another further aspect, the cysteine is added in an amount to provide from about 0.5 mM to about 2.0 mM cysteine in the cell culture medium. In yet another further aspect, the cysteine is added in an amount to provide about 0.8 mM cysteine in the cell culture medium.

Methods of producing a polypeptide by growing in a cell culture medium (*i*.*e*., culturing in a cell culture medium) a cell comprising an isolated nucleic acid encoding the polypeptide are also provided, wherein: (a) the cell expresses the polypeptide and (b) the chemically defined cell culture medium comprises one or more medium components as described in Table 1 (*e*.*g*., a medium comprising components (a), (b), (c), (d) and (e) or a medium comprising components (a), (f) and (g) or a medium comprising each of components (a)-(g) in any of the amounts listed in Table 1). In another variation, provided herein are methods of producing a polypeptide by growing in a cell culture medium (*i*.*e*., culturing in a cell culture medium) a cell comprising an isolated nucleic acid encoding the polypeptide, wherein: (a) the cell expresses the polypeptide and (b) the cell culture medium comprises one or more medium components as described in Table 1A (*e*.*g*., a medium comprising components (a)-(j), or a medium comprising components (a), (b), (c), (d), (e), (f), (g), (h), and (i), or a medium comprising components (b), (e), (g), (h) and (j) or a medium comprising only one of components (a)-(j) in any of the amounts listed in Table 1A). In a particular variation of a method of producing a polypeptide by growing in a cell culture medium (*i*.*e*., culturing in a cell culture medium) a cell comprising an isolated nucleic acid encoding the polypeptide, the cell culture medium is a CDM. In one aspect of the methods, the cells are grown (*i.e*., cultured) in a CDM basal medium. In another particular variation of a method of producing a polypeptide by growing in a cell culture medium (*i*.*e*., culturing in a cell culture medium) a cell comprising an isolated nucleic acid encoding the polypeptide, the cell culture medium is a chemically undefined cell culture medium. In one aspect of the methods, the cells are grown (*i*.*e*., cultured) in a chemically undefined cell culture basal medium. In some aspects, the culturing is during the cell's growth phase. In some aspects, the culturing is during the cell's production phase. In some variations, the method further comprises a step of adding cysteine to the cell culture medium. In a further variation, the cysteine is added in an amount to provide from about 80 mg/L to about 1500 mg/L cysteine in the cell culture medium. In another further variation, the cysteine is added in an amount to provide about 1500 mg/L cysteine in the cell culture medium. In yet another further variation, the cysteine is added in an amount to provide about 140 mg/L cysteine in the cell culture medium. In another further variation, the cysteine is added in an amount to provide from about 0.5 mM to about 2.0 mM cysteine in the cell culture medium. In yet another further variation, the cysteine is added in an amount to provide about 0.8 mM cysteine in the cell culture medium.

Methods of administering a polypeptide as detailed herein are also provided. For example, a method is provided for administering to an individual a formulation comprising a polypeptide, wherein the formulation has the polypeptide at a concentration greater than at least 100 mg/mL, at least 125 mg/mL, or at least 150 mg/mL and has a color intensity value greater than B3, B4, B5, B6, B7, B8, or B9 as measured by the COC assay. In some aspects, the color intensity value as determined by the COC assay can be any one of, but not limited to, B, BY, Y, GY, or R, wherein higher values indicate a lighter color intensity. In another example, a method is provided for administering to an individual a formulation comprising a polypeptide, wherein the formulation has the polypeptide at a concentration greater than at least 100 mg/mL, at least 125 mg/mL, or at least 150 mg/mL and has a color intensity value less than a color intensity value of a reference solution as measured by a color assay (*e*.*g*., the Total Color assay or the NIFTY assay). Formulations of the polypeptides can be administered by any suitable means, including parenteral, intrapulmonary, and intranasal, and, if desired for local treatment, intralesional administration. Parenteral infusions include intramuscular, intravenous, intraarterial, intraperitoneal, or subcutaneous administration. Dosing can be by any suitable route, e.g. by injections, such as intravenous or subcutaneous injections, depending in part on whether the administration is brief or chronic. Accordingly, polypeptide-containing formulations as provided herein may be suitable for injection, such as subcutaneous injection into an individual (*e*.*g*., subcutaneous injection into a human). In some aspects, a polypeptide-containing formation suitable for injection (*e*.*g*., suitable for subcutaneous injection) is at a concentration greater than at least 100 mg/mL, at least 125 mg/mL, or at least 150 mg/mL and has a color intensity value greater than B3, B4, B5, B6, B7, B8, or B9 as measured by the COC assay. In some aspects, the color intensity value as determined by the COC assay can be any one of, but not limited to, B, BY, Y, GY, or R, wherein higher values indicate a lighter color intensity. In some aspects, a polypeptide-containing formation suitable for injection (*e*.*g*., suitable for subcutaneous injection) is at a concentration greater than at least 100 mg/mL, at least 125 mg/mL, or at least 150 mg/mL and has a color intensity value less than a color intensity value of a reference solution as measured by a color assay (*e*.*g*., the Total Color assay or the NIFTY assay). Various dosing schedules including but not limited to single or multiple administrations over various time-points, bolus administration, and pulse infusion are contemplated herein.

Other methods are provided throughout, such as in the Brief Summary of the Invention and elsewhere.

### Cells

The methods and compositions provided may employ any cell that is suitable for growth and/or production of a polypeptide (*e*.*g*., an antibody) in a medium described herein, including animal, yeast or insect cells. In one aspect, a cell of the methods and compositions is any mammalian cell or cell type suitable to cell culture and to expression of polypeptides. The methods provided herein (*e*.*g*., methods of growing a cell (*i*.*e*., culturing a cell) and/or producing a polypeptide) and compositions may therefore employ any suitable type of cell, including an animal cell. In one aspect, the methods and compositions employ a mammalian cell. The methods and compositions may also employ hybridoma cells. In one variation, the mammalian cell is a non-hybridoma mammalian cell, which has been transformed with exogenous isolated nucleic acid encoding a desired polypeptide, such as an antibody, antibody fragment (including a ligand-binding fragment), and chimeric antibodies. In one variation, the methods and compositions employ mammalian cells selected from the group consisting of human retinoblasts (PER.C6 (CruCell, Leiden, The Netherlands)); monkey kidney CV1 line transformed by SV40 (COS-7, ATCC CRL 1651); human embryonic kidney line (293 or 293 cells subcloned for growth in suspension culture, Graham et al., J. Gen Virol., 36:59 (1977)); baby hamster kidney cells (BHK, ATCC CCL 10); Chinese hamster ovary cells/-DHFR (CHO, Urlaub and Chasin, Proc. Natl. Acad. Sci. USA, 77:4216 (1980)); mouse sertoli cells (TM4, Mather, Biol. Reprod., 23:243-251 (1980)); monkey kidney cells (CV1 ATCC CCL 70); African green monkey kidney cells (VERO-76, ATCC CRL-1 587); human cervical carcinoma cells (HeLa, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather et al., Annals N.Y. Acad. Sci., 383:44-68 (1982)); MRC 5 cells; FS4 cells; and a human hepatoma line (Hep G2). In a particular variation, the methods and compositions employ CHO cells. In a particular variation, the culturing of CHO cell lines and expression of polypeptides (*e*.*g*., antibodies) from CHO cell lines is employed. The polypeptides (*e*.*g*., antibodies) may be secreted into the medium (*e*.*g*., CDM) from which the polypeptides may be isolated and/or purified or the polypeptide may be released into the medium by lysis of a cell comprising an isolated nucleic acid encoding the polypeptide.

Methods, vectors, and host cells suitable for adaptation to the synthesis of the polypeptide of interest in recombinant vertebrate cell culture are known in the art and are described, for example, in Gething et al., Nature, 293:620-625 (1981); Mantei et al., Nature, 281:40-46 (1979); Levinson et al.; EP 117,060; and EP 117,058. A particularly useful plasmid for mammalian cell culture expression of the polypeptide is pRK5 (EP pub. no. 307,247) or pSVI6B (PCT pub. no. WO 91/08291 published Jun. 13, 1991).

Host cells are transformed with expression or cloning vectors and cultured in nutrient media modified as appropriate for inducing promoters, selecting transformants, or amplifying the genes encoding the desired sequences. For mammalian cells, the calcium phosphate precipitation method of Graham and van der Erb, Virology, 52:456-457 (1978) or the lipofectamine.TM. (Gibco BRL) Method of Hawley-Nelson, Focus 15:73 (1193) are preferred. General aspects of mammalian cell host system transformations are known in the art and have been described, for example, by Axel in U.S. Pat. No. 4,399,216 issued Aug. 16, 1983. For various techniques for transforming mammalian cells, see *e.g*., Keown et al., Methods in Enzymology (1989), Keown et al., Methods in Enzymology, 185:527-537 (1990), and Mansour et al., Nature, 336:348-352 (1988).

The methods and compositions also embrace the use of hybridomas which secrete monoclonal antibodies in cell culture. Monoclonal antibodies are prepared by recovering immune cells (typically spleen cells or lymphocytes from lymph node tissue) from immunized animals and immortalizing the cells in conventional fashion, *e*.*g*., by fusion with myeloma cells or by Epstein-Barr (EB)-virus transformation and screening for clones expressing the desired antibody. The hybridoma technique described originally by Kohler and Milstein, Eur. J. Immunol., 6:511 (1976), and also described by Hammerling et al., In: Monoclonal Antibodies and T-Cell Hybridomas, Elsevier, N.Y., pp. 563-681 (1981) has been widely applied to produce hybrid cell lines that secrete high levels of monoclonal antibodies against many specific antigens.

### Polypeptides

The polypeptides produced by the compositions (cells) and methods detailed herein and present in the compositions provided herein may be homologous to the host cell, or preferably, may be exogenous, meaning that they are heterologous, i.e., foreign, to the host cell being utilized, such as a human protein produced by a Chinese hamster ovary cell, or a yeast polypeptide produced by a mammalian cell. In one variation, the polypeptide is a mammalian polypeptide (such as an antibody) directly secreted into the medium by the host cell. In another variation, the polypeptide is released into the medium by lysis of a cell comprising an isolated nucleic acid encoding the polypeptide.

In one variation, the polypeptide is a sequence of amino acids for which the chain length is sufficient to produce the higher levels of tertiary and/or quaternary structure. In one aspect, the polypeptide will have a molecular weight of at least about 5-20 kD, alternatively at least about 15-20 kD, preferably at least about 20 kD.

Any polypeptide that is expressible in a host cell may be produced in accordance with the present disclosure and may be present in the compositions provided. The polypeptide may be expressed from a gene that is endogenous to the host cell, or from a gene that is introduced into the host cell through genetic engineering. The polypeptide may be one that occurs in nature, or may alternatively have a sequence that was engineered or selected by the hand of man. An engineered polypeptide may be assembled from other polypeptide segments that individually occur in nature, or may include one or more segments that are not naturally occurring.

Polypeptides that may desirably be expressed in accordance with the present invention will often be selected on the basis of an interesting biological or chemical activity. For example, the present invention may be employed to express any pharmaceutically or commercially relevant enzyme, receptor, antibody, hormone, regulatory factor, antigen, binding agent, etc.

Various polypeptides may be produced according to the methods provided herein, and present in the compositions provided herein. Examples of bacterial polypeptides include, *e*.*g*., alkaline phosphatase and .beta.-lactamase. Examples of mammalian polypeptides include molecules such as renin, a growth hormone, including human growth hormone; bovine growth hormone; growth hormone releasing factor; parathyroid hormone; thyroid stimulating hormone; lipoproteins; alpha-1-antitrypsin; insulin A-chain; insulin B-chain; proinsulin; follicle stimulating hormone; calcitonin; luteinizing hormone; glucagon; clotting factors such as factor VIIIC, factor IX, tissue factor, and von Willebrands factor; anti-clotting factors such as Protein C; atrial natriuretic factor; lung surfactant; a plasminogen activator, such as urokinase or human urine or tissue-type plasminogen activator (t-PA); bombesin; thrombin; hemopoietic growth factor; tumor necrosis factor-alpha and -beta; enkephalinase; RANTES (regulated on activation normally T-cell expressed and secreted); human macrophage inflammatory protein (MIP-1-alpha); a serum albumin such as human serum albumin; mullerian-inhibiting substance; relaxin A-chain; relaxin B-chain; prorelaxin; mouse gonadotropin-associated peptide; a microbial protein, such as beta-lactamase; DNase; inhibin; activin; vascular endothelial growth factor (VEGF); receptors for hormones or growth factors; integrin; protein A or D; rheumatoid factors; a neurotrophic factor such as bone-derived neurotrophic factor (BDNF), neurotrophin-3, -4, -5, or -6 (NT-3, NT-4, NT-5, or NT-6), or a nerve growth factor such as NGF-.beta.; platelet-derived growth factor (PDGF); fibroblast growth factor such as aFGF and bFGF; epidermal growth factor (EGF); transforming growth factor (TGF) such as TGF-alpha and TGF-beta, including TGF-.beta. 1, TGF-.beta.2, TGF-.beta.3, TGF-.beta.4, or TGF-.beta.5; insulin-like growth factor-I and -II (IGF-I and IGF-II); des(1-3)-IGF-I (brain IGF-I), insulin-like growth factor binding proteins; CD proteins such as CD-3, CD-4, CD-8, and CD-19; erythropoietin; osteoinductive factors; immunotoxins; a bone morphogenetic protein (BMP); an interferon such as interferon-alpha, -beta, and -gamma; colony stimulating factors (CSFs), *e.g.*, M-CSF, GM-CSF, and G-CSF; interleukins (ILs), *e.g.*, IL-1 to IL-10; superoxide dismutase; T-cell receptors; surface membrane proteins; decay accelerating factor; viral antigen such as, for example, a portion of the AIDS envelope; transport proteins; homing receptors; addressing; regulatory proteins; antibodies; and fragments of any of the above-listed polypeptides.

Antibodies are examples of mammalian polypeptides produced according to the methods provided herein and which may be present in the compositions provided. Antibodies are a preferred class of polypeptides that exhibit binding specificity to a specific antigen. Native antibodies are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies between the heavy chains of different immunoglobulin isotypes. Each heavy and light chain also has regularly spaced intrachain disulfide bridges. Each heavy chain has at one end a variable domain (V.sub.H) followed by a number of constant domains. Each light chain has a variable domain at one end (V.sub.L) and a constant domain at its other end; the constant domain of the light chain is aligned with the first constant domain of the heavy chain, and the light chain variable domain is aligned with the variable domain of the heavy chain. Particular amino acid residues are believed to form an interface between the light and heavy chain variable domains.

Antibodies are naturally occurring immunoglobulin molecules which have varying structures, all based upon the immunoglobulin fold. For example, IgG antibodies have two "heavy" chains and two "light" chains that are disulphide-bonded to form a functional antibody. Each heavy and light chain itself comprises a "constant" (C) and a "variable" (V) region. The V regions determine the antigen binding specificity of the antibody, while the C regions provide structural support and function in non-antigen-specific interactions with immune effectors. The antigen binding specificity of an antibody or antigen-binding fragment of an antibody is the ability of an antibody to specifically bind to a particular antigen.

The antigen binding specificity of an antibody is determined by the structural characteristics of the V region. The variability is not evenly distributed across the 110-amino acid span of the variable domains. Instead, the V regions consist of relatively invariant stretches called framework regions (FRs) of 15-30 amino acids separated by shorter regions of extreme variability called "hypervariable regions" that are each 9-12 amino acids long. The variable domains of native heavy and light chains each comprise four FRs, largely adopting a β-sheet configuration, connected by three hypervariable regions, which form loops connecting, and in some cases forming part of, the β-sheet structure. The hypervariable regions in each chain are held together in close proximity by the FRs and, with the hypervariable regions from the other chain, contribute to the formation of the antigen-binding site of antibodies (see Kabat et al., Sequences of Proteins of Immunological Interest, 5th Ed. Public Health Service, National Institutes of Health, Bethesda, Md. (1991)). The constant domains are not involved directly in binding an antibody to an antigen, but exhibit various effector functions, such as participation of the antibody in antibody dependent cellular cytotoxicity (ADCC).

Each V region typically comprises three complementarity determining regions ("CDRs", each of which contains a "hypervariable loop"), and four framework regions. An antibody binding site, the minimal structural unit required to bind with substantial affinity to a particular desired antigen, will therefore typically include the three CDRs, and at least three, preferably four, framework regions interspersed there between to hold and present the CDRs in the appropriate conformation. Classical four chain antibodies have antigen binding sites which are defined by VH and VL domains in cooperation. Certain antibodies, such as camel and shark antibodies, lack light chains and rely on binding sites formed by heavy chains only. Single domain engineered immunoglobulins can be prepared in which the binding sites are formed by heavy chains or light chains alone, in absence of cooperation between VH and VL.

The term "variable" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated in three segments called hypervariable regions both in the light chain and the heavy chain variable domains. The more highly conserved portions of variable domains are called the framework regions (FRs). The variable domains of native heavy and light chains each comprise four FRs, largely adopting a β-sheet configuration, connected by three hypervariable regions, which form loops connecting, and in some cases forming part of, the β-sheet structure. The hypervariable regions in each chain are held together in close proximity by the FRs and, with the hypervariable regions from the other chain, contribute to the formation of the antigen-binding site of antibodies (see Kabat et al., Sequences of Proteins of Immunological Interest, 5th Ed. Public Health Service, National Institutes of Health, Bethesda, MD. (1991)). The constant domains are not involved directly in binding an antibody to an antigen, but exhibit various effector functions, such as participation of the antibody in antibody dependent cellular cytotoxicity (ADCC).

The term "hypervariable region" when used herein refers to the amino acid residues of an antibody that are responsible for antigen binding. The hypervariable region may comprise amino acid residues from a "complementarity determining region" or "CDR" (*e.g*., around about residues 24-34 (L1), 50-56 (L2) and 89-97 (L3) in the VL, and around about 31-35B (H1), 50-65 (H2) and 95-102 (H3) in the VH (Kabat et al., Sequences of Proteins of Immunological Interest, 5th Ed. Public Health Service, National Institutes of Health, Bethesda, Md. (1991)) and/or those residues from a "hypervariable loop" (*e.g.* residues 26-32 (L1), 50-52 (L2) and 91-96 (L3) in the VL, and 26-32 (H1), 52A-55 (H2) and 96-101 (H3) in the VH (Chothia and Lesk J. Mol. Biol. 196:901-917 (1987)).

"Framework" or "FR" residues are those variable domain residues other than the hypervariable region residues as herein defined.

Papain digestion of antibodies produces two identical antigen-binding fragments, called "Fab" fragments, each with a single antigen-binding site, and a residual "Fc" fragment, whose name reflects its ability to crystallize readily. Pepsin treatment yields an F(ab')2 fragment that has two antigen-binding sites and is still capable of cross-linking antigen.

"Fv" is the minimum antibody fragment that contains a complete antigen-recognition and antigen-binding site. This region consists of a dimer of one heavy chain and one light chain variable domain in tight, non-covalent association. It is in this configuration that the three hypervariable regions of each variable domain interact to define an antigen-binding site on the surface of the VH-VL dimer. Collectively, the six hypervariable regions confer antigen-binding specificity to the antibody. However, even a single variable domain (or half of an Fv comprising only three hypervariable regions specific for an antigen) has the ability to recognize and bind antigen, although at a lower affinity than the entire binding site.

The Fab fragment also contains the constant domain of the light chain and the first constant domain (CH1) of the heavy chain. Fab' fragments differ from Fab fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear at least one free thiol group. F(ab')2 antibody fragments originally were produced as pairs of Fab' fragments that have hinge cysteines between them. Other chemical couplings of antibody fragments are also known.

The "light chains" of antibodies (immunoglobulins) from any vertebrate species can be assigned to one of two clearly distinct types, called kappa (κ) and lambda (λ), based on the amino acid sequences of their constant domains.

Depending on the amino acid sequence of the constant domain of their heavy chains, antibodies can be assigned to different classes. There are five major classes of intact antibodies: IgA, IgD, IgE, IgG, and IgM, and several of these may be further divided into subclasses (isotypes), *e.g*., IgG1, IgG2, IgG3, IgG4, IgA, and IgA2. The heavy chain constant domains that correspond to the different classes of antibodies are called α, δ, ε, γ, and µ, respectively. The subunit structures and three-dimensional configurations of different classes of immunoglobulins are well known.

"Single-chain Fv" or "scFv" antibody fragments comprise the VH and VL domains of antibody, wherein these domains are present in a single polypeptide chain. In some embodiments, the Fv polypeptide further comprises a polypeptide linker between the VH and VL domains that enables the scFv to form the desired structure for antigen binding. For a review of scFv see Plückthun in The Pharmacology of Monoclonal Antibodies, vol. 113, Rosenburg and Moore eds., Springer-Verlag, New York, pp. 269-315 (1994).

The term "diabodies" refers to small antibody fragments with two antigen-binding sites, which fragments comprise a heavy chain variable domain (VH) connected to a light chain variable domain (VL) in the same polypeptide chain (VH - VL). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., Proc. Natl. Acad. Sci. USA, 90:6444-6448 (1993).

For the purposes herein, an "intact antibody" is one comprising heavy and light variable domains as well as an Fc region. The constant domains may be native sequence constant domains (*e*.*g*. human native sequence constant domains) or amino acid sequence variant thereof. Preferably, the intact antibody has one or more effector functions.

"Native antibodies" are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies among the heavy chains of different immunoglobulin isotypes. Each heavy and light chain also has regularly spaced intrachain disulfide bridges. Each heavy chain has at one end a variable domain (VH) followed by a number of constant domains. Each light chain has a variable domain at one end (VL) and a constant domain at its other end; the constant domain of the light chain is aligned with the first constant domain of the heavy chain, and the light chain variable domain is aligned with the variable domain of the heavy chain. Particular amino acid residues are believed to form an interface between the light chain and heavy chain variable domains.

A "naked antibody" is an antibody (as herein defined) that is not conjugated to a heterologous molecule, such as a cytotoxic moiety or radiolabel.

An antibody is directed against an antigen of interest. Preferably, the antigen is a biologically important polypeptide and administration of the antibody to an individual suffering from a disease or condition can result in a therapeutic benefit in that mammal. However, antibodies directed against nonpolypeptide antigens (such as tumor-associated glycolipid antigens; see U.S. Pat. No. 5,091,178) can also be used.

Where the antigen is a polypeptide, it may be a transmembrane molecule (*e*.*g*. receptor) or ligand such as a growth factor. Exemplary antigens include molecules such as renin; a growth hormone, including human growth hormone and bovine growth hormone; growth hormone releasing factor; parathyroid hormone; thyroid stimulating hormone; lipoproteins; alpha-1-antitrypsin; insulin A-chain; insulin B-chain; proinsulin; follicle stimulating hormone; calcitonin; luteinizing hormone; glucagon; clotting factors such as factor VIIIC, factor IX, tissue factor (TF), and von Willebrands factor; anti-clotting factors such as Protein C; atrial natriuretic factor; lung surfactant; a plasminogen activator, such as urokinase or human urine or tissue-type plasminogen activator (t-PA); bombesin; thrombin; hemopoietic growth factor; tumor necrosis factor-alpha and -beta; enkephalinase; RANTES (regulated on activation normally T-cell expressed and secreted); human macrophage inflammatory protein (MIP-1-alpha); a serum albumin such as human serum albumin; Muellerian-inhibiting substance; relaxin A-chain; relaxin B-chain; prorelaxin; mouse gonadotropin-associated peptide; a microbial protein, such as beta-lactamase; DNase; IgE; a cytotoxic T-lymphocyte associated antigen (CTLA), such as CTLA-4; inhibin; activin; vascular endothelial growth factor (VEGF); receptors for hormones or growth factors; protein A or D; rheumatoid factors; a neurotrophic factor such as bone-derived neurotrophic factor (BDNF), neurotrophin-3, -4, -5, or -6 (NT-3, NT-4, NT-5, or NT-6), or a nerve growth factor such as NGF-.beta.; platelet-derived growth factor (PDGF); fibroblast growth factor such as aFGF and bFGF; epidermal growth factor (EGF); transforming growth factor (TGF) such as TGF-alpha and TGF-beta, including TGF-.beta.1, TGF-.beta.2, TGF-.beta.3, TGF-.beta.4, or TGF-.beta.5; insulin-like growth factor-I and -II (IGF-I and IGF-II); des(1-3)-IGF-I (brain IGF-I), insulin-like growth factor binding proteins; CD proteins such as CD3, CD4, CD8, CD18, CD19, CD20, and CD40; erythropoietin; osteoinductive factors; immunotoxins; a bone morphogenetic protein (BMP); an interferon such as interferon-alpha, -beta, and - gamma; colony stimulating factors (CSFs), *e.g*., M-CSF, GM-CSF, and G-CSF; interleukins (ILs), *e.g*., IL-1 to IL-10; superoxide dismutase; T-cell receptors; surface membrane proteins; decay accelerating factor; viral antigen such as, for example, a portion of the AIDS envelope; transport proteins; homing receptors; addressins; regulatory proteins; integrins such as CD11a, CD11b, CD11c, CD18, an ICAM, VLA-4 and VCAM; a tumor associated antigen such as HER2, HER3 or HER4 receptor; and fragments of any of the above-listed polypeptides.

Preferred molecular targets for antibodies detailed herein include CD proteins such as CD3, CD4, CD8, CD18, CD19, CD20, CD34, and CD40; members of the ErbB receptor family such as the EGF receptor, HER2, HER3 or HER4 receptor; cell adhesion molecules such as LFA-1, Macl, p150.95, VLA-4, ICAM-1, VCAM, .alpha.4/.beta.7 integrin, and .alpha.v/.beta.3 integrin including either .alpha. or .beta. subunits thereof (*e*.*g*. anti-CDlla, anti-CD18 or anti-CD11b antibodies); growth factors such as VEGF; tissue factor (TF); alpha interferon (.alpha.-IFN); an interleukin, such as IL-8; IgE; blood group antigens; flk2/flt3 receptor; obesity (OB) receptor; mpl receptor; CTLA-4; protein C, and the like.

Antibodies (including fragments thereof, including in turn antigen-binding fragments thereof) that may be produced by the methods herein include without limitation anti-HER2, antibody 2C4, anti-VEGF, antibody C2B8, antiCD11a, anti-tissue factor, IgG4b, anti-CD40, anti-CD20, anti-IgE, E25, E26, anti-PCSK9 and anti-Beta7.

### Cell growth and polypeptide production

Generally the cells are combined (contacted) with any of the cell culture media described herein under one or more conditions that promote any of cell growth, maintenance and/or polypeptide production. Methods of growing a cell (*i*.*e*., culturing a cell) and producing a polypeptide employ a culturing vessel (bioreactor) to contain the cell and cell culture medium. The culturing vessel can be composed of any material that is suitable for culturing cells, including glass, plastic or metal. Typically, the culturing vessel will be at least 1 liter and may be 10, 100, 250, 500, 1000, 2500, 5000, 8000, 10,000 liters or more. Culturing conditions that may be adjusted during the culturing process include but are not limited to pH and temperature.

A cell culture is generally maintained in the initial growth phase under conditions conducive to the survival, growth and viability (maintenance) of the cell culture. The precise conditions will vary depending on the cell type, the organism from which the cell was derived, and the nature and character of the expressed polypeptide.

The temperature of the cell culture in the initial growth phase will be selected based primarily on the range of temperatures at which the cell culture remains viable. For example, during the initial growth phase, CHO cells grow well at 37 °C. In general, most mammalian cells grow well within a range of about 25 °C. to 42 °C. Preferably, mammalian cells grow well within the range of about 35 °C. to 40 °C. Those of ordinary skill in the art will be able to select appropriate temperature or temperatures in which to grow cells, depending on the needs of the cells and the production requirements.

In one embodiment of the present disclosure, the temperature of the initial growth phase is maintained at a single, constant temperature. In another embodiment, the temperature of the initial growth phase is maintained within a range of temperatures. For example, the temperature may be steadily increased or decreased during the initial growth phase. Alternatively, the temperature may be increased or decreased by discrete amounts at various times during the initial growth phase. One of ordinary skill in the art will be able to determine whether a single or multiple temperatures should be used, and whether the temperature should be adjusted steadily or by discrete amounts.

The cells may be grown during the initial growth phase for a greater or lesser amount of time. In one variation, the cells are grown for a period of time sufficient to achieve a viable cell density that is a given percentage of the maximal viable cell density that the cells would eventually reach if allowed to grow undisturbed. For example, the cells may be grown for a period of time sufficient to achieve a desired viable cell density of 1, 5, 10, 15, 20, 25, 30, 35, 40, 45, 50, 55, 60, 65, 70, 75, 80, 85, 90, 95 or 99 percent of maximal viable cell density.

In another embodiment the cells are allowed to grow for a defined period of time. For example, depending on the starting concentration of the cell culture, the temperature at which the cells are grown, and the intrinsic growth rate of the cells, the cells may be grown for 0, 1, 2, 3, 4, 5, 6, 7, 8, 9, 10, 11, 12, 13, 14, 15, 16, 17, 18, 19, 20 or more days. In some cases, the cells may be allowed to grow for a month or more.

The cell culture may be agitated or shaken during the initial culture phase in order to increase oxygenation and dispersion of nutrients to the cells. In accordance with the present disclosure, one of ordinary skill in the art will understand that it can be beneficial to control or regulate certain internal conditions of the bioreactor during the initial growth phase, including but not limited to pH, temperature, oxygenation, etc. For example, pH can be controlled by supplying an appropriate amount of acid or base and oxygenation can be controlled with sparging devices that are well known in the art.

An initial culturing step is a growth phase, wherein batch cell culture conditions are modified to enhance growth of recombinant cells, to produce a seed train. The growth phase generally refers to the period of exponential growth where cells are generally rapidly dividing, *e*.*g*. growing. During this phase, cells are cultured for a period of time, usually 1 to 4 days, *e.g.* 1, 2, 3, or 4 days, and under such conditions that cell growth is optimal. The determination of the growth cycle for the host cell can be determined for the particular host cell by methods known to those skilled in the art.

In the growth phase, the basal culture medium and cells may be supplied to the culturing vessel in batch. The culture medium in one aspect contains less than about 5% or less than 1% or less than 0.1% serum and other animal-derived proteins. However, serum and animal-derived proteins can be used if desired. In a particular variation, the basal medium is a CDM. Amino acids, vitamins, trace elements and other media components at one or two times the ranges specified in European Patent EP 307,247 or U.S. Pat. No. 6,180,401 may be useds.

Alternatively, commercially available media such as Ham's F10 (Sigma), Minimal Essential Medium ([MEM], Sigma), RPMI-1640 (Sigma), and Dulbecco's Modified Eagle's Medium ([DMEM], Sigma) are suitable for culturing the animal cells and may be supplemented with chemically defined media constituents as detailed herein (*e*.*g*., by use of a kit as provided). In addition, any of the media described in Ham and Wallace, Meth. Enz., 58:44 (1979), Barnes and Sato, Anal. Biochem., 102:255 (1980), U.S. Pat. Nos. 4,767,704; 4,657,866; 4,927,762; or 4,560,655; WO 90/03430; WO 87/00195; U.S. Pat. No. Re. 30,985; or U.S. Pat. No. 5,122,469, may be used as culture media for the host cells, each of which may be supplemented with chemically defined media constituents as detailed herein (*e*.*g*., by use of a kit as provided). In one aspect, if a medium contains an animal-based product, the medium may be supplemented with CDM.

Any media provided herein may also be supplemented as necessary with hormones and/or other growth factors (such as insulin, transferrin, or epidermal growth factor), ions (such as sodium, chloride, calcium, magnesium, and phosphate), buffers (such as HEPES), nucleosides (such as adenosine and thymidine), trace elements (defined as inorganic compounds usually present at final concentrations in the micromolar range), and glucose or an equivalent energy source. Any other necessary supplements may also be included at appropriate concentrations that would be known to those skilled in the art.

At a particular point in their growth, the cells may form an inoculum to inoculate a culture medium at the start of culturing in the production phase. Alternatively, the production phase may be continuous with the growth phase. The cell growth phase is generally followed by a polypeptide production phase.

During the polypeptide production phase, the cell culture may be maintained under a second set of culture conditions (as compared to the growth phase) conducive to the survival and viability of the cell culture and appropriate for expression of the desired polypeptide. For example, during the subsequent production phase, CHO cells express recombinant polypeptides and proteins well within a range of 25°C to 35°C. Multiple discrete temperature shifts may be employed to increase cell density or viability or to increase expression of the recombinant polypeptide or protein. In one aspect, a medium as provided herein reduces the presence of contaminants when used in a method of increasing polypeptide production as compared to contaminants obtained when the polypeptide is produced in a different medium. In one variation, the contaminants are charge variants or reactive oxygen species. In one aspect, a medium as provided herein reduces color intensity of a polypeptide product when used in a method of increasing production of the polypeptide as compared to color intensity obtained when the polypeptide product is produced in a different media. In one variation, a method of increasing polypeptide production comprises a temperature shift step during the polypeptide production phase. In a further variation, a temperature shift step comprises a shift of the temperature from 31°C to 37°C, from 32°C to 37°C, from 33°C to 37°C, from 34°C to 37°C, from 35°C to 37°C, from 36°C to 37°C, from 31°C to 32°C, from 31°C to 33°C, from 31°C to 34°C, from 31°C to 35°C, or from 31°C to 36°C.

The cells may be maintained in the subsequent production phase until a desired cell density or production titer is reached. In one embodiment, the cells are maintained in the subsequent production phase until the titer to the recombinant polypeptide reaches a maximum. In other embodiments, the culture may be harvested prior to this point. For example, the cells may be maintained for a period of time sufficient to achieve a viable cell density of 1, 5, 10, 15, 20, 25, 30, 35, 40, 45, 50, 55, 60, 65, 70, 75, 80, 85, 90, 95 or 99 percent of maximal viable cell density. In some cases, it may be desirable to allow the viable cell density to reach a maximum, and then allow the viable cell density to decline to some level before harvesting the culture.

In certain cases, it may be beneficial or necessary to supplement the cell culture during the subsequent production phase with nutrients or other medium components that have been depleted or metabolized by the cells. For example, it might be advantageous to supplement the cell culture with nutrients or other medium components observed to have been depleted during monitoring of the cell culture. Alternatively or additionally, it may be beneficial or necessary to supplement the cell culture prior to the subsequent production phase. As non-limiting examples, it may be beneficial or necessary to supplement the cell culture with hormones and/or other growth factors, particular ions (such as sodium, chloride, calcium, magnesium, and phosphate), buffers, vitamins, nucleosides or nucleotides, trace elements (inorganic compounds usually present at very low final concentrations), amino acids, lipids, or glucose or other energy source.

### Polypeptide Purification

The polypeptide of interest preferably is recovered from the culture medium as a secreted polypeptide, although it also may be recovered from host cell lysates when directly expressed without a secretory signal. In one aspect, the polypeptide produced is an antibody, such as a monoclonal antibody.

The culture medium or lysate may be centrifuged to remove particulate cell debris. The polypeptide thereafter may be purified from contaminant soluble proteins and polypeptides, with the following procedures being exemplary of suitable purification procedures: by fractionation on immunoaffinity or ion-exchange columns; ethanol precipitation; reverse phase HPLC; chromatography on silica or on a cation-exchange resin such as DEAE; chromatofocusing; SDS-PAGE; ammonium sulfate precipitation; gel filtration using, for example, Sephadex G-75; and protein A Sepharose columns to remove contaminants such as IgG. A protease inhibitor such as phenyl methyl sulfonyl fluoride (PMSF) also may be useful to inhibit proteolytic degradation during purification. One skilled in the art will appreciate that purification methods suitable for the polypeptide of interest may require modification to account for changes in the character of the polypeptide upon expression in recombinant cell culture. Antibodies can be generally purified using chromatographic techniques (*e*.*g*., protein A, affinity chromatography with a low pH elution step and ion exchange chromatography to remove process impurities). Purified proteins may concentrated to provide a concentrated protein drug product, *e.g*., one with a protein concentration of at least 100 mg/mL or 125 mg/mL or 150 mg/mL or a concentration of about 100 mg/mL or 125 mg/mL or 150 mg/mL. Purified proteins may also be concentrated to provide a concentrated protein drug product, *e.g*., one with a protein concentration of at least 1 mg/mL or 10 mg/mL or 25 mg/mL or 50 mg/mL or 75 mg/mL or a concentration of at least about any one of 1 mg/mL or 10 mg/mL or 50 mg/mL or 75 mg/mL to about 125 mg/mL or to about 150 mg/mL). It is understood that concentrated polypeptide products may be concentrated up to levels that are permissible under the concentration conditions, *e*.*g*., up to a concentration at which the polypeptide is no longer soluble in solution. For example, a polypeptide purification process can comprise the steps of harvesting cell culture fluid from polypeptide-producing cells and purifying the polypeptide through protein A affinity chromatography with further purification through anion and cation exchange chromatography, filtration for removal of virus, and a final ultrafiltration and diafiltration step for final formulation and concentration of the polypeptide. Non-limiting examples of methods for producing and purifying polypeptides for drug formulations are described in Kelley, B. MAbs., 2009, 1(5):443-452.

### Polypeptide Color Assessment

The polypeptides produced by the methods detailed herein and present in the compositions provided may be assessed for color at any step of the protein purification process. A method for assessing color may involve harvesting the cell culture fluid from cells grown in the media detailed herein, purifying the polypeptide from cell culture fluid to obtain a composition (*e*.*g*., a solution) comprising the polypeptide and assessing the solution comprising the polypeptide for color. In one variation, a composition comprising the polypeptide is assessed for color after purification with Protein A affinity chromatography. In a further variation, a composition comprising the polypeptide is assessed for color after purification by ion exchange chromatography. In another variation, a composition comprising the polypeptide is assessed for color after purification by high performance liquid chromatography. In yet another variation, a composition comprising the polypeptide is assessed for color after purification by hydrophobic interaction chromatography. In still another variation, a composition comprising the polypeptide is assessed for color after purification by size exclusion chromatography. In one variation, a composition comprising the polypeptide is assessed for color after purification by filtration including microfiltration or ultrafiltration. In one variation, the composition comprising the polypeptide is concentrated prior to assessing for color (*e*.*g*., the composition may comprise at least 100 mg/mL, 125 mg/mL or 150 mg/mL polypeptide, such as an antibody). In some variations, the concentrated composition comprises at least 1 mg/mL, 25 mg/mL, 50 mg/mL, or 75 mg/mL polypeptide (*e*.*g*., antibody) prior to assessing for color. The composition comprising the polypeptide can be concentrated by centrifugation, filter devices, semi-permeable membranes, dialysis, precipitation, ion exchange chromatography, affinity chromatography, high performance liquid chromatography, or hydrophobic interaction chromatography. In one variation, the polypeptide can be concentrated by lyophilization and resuspended prior to assessment for color. The composition comprising the polypeptide may be assessed for color after purification with one or more of the techniques detailed herein. Color assessment of the composition comprising the polypeptide after the composition has undergone one or more freeze thaw cycle(s) is contemplated herein. Methods for color assessment of cell culture fluid containing the polypeptide prior to purification or concentration of the polypeptide is further contemplated herein.

The polypeptides produced by the methods detailed herein with the media described herein (or present in the compositions provided) may be assessed for color by use of one or more visual color standards. Methods for color assessment of composition comprising the polypeptide include use of an international or national color standard such as, but not limited to, the United States Pharmacopoeia color standard and the European Pharmacopoeia color standard. See USP-24 Monograph 631 Color and Achromaticity. *United States Pharmacopoeia Inc*., 2000, p. 1926-1927 and Council of Europe. European Pharmacopoeia, 2008, 7th Ed. P.22. For example, the Color, Opalescence and Coloration (COC) assay may be used to assess color of a solution containing the polypeptide. In one variation, identical tube of colorless, transparent, neutral glass of 12 mm external diameter are used to compare 2.0 mL of the composition comprising the polypeptide with 2.0 mL of water or of the solvent or of the reference solution prescribed in the monograph. The colors are compared in diffused daylight and viewed horizontally against a white background for color determination, measurement, or assessment. In another
variation, identical tubes of colorless, transparent, neutral glass with a flat base and an internal diameter of 15 mm to 25 mm are used to compare the composition comprising the polypeptide with water or the solvent or the reference solution prescribed in the monograph, the depth of the layer being 40 mm. The colors are compared in diffused daylight and viewed vertically against a white background for color determination, measurement, or assessment. In one variation, color determination, measurement or assessment can be done by human visual inspection. In another variation, color determination, measurement, or assessment can be done by using an automated process. For example, the tubes can be loaded in a machine that images the tubes for processing of the images with an algorithm to determine, measure, or assess the color. It is understood that the reference standards for the COC assay can be any one of, but not limited to, brown (B), brownish-yellow (BY), yellow (Y), greenish-yellow (GY), or red (R). Compositions comprising the polypeptide that are compared to the brown reference standard can be given a brown reference standard value of B1 (darkest), B2, B3, B4, B5, B6, B7, B8, or B9 (lightest). Compositions comprising the polypeptide that are compared to the brownish-yellow reference standard can be given a brownish-yellow reference standard value of BY1 (darkest), BY2, BY3, BY4, BY5, BY6, or BY7 (lightest). Compositions comprising the polypeptide that are compared to the yellow reference standard can be given a yellow reference standard value of Y1 (darkest), Y2, Y3, Y4, Y5, Y6, or Y7 (lightest). Compositions comprising the polypeptide that are compared to the greenish-yellow reference standard can be given a greenish-yellow reference standard value of GY1 (darkest), GY2, GY3, GY4, GY5, GY6, or GY7 (lightest). Compositions comprising the polypeptide that are compared to the red reference standard can be given a red reference standard value of R1 (darkest), R2, R3, R4, R5, R6, or R7 (lightest). In one aspect, an acceptable color is any color except that which measures darkest on a scale provided herein (*e*.*g*., except R1 for a red reference standard value). In one variation, the color of the composition comprising the polypeptide produced by cells cultured in the media detailed herein has a reference standard value as described in Table 2. As is described herein, it is understood that in one aspect the media that may be used in the methods and compositions herein result in a polypeptide composition (which in one variation is a composition comprising at least 100 mg/mL or 125 mg/mL or 150 mg/ml polypeptide) having a reference standard color value selected from the group consisting of B3, B4, B5, B6, B7, B8, B9, BY3, BY4, BY5, BY6, BY7, Y3, Y4, Y5, Y6, Y7, GY3, GY4, GY5, GY6, GY7, R3, R4, R5, R6 and R7. In one aspect, the media that may be used in the methods and compositions herein result in a polypeptide composition (which in one variation is a composition comprising at least 100 mg/mL or 125 mg/mL or 150 mg/ml polypeptide) having a reference standard color value of greater than any one of B4, B5, B6, B7, B8, BY4, BY5, BY6, Y4, Y5, Y6, GY4, GY5, GY6, GY7, R3, R4, R5 and R6. As would be understood to the skilled artisan, descriptions of reference standard color values are applicable to, and may further modify descriptions of, any of the media, methods or compositions detailed herein.

**Table 2. Exemplary reference standard values**

| **Reference standard** | **Reference standard value** |
|---|---|
| (a) Brown | from about B1 to about B9; from about B1 to about B8; from about B1 to about B7; from about B1 to about B6; from about B1 to about B5; from about B1 to about B4; from about B1 to about B3; from about B1 to about B2; from about B2 to about B9; from about B3 to about B9; from about B4 to about B9; from about B5 to about B9; from about B6 to about B9; from about B7 to about B9; from about B8 to about B9; from about B2 to about B8; from about B3 to about B7; from about B4 to about B6; from about B5 to about B7; from about B6 to about B8; about any of B1 or B2 or B3 or B4 or B5 or B6 or B7 or B8 or B9; at least about any of B1 or B2 or B3 or B4 or B5 or B6 or B7 or B8 or B9. Preferably B3 to B9. Most preferably B4 to B9. |
| (b) Brownish-Yellow | from about BY1 to about BY7; from about BY1 to about BY6; from about BY1 to about BY5; from about BY1 to about BY4; from about BY1 to about BY3; from about BY1 to about BY2; from about BY2 to about BY7; from about BY3 to about BY7; from about BY4 to about BY7; from about BY5 to about BY7; from about BY6 to about BY7; from about BY2 to about BY6; from about BY3 to about BY5; from about BY4 to about BY6; from about BY5 to about BY6; about any of BY1 or BY2 or BY3 or BY4 or BY5 or BY6 or BY7; at least about any of BY1 or BY2 or BY3 or BY4 or BY5 or BY6 or BY7. Preferably BY3 to BY7. Most preferably BY4 to BY7. |
| (c) Yellow | from about Y1 to about Y7; from about Y1 to about Y6; from about Y1 to about Y5; from about Y1 to about Y4; from about Y1 to about Y3; from about Y1 to about Y2; from about Y2 to about Y7; from about Y3 to about Y7; from about Y4 to about Y7; from about Y5 to about Y7; from about Y6 to about Y7; from about Y2 to about Y6; from about Y3 to about Y5; from about Y4 to about Y6; from about Y5 to about Y6; about any of Y1 or Y2 or Y3 or Y4 or Y5 or Y6 or Y7; at least about any of Y1 or Y2 or Y3 or Y4 or Y5 or Y6 or Y7. Preferably Y3 to Y7. Most preferably Y4 to Y7. |
| (d) Greenish-Yellow | from about GY1 to about GY7; from about GY1 to about GY6; from about GY1 to about GY5; from about GY1 to about GY4; from about GY1 to about GY3; from about GY1 to about GY2; from about GY2 to about GY7; from about GY3 to about GY7; from about GY4 to about GY7; from about GY5 to about GY7; from about GY6 to about GY7; from about GY2 to about GY6; from about GY3 to about GY5; from about GY4 to about GY6; from about GY5 to about GY6; about any of GY1 or GY2 or GY3 or GY4 or GY5 or GY6 or GY7; at least about any of GY1 or GY2 or GY3 or GY4 or GY5 or GY6 or GY7. Preferably GY3 to GY7. Most preferably GY4 to GY7. |
| (e) Red | from about R1 to about R7; from about R1 to about R6; from about R1 to about R5; from about R1 to about R4; from about R1 to about R3; from about R1 to about R2; from about R2 to about R7; from about R3 to about R7; from about R4 to about R7; from about R5 to about R7; from about R6 to about R7; from about R2 to about R6; from about R3 to about R5; from about R4 to about R6; from about R5 to about R6; about any of R1 or R2 or R3 or R4 or R5 or R6 or R7; at least about any of R1 or R2 or R3 or R4 or R5 or R6 or R7. Preferably R3 to R7. Most preferably R4 to R7. |

In another example, the polypeptides produced by the methods detailed herein with the media described herein (or present in the compositions provided) may be assessed for color with a quantitative assay. In a variation, the quantitative assay can be done using an automated process. In a variation, the quantitative assay is the normalized fluorescence intensity (NIFTY) assay or the Total Color assay described herein. For example, a solution containing a polypeptide produced by any of the methods described herein may be assessed for color intensity by the NIFTY assay by subjecting a solution comprising a polypeptide to the following steps: 1) applying polypeptide test sample to size exclusion chromatography (SEC) wherein the mobile phase for SEC comprises a buffer at a specific pH with the column maintained at a specific temperature; 2) monitoring the SEC eluent for UV absorption at a specific wavelength (*e*.*g*., 280nm) and for fluorescence with a specific excitation wavelength (*e*.*g*., at 350nm) and emission wavelength (*e*.*g*., at 425nm); 3) integrating the SEC peaks of the polypeptide species using software known in the art (*e*.*g*., Agilent Chemstation software) on the UV absorbance and the fluorescence emission chromatograms and normalizing the fluorescence by dividing the fluorescence peak area of the main peak by the UV absorbance peak area of the main peak; and 4) calculating the ratio of the normalized fluorescence of the polypeptide test sample to that of a polypeptide reference sample containing a known COC value based on any of the reference standards disclosed herein such as brown (B), brownish-yellow (BY), yellow (Y), greenish-yellow (GY), or red (R) to obtain a numerical value, wherein a higher numerical value (*e*.*g*., a higher NIFTY value) indicates a higher color intensity and a lower numerical value (*e*.*g*., a lower NIFTY value) indicates a lower color intensity. In another example, a solution containing a polypeptide produced by any of the methods described herein is assessed for color intensity by the Total Color assay as described herein. For example, a solution containing a polypeptide produced by any of the methods described herein may be assessed for color intensity by the Total Color assay by subjecting a solution comprising a polypeptide to the following steps: 1) obtaining the absorption spectrum of a polypeptide test sample by measuring the sample in the visible region (380-780nm) using a spectrophotometer; 2) converting the absorption spectrum to the CIE L*a*b* color scale as described in Standard Practice for Calculation of Color Tolerances and Color Differences from Instrumentally Measured Color Coordinates, Annual Book of ASTM Standards, Vol. 06.01, (2011); 3) obtaining a Total Color measurement wherein the measurement represents the Delta E which corresponds to the Euclidian distance between the polypeptide test sample and water in the three dimensional CIE L*a*b* color space; and 4) determining the color intensity value by calculating the ratio of the "Total Color" measurement of the polypeptide test sample to that of a polypeptide reference sample containing a known COC value based on any of the reference standards disclosed herein such as brown (B), brownish-yellow (BY), yellow (Y), greenish-yellow (GY), or red (R), wherein a higher Total Color value indicates a higher color intensity and a lower Total Color value indicates a lower color intensity.

A color assay detailed herein may find use in assessing color of any solution (*e*.*g*., a polypeptide-containing solution), including, but not limited to, the polypeptide compositions provided herein.

### Polypeptide Charge Variant Assessment

Methods for reducing the presence of charge variants (*e*.*g*., acidic charge variants) are provided, wherein the media containing components at concentrations detailed herein reduces the presence of charge variants (*e*.*g*., acidic charge variants) when used in a method of producing a polypeptide as compared to charge variants (*e*.*g*., acidic charge variants) obtained when the polypeptide is produced in a different media (*e.g*., one that does not comprise the same components and/or component concentrations as detailed herein). As would be understood to the skilled artisan, descriptions of reference charge variants are applicable to, and may further modify descriptions of, any of the media, methods or compositions detailed herein. It is also understood that any variations or embodiments of media provided in this section applies equally to media descriptions detailed throughout. As used herein the term "reduces the presence of charge variants" can refer to the reduced amounts or presence of all types of charge variants (*e*.*g*., acidic charge variants, basic charge variants, and neutral charge variants) or the reduced amounts or presence of specific charge variants such as acidic charge variants.

Methods for reducing the presence of charge variants (*e*.*g*., acidic charge variants) are provided, as are compositions comprising a reduced level of charge variants (*e*.*g*., acidic charge variants), wherein the media detailed herein reduces the presence of charge variants when used in a method of producing a polypeptide as compared to charge variants obtained when the polypeptide is produced in a different media. In one variation, the media is a chemically undefined cell culture medium comprising from about 300 mg/L (in some embodiments, 200 mg/L) to about 1200 mg/L cystine, from about 0.05 mg/L to about 1.0 mg/L vitamin B2, from about 0.05 mg/L to about 10.0 mg/L vitamin B6, from about 0.05 mg/L to about 12.0 mg/L vitamin B9 and from about 0.05 mg/L to about 2.5 mg/L vitamin B12. In a variation, vitamin B2 is at a concentration of from about 0.05 mg/L to about 0.50 mg/L. In another variation, vitamin B2 is at a concentration of from about 0.05 mg/L to about 0.40 mg/L. In another variation, vitamin B2 is at a concentration of from about 0.05 mg/L to about 0.30 mg/L. In a variation, vitamin B6 is at a concentration of from about 0.05 mg/L to about 8.0 mg/L. In another variation, vitamin B6 is at a concentration of from about 0.05 mg/L to about 7.0 mg/L. In another variation, vitamin B6 is at a concentration of from about 0.05 mg/L to about 6.0 mg/L. In one variation, the chemically undefined culture medium further comprises an iron source. In a variation the iron source is ferric citrate or ferrous sulfate. In one variation, the chemically undefined cell culture medium comprises ferric citrate at a concentration of from about 2 µM to about 80 µM. In any of the variations herein the chemically undefined cell culture medium further comprises hydrocortisone. In a variation the hydrocortisone at a concentration of from about 0.05 µM to about 0.25 µM.

Methods for reducing the presence of charge variants (*e*.*g*., acidic charge variants) are provided, as are compositions comprising a reduced level of charge variants (*e*.*g*., acidic charge variants), wherein the media detailed herein reduces the presence of charge variants when used in a method of producing a polypeptide as compared to charge variants obtained when the polypeptide is produced in a different media. In one variation, the media is a chemically defined cell culture medium comprising from about from about 300 mg/L (in some embodiments, 200 mg/L) to about 1200 mg/L cystine, from about 2 µM to about 80 µM ferric citrate, and from about 0.05 µM to about 0.5 µM hydrocortisone. In a variation, the chemically defined culture medium further comprises vitamin B2, vitamin B6, vitamin B9 and vitamin B12. In a variation the vitamin B2 is at a concentration from about 0.05 mg/L to about 1.0 mg/L. In another variation, vitamin B2 is at a concentration of from about 0.05 mg/L to about 0.50 mg/L. In another variation, vitamin B2 is at a concentration of from about 0.05 mg/L to about 0.40 mg/L. In another variation, vitamin B2 is at a concentration of from about 0.05 mg/L to about 0.30 mg/L. In a further variation the vitamin B6 is at a concentration from about 0.05 mg/L to about 10.0 mg/L. In a further variation, vitamin B6 is at a concentration of from about 0.05 mg/L to about 8.0 mg/L. In a further variation, vitamin B6 is at a concentration of from about 0.05 mg/L to about 7.0 mg/L. In a further variation, vitamin B6 is at a concentration of from about 0.05 mg/L to about 6.0 mg/L. In any of the variations herein the vitamin B6 is at a concentration from about 0.05 mg/L to about 10.0 mg/L, from about 0.05mg/L to about 8.0 mg/L, from about 0.05 mg/L to about 7.0 mg/L or from about 0.05mg/L to about 6.0 mg/L. In a further variation the vitamin B9 is at a concentration from about 0.05 mg/L to about 12.0 mg/L. In any of the variations herein the vitamin B9 is at a concentration from about 0.05 mg/L to about 12.0 mg/L. In a further variation the vitamin B12 is at a concentration from about 0.05 mg/L to about 2.5 mg/L. In any of the variations herein the vitamin B12 is at a concentration from about 0.05 mg/L to about 2.5 mg/L.

Methods for reducing the presence of charge variants (*e*.*g*., acidic charge variants) are provided, as are compositions comprising a reduced level of charge variants (*e*.*g*., acidic charge variants), wherein the media detailed herein reduces the presence of charge variants when used in a method of producing a polypeptide as compared to charge variants obtained when the polypeptide is produced in a different media. In one variation, the media is a chemically undefined cell culture medium comprising from about from about 300 mg/L (in some embodiments, 200 mg/L) to about 1200 mg/L cystine, from about 2 µM to about 80 µM ferric citrate, and from about 0.05 µM to about 0.5 µM hydrocortisone. In a variation, the chemically undefined culture medium further comprises vitamin B2, vitamin B6, vitamin B9 and vitamin B12. In a variation the vitamin B2 is at a concentration from about 0.05 mg/L to about 1.0 mg/L. In another variation, vitamin B2 is at a concentration of from about 0.05 mg/L to about 0.50 mg/L. In another variation, vitamin B2 is at a concentration of from about 0.05 mg/L to about 0.40 mg/L. In another variation, vitamin B2 is at a concentration of from about 0.05 mg/L to about 0.30 mg/L. In a further variation the vitamin B6 is at a concentration from about 0.05 mg/L to about 10.0 mg/L. In a further variation, vitamin B6 is at a concentration of from about 0.05 mg/L to about 8.0 mg/L. In a further variation, vitamin B6 is at a concentration of from about 0.05 mg/L to about 7.0 mg/L. In a further variation, vitamin B6 is at a concentration of from about 0.05 mg/L to about 6.0 mg/L. In any of the variations herein the vitamin B6 is at a concentration from about 0.05 mg/L to about 10.0 mg/L, from about 0.05mg/L to about 8.0 mg/L, from about 0.05 mg/L to about 7.0 mg/L or from about 0.05mg/L to about 6.0 mg/L. In any of the variations herein the vitamin B6 is at a concentration from about 0.05 mg/L to about 10.0 mg/L. In a further variation the vitamin B9 is at a concentration from about 0.05 mg/L to about 12.0 mg/L. In any of the variations herein the vitamin B9 is at a concentration from about 0.05 mg/L to about 12.0 mg/L. In a further variation the vitamin B12 is at a concentration from about 0.05 mg/L to about 2.5 mg/L. In any of the variations herein the vitamin B12 is at a concentration from about 0.05 mg/L to about 2.5 mg/L.

The polypeptides, including compositions comprising polypeptides, produced by the methods detailed herein may be assessed for the presence of charge variants at any step of the protein purification process. A method for assessing the presence of charge variants may involve harvesting the cell culture fluid from cells grown in the media detailed herein, purifying the polypeptide from cell culture fluid to obtain a composition comprising the polypeptide and assessing the composition comprising the polypeptide for reduced presence of charge variants as compared to the presence of charge variants in a composition comprising the polypeptide when produced in a different media. In one variation, the composition comprising the polypeptide may contain acidic charge variants or basic charge variants. In another variation, the composition comprising the polypeptide comprises acidic charge variants. Charge variants can form due to, but not limited to, deamidation, sialyation, C-terminal lysine cleavage, glycation, C-terminal lysine amidation, C-terminal glycine amidation, succinamide formation, amino acid oxidation, removal of sialic acid or combinations thereof. Methods for assessment for the presence of charge variants in cell culture fluid containing the polypeptide prior to purification or concentration of the polypeptide is further contemplated herein. Methods for detection of charge variants in solutions containing the polypeptide include use of chromatography techniques such as, but not limited to, ion exchange chromatography. See Khawli, L.A., mAbs., 2010, 2(6):613-624.

In one variation of the compositions provided herein, charge variants (which in one aspect are acidic charge variants) constitute no more than 25% or 20% or 18% or 15% or 10% of the polypeptide product. In another variation of the compositions and methods provided herein, at least 75% or 80% or 85% or 90% or 95% or more of the polypeptide product is a main species protein. As used herein the term "main species protein" can further include a quantitatively predominant protein as identified by the amino acid sequence, the secondary structure, and/or the tertiary structure of the protein as well as any post-translational modifications such as glycosylation.

### Kits

A kit for supplementing a cell culture medium with chemically defined constituents is described. The kit may contain dried constituents to be reconstituted, and may also contain instructions for use (*e*.*g*., for use in supplementing a medium with the kit constituents). The kit may contain the medium constituents provided herein in amounts suitable to supplement a cell culture medium. In one variation, a kit comprises medium components of Table 1. In another variation, a kit comprises medium components of Table 1A. Various exemplary kit embodiments are provided in the "Exemplary Embodiments" section. In addition, the disclosure also includes variations in which the kit for supplementing a cell culture medium comprises any one or more media components of Table 1 or Table 1A in an amount to provide the one or more media components in the cell culture medium at a concentration as shown in Table 1 or Table 1A. In one variation, a kit for supplementing a cell culture medium comprises cystine in an amount to provide from about 0.8 mM (in one aspect, 0.7 mM) to about 2.5 mM cystine in the cell culture medium; vitamin B2 in an amount to provide from about 0.11 µM to about 0.72 µM vitamin B2 in the cell culture medium; vitamin B6 in an amount to provide from about 4.5 µM to about 30.0 µM vitamin B6 in the cell culture medium; vitamin B9 in an amount to provide from about 3.4 µM to about 22.0 µM vitamin B9 in the cell culture medium; and vitamin B12 in an amount to provide from about 0.2 µM to about 1.5 µM vitamin B12 in the cell culture medium. In any variation herein, the kit can further comprise an iron source such as ferric citrate or ferrous sulfate in an amount to provide from about 2 µM to about 80 µM (in some aspects, 11.0 µM to about 36.0 µM) in the cell culture medium.

In another variation, a kit for supplementing a cell culture medium comprises cystine in an amount to provide from about 0.8 mM (in some aspects, 0.7 mM) to about 2.5 mM cystine in the cell culture medium; ferric citrate in an amount to provide from about 2 µM to about 80 µM ferric citrate in the cell culture medium; hydrocortisone in an amount to provide from about 0.05 µM to about 0.5 µM hydrocortisone in the cell culture medium; vitamin B2 in an amount to provide from about 0.11 µM to about 0.72 µM vitamin B2 in the cell culture medium; vitamin B6 in an amount to provide from about 4.5 µM to about 30.0 µM vitamin B6 in the cell culture medium; vitamin B9 in an amount to provide from about 3.4 µM to about 22.0 µM vitamin B9 in the cell culture medium; and vitamin B12 in an amount to provide from about 0.2 µM to about 1.5 µM vitamin B12 in the cell culture medium.

In any variation herein, the kit may further comprise any one or more of vitamin B1 in an amount to provide from about 2.0 µM to about 14.0 µM vitamin B1 in the cell culture medium;
vitamin B3 in an amount to provide from about 11.0 µM to about 72.0 µM vitamin B3 in the cell culture medium; vitamin B5 in an amount to provide from about 6.8 µM to about 44.0 µM vitamin B5 in the cell culture medium; and vitamin B7 in an amount to provide from about 0.02 µM to about 0.24 µM vitamin B7 in the cell culture medium.

In any of the variations herein, the kit can further comprise cysteine in an amount to provide from about 80 mg/L to about 1500 mg/L cysteine (in some aspects, 0.5 mM to about 2.0 mM) in the cell culture medium.

In any of the variations herein, the cell culture media can be a CDM or a chemically undefined cell culture media.

### Compositions

Compositions comprising the cell culture medium and one or more other components, such as a cell or a desired polypeptide (*e*.*g*., an antibody), are also provided. In one variation is provided a composition comprising: (a) a cell comprising an isolated nucleic acid encoding a polypeptide; and (b) a cell culture medium as provided herein. In another variation is provided a composition comprising: (a) a polypeptide; and (b) a cell culture medium as provided herein, where in one aspect the polypeptide is secreted into the medium by a cell comprising an isolated nucleic acid encoding the polypeptide. In yet another variation is provided a composition comprising: (a) a polypeptide; and (b) a cell culture medium as provided herein, where in one aspect the polypeptide is released into the medium by lysis of a cell comprising an isolated nucleic acid encoding the polypeptide. The cell of the composition may be any cell detailed herein (*e*.*g*., a CHO cell) and the medium of the composition may be any medium detailed herein, such as a medium (which may be a CDM) comprising medium components as detailed in Table 1 or Table 1A. Likewise, the polypeptide of the composition may be any polypeptide detailed herein, such as an antibody.

In one variation the composition may have a color. In one variation the color is determined, measured, or assessed by use of one or more visual color standards. The visual color standard can be an international or national color standard such as, but not limited to, the United States Pharmacopoeia color standard and the European Pharmacopoeia color standard. See USP-24 Monograph 631 Color and Achromaticity. *United States Pharmacopoeia Inc*., 2000, p. 1926-1927 and Council of Europe. European Pharmacopoeia, 2008, 7th Ed. P.22. For example, the color of the composition may be determined, measured or assessed by use of the Color, Opalescence and Coloration (COC) assay. It is understood that the reference standards for the COC assay can be any one of, but not limited to, brown (B), brownish-yellow (BY), yellow (Y), greenish-yellow (GY), or red (R). Compositions comprising the polypeptide that are compared to the brown reference standard can be given a brown reference standard value of B1 (darkest), B2, B3, B4, B5, B6, B7, B8, or B9 (lightest). Compositions comprising the polypeptide that are compared to the brownish-yellow reference standard can be given a brownish-yellow reference standard value of BY1 (darkest), BY2, BY3, BY4, BY5, BY6, or BY7 (lightest). Compositions comprising the polypeptide that are compared to the yellow reference standard can be given a yellow reference standard value of Y1 (darkest), Y2, Y3, Y4, Y5, Y6, or Y7 (lightest). Compositions comprising the polypeptide that are compared to the greenish-yellow reference standard can be given a greenish-yellow reference standard value of GY1 (darkest), GY2, GY3, GY4, GY5, GY6, or GY7 (lightest). Compositions comprising the polypeptide that are compared to the red reference standard can be given a red reference standard value of R1 (darkest), R2, R3, R4, R5, R6, or R7 (lightest). Compositions as detailed herein can have a reference standard value as described in Table 2 or as detailed throughout. In a particular variation, a composition as provided herein comprises a polypeptide at a concentration of at least 100 mg/mL or 125 mg/mL or 150 mg/mL or at a concentration of about 100 mg/mL or 125 mg/mL or 150 mg/mL or 175 mg/mL or 200 mg/mL. In another variation, a composition as provided herein comprises a polypeptide at a concentration of at least 1 mg/mL or 10 mg/mL or 25 mg/mL or 50 mg/mL or 75 mg/mL or at a concentration of about 1 mg/mL or 10 mg/mL or 25 mg/mL or 50 mg/mL or 75 mg/mL. In another variation, a composition as provided herein comprises a polypeptide at a concentration of at least about any one of 1 mg/mL or 10 mg/mL or 50 mg/mL or 75 mg/mL to about 125 mg/mL or to about 150 mg/mL. Any composition provided herein can comprise the polypeptide at a concentration up to the solubility limit of the polypeptide or at a concentration that provides a therapeutically effective amount of the polypeptide when administered to a subject. As used herein, a "therapeutically effective amount" of a polypeptide (*e*.*g*., an antibody) refers to an amount effective in the prevention or treatment of a disorder for the treatment of which the polypeptide is effective. In a further variation, a composition as provided herein has a color reference standard value selected from any one of B4-B9, BY4-BY7, Y4-Y7, GY4-GY7 and R4-R7. Also provided are compositions comprising a polypeptide at a concentration of at least 100 mg/mL or 125 mg/mL or 150 mg/mL or at a concentration of about 100 mg/mL or 125 mg/mL or 150 mg/mL or 175 mg/mL or 200 mg/mL and wherein the composition has a color reference standard value selected from any one of B4-B9, BY4-BY7, Y4-Y7, GY4-GY7 and R4-R7. Provided herein are also compositions comprising a polypeptide at a concentration of at least 1 mg/mL or 10 mg/mL or 25 mg/mL or 50 mg/mL or 75 mg/mL or at a concentration of about 1 mg/mL or 10 mg/mL or 25 mg/mL or 50 mg/mL or 75 mg/mL or at least about any one of 1 mg/mL or 10 mg/mL or 50 mg/mL or 75 mg/mL to about 125 mg/mL or to about 150 mg/mL and wherein the composition has a color reference standard value selected from any one of B4-B9, BY4-BY7, Y4-Y7, GY4-GY7 and R4-R7.

In another example, the color of the composition may be determined, measured or assessed by use of a quantitative assay such as the NIFTY assay or the Total Color assay. In a variation, higher numerical values obtained by the quantitative assay indicate higher color intensity and lower numerical values indicate lower color intensity.

Compositions (*e*.*g*., pharmaceutical formulations) of the polypeptides (*e*.*g*., antibodies) produced by any of the methods described herein are prepared by mixing a polypeptide having the desired degree of purity with one or more optional pharmaceutically acceptable carriers (Remington's Pharmaceutical Sciences 16th edition, Osol, A. Ed. (1980)), in the form of lyophilized formulations or aqueous solutions. Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG). Exemplary pharmaceutically acceptable carriers herein further include insterstitial drug dispersion agents such as soluble neutral-active hyaluronidase glycoproteins (sHASEGP), for example, human soluble PH-20 hyaluronidase glycoproteins, such as rHuPH20 (HYLENEX®, Baxter International, Inc.). Certain exemplary sHASEGPs and methods of use, including rHuPH20, are described in US Patent Publication Nos. 2005/0260186 and 2006/0104968. In one aspect, a sHASEGP is combined with one or more additional glycosaminoglycanases such as chondroitinases. Exemplary lyophilized polypeptide formulations are described in US Patent No. 6,267,958. Aqueous polypeptide formulations include those described in US Patent No. 6,171,586 and WO2006/044908, the latter formulations including a histidine-acetate buffer. The formulations to be used for *in vivo* administration are generally sterile. Sterility may be readily accomplished, *e*.*g*., by filtration through sterile filtration membranes. In a particular variation, a pharmaceutical formulation as provided herein comprises a polypeptide at a concentration of at least 100 mg/mL or 125 mg/mL or 150 mg/mL or at a concentration of about 100 mg/mL or 125 mg/mL or 150 mg/mL or 175 mg/mL or 200 mg/mL. In another variation, a pharmaceutical formulation as provided herein comprises a polypeptide at a concentration of at least 1 mg/mL or 10 mg/mL or 25 mg/mL or 50 mg/mL or 75 mg/mL or at a concentration of about 1 mg/mL or 10 mg/mL or 25 mg/mL or 50 mg/mL or 75 mg/mL. In another variation, a pharmaceutical formulation as provided herein comprises a polypeptide at a concentration of at least about any one of 1 mg/mL or 10 mg/mL or 50 mg/mL or 75 mg/mL to about 125 mg/mL or to about 150 mg/mL. In a variation, the pharmaceutical formulations comprising a polypeptide produced by the methods disclosed herein are assessed for color intensity using a color assay such as, but not limited to, the COC assay, the Total Color assay, or the NIFTY assay. In some aspects, a pharmaceutical formulation as provided herein comprises a polypeptide at a concentration greater than at least 100 mg/mL, at least 125 mg/mL, or at least 150 mg/mL and has a color intensity value greater than B3, B4, B5, B6, B7, B8, or B9 as measured by the COC assay. In some aspects, a pharmaceutical formulation as provided herein comprises a polypeptide at a concentration greater than at least 1 mg/mL, at least 10 mg/mL, at least 25 mg/mL, at least 50 mg/mL or at least 75 mg/mL and has a color intensity value greater than B3, B4, B5, B6, B7, B8, or B9 as measured by the COC assay. In some aspects, the color intensity value as determined by the COC assay can be any one of, but not limited to, B, BY, Y, GY, or R, wherein higher values indicate a lighter color intensity. In some aspects, a pharmaceutical formulation as provided herein comprises a polypeptide at a concentration greater than at least 100 mg/mL, at least 125 mg/mL, or at least 150 mg/mL and has a color intensity value less than a color intensity value of a reference solution as measured by a color assay (*e*.*g*., the Total Color assay or the NIFTY assay). In some aspects, a pharmaceutical formulation as provided herein comprises a polypeptide at a concentration greater than at least 1 mg/mL, at least 10 mg/mL, at least 25 mg/mL, at least 50 mg/mL or at least 75 mg/mL and has a color intensity value less than a color intensity value of a reference solution as measured by a color assay (*e*.*g*., the Total Color assay or the NIFTY assay).

### Exemplary Embodiments

1. A method of culturing cells, comprising the step of contacting
   the cells with a cell culture medium comprising:
   from about 200 mg/L to about 1200 mg/L cystine;
   from about 0.05 mg/L to about 1.0 mg/L vitamin B2;
   from about 0.05 mg/L to about 10.0 mg/L vitamin B6;
   from about 0.05 mg/L to about 12.0 mg/L vitamin B9; and
   from about 0.05 mg/L to about 2.5 mg/L vitamin B12.
2. The method of embodiment 1, comprising the step of
   contacting the cells with a cell culture medium comprising:
   from about 0.7 mM to about 2.5 mM cystine;
   from about 0.11 µM to about 0.72 µM vitamin B2;
   from about 4.5 µM to about 30.0 µM vitamin B6;
   from about 3.4 µM to about 22.0 µM vitamin B9; and
   from about 0.2 µM to about 1.5 µM vitamin B12.
3. The method of embodiment 1 or 2, wherein the cell culture medium further comprises any one or more of vitamin B1, vitamin B3, vitamin B5 and vitamin B7.
4. The method of embodiment 3, wherein the cell culture medium further comprises any one or more of:
   from about 2.0 µM to about 14.0 µM vitamin B1;
   from about 11.0 µM to about 72.0 µM vitamin B3;
   from about 6.8 µM to about 44.0 µM vitamin B5; and
   from about 0.02 µM to about 0.14 µM vitamin B7.
5. The method of any one of embodiments 1-4, wherein the cell culture medium further comprises an iron source.
6. The method of embodiment 5, wherein the iron source is ferric citrate or ferrous sulfate.
7. The method of any one of embodiments 1-6, wherein the cell culture medium comprises ferric citrate at a concentration of from about 2 µM to about 80 µM.
8. The method of any one of embodiments 1-7, wherein the cell culture medium comprises ferric citrate at a concentration of from about 11.0 µM to about 36.0 µM,
9. The method of any one of embodiments 1-8, wherein the cell culture medium further comprises hydrocortisone.
10. The method of embodiment 9, wherein the concentration of hydrocortisone in the cell culture medium is from about 0.05 µM to about 0.25 µM.
11. The method of any one of embodiments 1-10, wherein the cell culture medium is a chemically defined cell culture medium.
12. The method of any one of embodiments 1-10, wherein the cell culture medium is a chemically undefined cell culture medium.
13. The method of any one of embodiments 1-12, wherein the cells are contacted with the cell culture medium during the cells' growth phase.
14. The method of any one of embodiments 1-13, wherein the cells are contacted with the cell culture medium during the cells' production phase.
15. The method of embodiment 14, wherein the method further comprises a step of adding cysteine to the cell culture medium.
16. The method of embodiment 15, wherein the cysteine is added in an amount to provide from about 80 mg/L to about 1500 mg/L cysteine in the cell culture medium.
17. The method of embodiment 15, wherein the cysteine is added in an amount to provide about 1500 mg/L cysteine in the cell culture medium.
18. The method of embodiment 15, wherein the cysteine is added in an amount to provide about 140 mg/L cysteine in the cell culture medium.
19. A method of producing a polypeptide comprising the step of culturing in a cell culture medium a cell comprising an isolated nucleic acid encoding the polypeptide, wherein:
   (a) the cell culture medium comprises:
      from about 200 mg/L to about 1200 mg/L cystine;
      from about 0.05 mg/L to about 1.0 mg/L vitamin B2;
      from about 0.05 mg/L to about 10.0 mg/L vitamin B6;
      from about 0.05 mg/L to about 12.0 mg/L vitamin B9;
      from about 0.05 mg/L to about 2.5 mg/L vitamin B12; and
   (b) the cell expresses the polypeptide.
20. The method of embodiment 19, wherein the cell culture medium comprises:
   from about 0.7 mM to about 2.5 mM cystine;
   from about 0.11 µM to about 0.72 µM vitamin B2;
   from about 4.5 µM to about 30.0 µM vitamin B6;
   from about 3.4 µM to about 22.0 µM vitamin B9; and
   from about 0.2 µM to about 1.5 µM vitamin B12.
21. The method of embodiment 19 or 20, wherein the cell culture medium further comprises any one or more of vitamin B1, vitamin B3, vitamin B5 and vitamin B7.
22. The method of embodiment 21, wherein the cell culture medium further comprises any one or more of:
   from about 2.0 µM to about 14.0 µM vitamin B1;
   from about 11.0 µM to about 72.0 µM vitamin B3;
   from about 6.8 µM to about 44.0 µM vitamin B5; and
   from about 0.02 µM to about 0.14 µM vitamin B7.
23. The method of any one of embodiments 19-22, wherein the cell culture medium further comprises an iron source.
24. The method of embodiment 23, wherein the iron source is ferric citrate or ferrous sulfate.
25. The method of any one of embodiments 19-24, wherein the cell culture medium comprises ferric citrate at a concentration of from about 2 µM to about 80 µM.
26. The method of any one of embodiments 19-25, wherein the cell culture medium comprises ferric citrate at a concentration of from about 11.0 µM to about 36.0 µM.
27. The method of any one of embodiments 19-26, wherein the cell culture medium further comprises hydrocortisone.
28. The method of embodiment 27, wherein the concentration of hydrocortisone in the cell culture medium is from about 0.05 µM to about 0.25 µM.
29. The method of any one of embodiments 19-28, wherein the cell culture medium is a chemically defined cell culture medium.
30. The method of any one of embodiments 19-28, wherein the cell culture medium is a chemically undefined cell culture medium.
31. The method of any one of embodiments 19-30, wherein the culturing is during the cell's growth phase.
32. The method of any one of embodiments 19-31, wherein the culturing is during the cell's production phase.
33. The method of embodiment 32, wherein the method further comprises a step of adding cysteine to the cell culture medium.
34. The method of embodiment 33, wherein the cysteine is added in an amount to provide from about 80 mg/L to about 1500 mg/L cysteine in the cell culture medium.
35. The method of embodiment 33, wherein the cysteine is added in an amount to provide about 1500 mg/L cysteine in the cell culture medium.
36. The method of embodiment 33, wherein the cysteine is added in an amount to provide about 140 mg/L cysteine in the cell culture medium.
37. The method of any one of embodiments 19-36, wherein the polypeptide is an antibody.
38. The method of embodiment 37, wherein the antibody is an IgG1 antibody.
39. The method of embodiment 37 or 38, wherein the antibody is an anti-VEGF, anti-mesothelin, anti-PCSK9 or anti-Beta7 antibody.
40. The method of any one of embodiments 19-39, further comprising the step of isolating the polypeptide from the cell culture medium.
41. The method of embodiment 40, wherein a composition comprising the isolated polypeptide appears as a colorless or slightly colored liquid.
42. The method of embodiment 41, wherein the composition comprises the isolated polypeptide at a concentration of at least 100 mg/mL.
43. A polypeptide produced by the method of any one of embodiments 19-42.
44. A pharmaceutical composition comprising a polypeptide of embodiment 43 and a pharmaceutically acceptable carrier.
45. A kit for supplementing a cell culture medium with chemically defined constituents, the kit comprising:
   cystine in an amount to provide from about 200 mg/L to about 1200 mg/L cystine in the cell culture medium;
   vitamin B2 in an amount to provide from about 0.05 mg/L to about 1.0 mg/L vitamin B2 in the cell culture medium;
   vitamin B6 in an amount to provide from about 0.05 mg/L to about 10.0 mg/L vitamin B6 in the cell culture medium;
   vitamin B9 in an amount to provide from about 0.05 mg/L to about 12.0 mg/L vitamin B9 in the cell culture medium; and
   vitamin B12 in an amount to provide from about 0.05 mg/L to about 2.5 mg/L vitamin B12.
46. The kit of embodiment 45, the kit comprising:
   cystine in an amount to provide from about 0.7 mM to about 2.5 mM cystine in the cell culture medium;
   vitamin B2 in an amount to provide from about 0.11 µM to about 0.72 µM vitamin B2 in the cell culture medium;
   vitamin B6 in an amount to provide from about 4.5 µM to about 30.0 µM vitamin B6 in the cell culture medium;
   vitamin B9 in an amount to provide from about 3.4 µM to about 22.0 µM vitamin B9 in the cell culture medium; and
   vitamin B12 in an amount to provide from about 0.2 µM to about 1.5 µM vitamin B12 in the cell culture medium.
47. The kit of embodiment 45 or 46, wherein the kit further comprises any one or more of vitamin B1, vitamin B3, vitamin B5 and vitamin B7.
48. The kit of embodiment 47, wherein the kit further comprises any one or more of:
   vitamin B1 in an amount to provide from about 2.0 µM to about 14.0 µM vitamin B1 in the cell culture medium;
   vitamin B3 in an amount to provide from about 11.0 µM to about 72.0 µM vitamin B3 in the cell culture medium;
   vitamin B5 in an amount to provide from about 6.8 µM to about 44.0 µM vitamin B5 in the cell culture medium; and
   vitamin B7 in an amount to provide from about 0.02 µM to about 0.14 µM vitamin B7 in the cell culture medium.
49. The kit of any one of embodiments 45-48, wherein the kit further comprises an iron source.
50. The kit of embodiment 49, wherein the iron source is ferric citrate or ferrous sulfate.
51. The kit of any one of embodiments 45-50, wherein the kit further comprises hydrocortisone.
52. A cell culture medium comprising:
   from about 200 mg/L to about 1200 mg/L cystine;
   from about 0.05 mg/L to about 1.0 mg/L vitamin B2;
   from about 0.05 mg/L to about 10.0 mg/L vitamin B6;
   from about 0.05 mg/L to about 12.0 mg/L vitamin B9; and
   from about 0.05 mg/L to about 2.5 mg/L vitamin B12.
53. The medium of embodiment 52, comprising:
   from about 0.7 mM to about 2.5 mM cystine;
   from about 0.11 µM to about 0.72 µM vitamin B2;
   from about 4.5 µM to about 30.0 µM vitamin B6;
   from about 3.4 µM to about 22.0 µM vitamin B9; and
   from about 0.2 µM to about 1.5 µM vitamin B12.
54. The medium of embodiment 52 or 53, further comprising any one or more of vitamin B1, vitamin B3, vitamin B5 and vitamin B7.
55. The medium of embodiment 54, further comprising any one or more of:
   from about 2.0 µM to about 14.0 µM vitamin B1;
   from about 11.0 µM to about 72.0 µM vitamin B3;
   from about 6.8 µM to about 44.0 µM vitamin B5; and
   from about 0.02 µM to about 0.14 µM vitamin B7.
56. The medium of any one of embodiments 52-55, wherein the cell culture medium further comprises an iron source.
57. The medium of embodiment 56, wherein the iron source is ferric citrate or ferrous sulfate.
58. The medium of embodiment 57, wherein the cell culture medium comprises ferric citrate at a concentration of from about 2 µM to about 80 µM.
59. The medium of embodiment 58, wherein the cell culture medium comprises ferric citrate at a concentration of from about 11.0 µM to about 36.0 µM.
60. The medium of any one of embodiments 52-59, wherein the cell culture medium further comprises hydrocortisone.
61. The medium of embodiment 60, wherein the cell culture medium comprises hydrocortisone at a concentration of from about 0.05 µM to about 0.25 µM.
62. A method of culturing cells, comprising the step of contacting the cells with a cell culture medium comprising:
   from about 200 mg/L to about 1200 mg/L cystine;
   from about 2 µM to about 80 µM ferric citrate; and
   from about 0.05 µM to about 0.5 µM hydrocortisone.
63. The method of embodiment 62, wherein the cell culture medium further comprises vitamin B2, vitamin B6, vitamin B9 and/or vitamin B12.
64. The method of embodiment 62 or 63, wherein the cell culture medium comprises from about 0.05 mg/L to about 1.0 mg/L of vitamin B2.
65. The method of any one of embodiments 62-64, wherein the cell culture medium comprises from about 0.05 mg/L to about 10.0 mg/L of vitamin B6.
66. The method of any one of embodiments 62-65, wherein the cell culture medium comprises from about 0.05 mg/L to about 12.0 mg/L of vitamin B9.
67. The method of any one of embodiments 62-66, wherein the cell culture medium comprises from about 0.05 mg/L to about 2.5 mg/L of vitamin B12.
68. The method of any one of embodiments 62-67, wherein the cell culture medium is a chemically defined cell culture medium.
69. The method of any one of embodiments 62-67, wherein the cell culture medium is a chemically undefined cell culture medium.
70. The method of any one of embodiments 62-69, wherein the cells are contacted with the cell culture medium during the cells' growth phase.
71. The method of any one of embodiments 62-70, wherein the cells are contacted with the cell culture medium during the cells' production phase.
72. The method of embodiment 71, wherein the method further comprises a step of adding cysteine to the cell culture medium.
73. The method of embodiment 72, wherein the cysteine is added in an amount to provide from about 80 mg/L to about 1500 mg/L cysteine in the cell culture medium.
74. The method of embodiment 72, wherein the cysteine is added in an amount to provide about 1500 mg/L cysteine in the cell culture medium.
75. The method of embodiment 72, wherein the cysteine is added in an amount to provide about 140 mg/L cysteine in the cell culture medium.
76. A method of producing a polypeptide comprising the step of culturing in a cell culture medium a cell comprising an isolated nucleic acid encoding the polypeptide, wherein:
   (a) the cell culture medium comprises:
      from about 200 mg/L to about 1200 mg/L cystine;
      from about 2 µM to about 80 µM ferric citrate; and
      from about 0.05 µM to about 0.5 µM hydrocortisone; and
   (b) the cell expresses the polypeptide.
77. The method of embodiment 76, wherein the cell culture medium further comprises vitamin B2, vitamin B6, vitamin B9 and/or vitamin B12.
78. The method of embodiment 76 or 77, wherein the cell culture medium comprises from about 0.05 mg/L to about 1.0 mg/L of vitamin B2.
79. The method of any one of embodiments 76-78, wherein the cell culture medium comprises from about 0.05 mg/L to about 10.0 mg/L of vitamin B6.
80. The method of any one of embodiments 76-79, wherein the cell culture medium comprises from about 0.05 mg/L to about 12.0 mg/L of vitamin B9.
81. The method of any one of embodiments 76-80, wherein the cell culture medium comprises from about 0.05 mg/L to about 2.5 mg/L of vitamin B12.
82. The method of any one of embodiments 76-81, wherein the cell culture medium is a chemically defined cell culture medium.
83. The method of any one of embodiments 76-81, wherein the cell culture medium is a chemically undefined cell culture medium.
84. The method of any one of embodiments 76-83, wherein the culturing is during the cell's growth phase.
85. The method of any one of embodiments 76-84, wherein the culturing is during the cell's production phase.
86. The method of embodiment 85, wherein the method further comprises a step of adding cysteine to the cell culture medium.
87. The method of embodiment 86, wherein the cysteine is added in an amount to provide from about 80 mg/L to about 1500 mg/L cysteine in the cell culture medium.
88. The method of embodiment 86, wherein the cysteine is added in an amount to provide about 1500 mg/L cysteine in the cell culture medium.
89. The method of embodiment 86, wherein the cysteine is added in an amount to provide about 140 mg/L cysteine in the cell culture medium.
90. The method of any one of embodiments 76-89, wherein the polypeptide is an antibody.
91. The method of embodiment 90, wherein the antibody is an IgG1 antibody.
92. The method of embodiment 90 or 91, wherein the antibody is an anti-VEGF, anti-mesothelin, anti-PCSK9 or anti-Beta7 antibody.
93. The method of any one of embodiments 76-92, further comprising the step of isolating the polypeptide from the cell culture medium.
94. The method of embodiment 93, wherein a composition comprising the isolated polypeptide appears as a colorless or slightly colored liquid.
95. The method of embodiment 94, wherein the composition comprises the isolated polypeptide at a concentration of at least 100 mg/mL.
96. A polypeptide produced by the method of any one of embodiments 76-95.
97. A pharmaceutical composition comprising a polypeptide of embodiment 96 and a pharmaceutically acceptable carrier.
98. A kit for supplementing a cell culture medium with chemically defined constituents, the kit comprising:
   cystine in an amount to provide from about 200 mg/L to about 1200 mg/L cystine in the cell culture medium;
   ferric citrate in an amount to provide a concentration of from about 2 µM to about 80 µM ferric citrate in the cell culture medium; and
   hydrocortisone in an amount to provide a concentration of from about 0.05 µM to about 0.5 µM hydrocortisone in the cell culture medium.
99. The kit of embodiment 98, wherein the kit further comprises vitamin B2, vitamin B6, vitamin B9 and/or vitamin B12.
100. The kit of embodiment 98 or 99, wherein the kit comprises vitamin B2 in an amount to provide from about 0.05 mg/L to about 1.0 mg/L of vitamin B2 in the cell culture medium.
101. The kit of any one of embodiments 98-100, wherein the kit comprises vitamin B6 in an amount to provide from about 0.05 mg/L to about 10.0 mg/L of vitamin B6 in the cell culture medium.
102. The kit of any one of embodiments 98-101, wherein the kit comprises vitamin B9 in an amount to provide from about 0.05 mg/L to about 12.0 mg/L of vitamin B9 in the cell culture medium.
103. The kit of any one of embodiments 98-102, wherein the kit comprises vitamin B12 in an amount to provide from about 0.05 mg/L to about 2.5 mg/L of vitamin B12 in the cell culture medium.
104. A cell culture medium comprising:
   from about 200 mg/L to about 1200 mg/L cystine;
   from about 2 µM to about 80 µM ferric citrate; and
   from about 0.05 µM to about 0.5 µM hydrocortisone.
105. The medium of embodiment 104, wherein the medium further comprises vitamin B2, vitamin B6, vitamin B9 and/or vitamin B12.
106. The medium of embodiment 104 or 105, wherein the medium comprises from about 0.05 mg/L to about 1.0 mg/L of vitamin B2.
107. The medium of any one of embodiments 104-106, wherein the medium comprises from about 0.05 mg/L to about 10.0 mg/L of vitamin B6.
108. The medium of any one of embodiments 104-107, wherein the medium comprises from about 0.05 mg/L to about 12.0 mg/L of vitamin B9.
109. The medium of any one of embodiments 104-108, wherein the medium comprises from about 0.05 mg/L to about 2.5 mg/L of vitamin B12.
110. A composition comprising (a) a cell comprising an isolated nucleic acid encoding a polypeptide; and (b) a medium according to any one of embodiments 52-61 and 104-109.
111. A composition comprising: (a) a polypeptide; and (b) a medium according to any one of embodiments 52-61 and 104-109.

The following Examples are provided to illustrate but not to limit the invention.

### EXAMPLES

Media have been identified that produce a protein drug product with acceptable quality attributes, such as color, particularly when the protein product is present as a concentrated solution (*e.g*., to a concentration of at least 100 mg/mL). In some aspects, the protein product is present as a composition comprising at least 1 mg/mL. Methods of culturing cells in the media provided herein are described, as are methods of producing a polypeptide using the media. A media may in one aspect comprise cystine and/or cysteine and vitamins B2, B6, B9 and B12, with the optional addition of an iron source and hydrocortisone. A media may in another aspect comprise cystine and/or cysteine, hydrocortisone and an iron source, with the optional addition of vitamins B2, B6, B9 and B12. Cystine containing compositions are particularly contemplated. Each of the media constituents may be present in any value provided throughout. The media may be chemically defined or chemically undefined. The media may reduce the presence of polypeptide charge variants and/or reduce the presence of reactive oxygen species when used in a method of polypeptide production as compared to the polypeptide produced in different media. The media find use through all phases of cell culture and polypeptide production and may be used in the basal and/or feed medium. A polypeptide produced by any of the methods is provided, as is a pharmaceutical composition comprising a polypeptide produced as detailed herein. In one aspect the pharmaceutical compositions comprise the polypeptide at a concentration of at least or about any of 100 mg/mL, 125 mg/mL and 150 mg/mL. In another aspect the pharmaceutical compositions comprise the polypeptide at a concentration of at least or about any of 1 mg/mL, 10 mg/mL, 25 mg/mL, 50 mg/mL, and 75 mg/mL. Method of making and compositions comprising antibodies are particularly contemplated. Kits for supplementing a cell culture medium with chemically defined constituents is also described.

Throughout process development of drug substances it is important that product quality meets certain industry standards for use in the clinic. Recent trends towards subcutaneous delivery of monoclonal antibodies have required an increase in concentration of the formulated drug substance to ≥ 100 mg/mL. However, at these high concentrations, the color of the drug substance is more intense making it more difficult to meet established quality expectations about the color of the product. As described herein, several modifications to media (whether chemically undefined or CDM) have been identified that can reduce color intensity of the drug substance to meet product quality standards.

The CHO cell line described in the Examples was genetically engineered to secrete a recombinant humanized antibody (referred herein as IgG1 monoclonal antibody) using a dihydrofolate reductase(dhfr)/methotrexate selection method similar to a method previously described in Kaufman et al., Mol. Cell. Biol., 2(11):1304-1319 (1982). The original transfection used the GS selection system with methionine sulfoximine as the selective agent and glutamine free media. The subsequent super-transfection used the dhfr selection system and methotrexate as the selective agent. Prior to initiating growth phase in 2-liter stirred suspension bioreactors, cryogenically frozen ampules of this CHO cell line were thawed and cultivated in chemically-defined medium in shaker flasks for at least two weeks at 37°C in a humidified incubator with 5% CO₂ to obtain a cell culture suspension with good growth and viability characteristics.

### Example 1: Color intensity exhibited in formulations containing antibody isolated from antibody-producing cell lines.

A CHO cell line capable of producing an IgG1 monoclonal antibody (anti-Beta7) was cultured in peptone containing chemically undefined media. The isolated antibody was purified and assayed for color using the standard Clarity, Opalescence and Coloration (COC) assay (Council of Europe. European Pharmacopoeia., 2008, 7th Ed., p. 22). Briefly, the COC assay was performed by using identical tubes of colorless, transparent, neutral glass with a flat base and an internal diameter of 15 mm to 25 mm. A tube was filled up to a depth of 40 mm with a 150 g/L protein solution prepared from purified and concentrated cell culture fluid containing the secreted IgG1 monoclonal antibody. The tube containing the antibody solution was compared to nine reference tubes, each filled with a reference solution ranging from B1 (darkest) to B9 (lightest), by viewing vertically against a white background in diffused daylight. The IgG1 monoclonal antibody solution was measured at a COC value of ≤ B5 (Fig. 1; Formulation I).

A CHO cell line that produces an IgG1 monoclonal antibody (anti-Beta7) was cultured in CDM. For cell media preparation, basal CDM (Media 1) and feed CDM (Media 2) solutions were prepared by combining the components into a single custom formulated blended powder that was dissolved in water and adjusted to a final pH and osmolality that ensured optimal cell growth. Basal Media 1 and feed Media 2 each had an excess of 20 components with components of interest listed in Table A. Some media components such as glucose were not combined into the blended powder but added separately during media preparation. For initiating the growth phase of the cell culture, CHO cells were inoculated at approximately 1.0 x 10⁶ cells/mL in 2-liter stirred bioreactors (Applikon, Foster City, CA) containing 1L of basal Media 1. The cells were cultured in fed-batch mode with addition of 100 mL of feed Media 2 per liter of cell culture fluid at days 3, 6 and 9 for initiation of the production phase. The concentration of glucose was analyzed every day and if the glucose concentration fell below 3 g/L, it was replenished from a 500 g/L stock solution of glucose for prevention of glucose depletion. Reactors were equipped with calibrated dissolved oxygen, pH and temperature probes. Dissolved oxygen was controlled on-line through sparging with air and/or oxygen. pH was controlled through addition of CO₂ or Na₂CO₃ and antifoam was added to the cultures as needed. The cell cultures were maintained at pH 7.0 and a temperature of 37°C from days 0 through 3, and then at 33°C after day 3. The cell cultures were agitated at 275 rpm and the dissolved oxygen level was at 30% of air saturation. Osmolality was monitored using an osmometer from Advanced Instruments (Norwood, MA). In addition, offline pH and metabolite concentrations were also determined daily using a Nova Bioprofile 400 (Nova Biomedical, Waltham, MA). Viable cell density (VCC) and cell viability was measured daily using a ViCell® automated cell counter (Beckman Coulter, Fullerton, CA). Graduated centrifuge tubes (Kimble Science Products, Fullerton, CA) were used to measure packed cell volume (PCV) after centrifugation of cell suspension for 10 min at 700 x g or at 836 x g. PCV was expressed as percent of the total culture volume. At the end of the cell culture duration on day 14, when the amount of protein in the culture was approximately 2-10 g/L, the cell culture fluid was harvested by centrifugation. The monoclonal antibody in the harvested cell culture fluid was purified using protein A affinity chromatography. After purification, concentration of protein in the eluted protein A pool was approximately 5-10 g/L. The protein A pool was further concentrated to 150 g/L using Amicon Centricon centrifugal filter devices (Millipore Corporation, Billerica, MA). Color was measured in the concentrated protein A pool using the standard COC assay. Alternatively, harvested cell culture fluid was purified using a standard antibody purification process, which included affinity purification through protein A affinity chromatography, further purification through anion and cation exchange chromatography, filtration for removal of virus, and a final ultrafiltration and diafiltration step for final formulation and concentration of the antibody before measurement of color with the standard COC assay. See Kelley, B. mAbs., 2009, 1(5):443-452. Even though slightly higher, measurement of color in the concentrated protein A pool was broadly indicative of the expected color in the final antibody formulation produced by the described standard antibody purification process. The cell culture fluid was collected daily for determination of antibody titer by centrifuging 1 mL of cell culture fluid before purification with high performance liquid chromatography. The COC assay was performed by using identical tubes of colorless, transparent, neutral glass with a flat base and an internal diameter of 15 mm to 25 mm. Two tubes were each filled up to a depth of 40 mm with a 150 g/L protein solution prepared from purified and concentrated cell culture fluid containing the secreted IgG1 monoclonal antibody. Each tube that contained the antibody solution was compared to nine reference tubes, each filled with a reference solution ranging from B1 (darkest) to B9 (lightest), by viewing vertically against a white background in diffused daylight. Color analysis of the antibody-containing solutions demonstrated a COC value of ≤ B4 or ≤ B3 (Fig. 1; Formulation II and III, respectively).

**Table A. Components of Interest**

| **Media Components** | **Media 1 (Basal)** | **Media 2 (Feed)** |
|---|---|---|
| Ferrous sulfate (µM) | 75 | 0 |
| Vitamin B2 (mg/L) | 1.41 | 10 |
| Vitamin B6/ Pyridoxine (mg/L) | 15.42 | 7 |
| Vitamin B6/ Pyridoxal (mg/L) | 0 | 60 |
| Vitamin B9 (mg/L) | 9.93 | 197 |
| Vitamin B12 (mg/L) | 3.05 | 48 |
| Cysteine (mg/L) | 525 | 1500 |
| Cystine (mg/L) | 0 | 0 |
| Hydrocortisone (nM) | 150 | 0 |

### Example 2: Color intensity of antibodies isolated from antibody-producing cell lines is reduced by alteration of specific components in cell culture media.

Basal Media 1 and feed Media 2 were reformulated to contain decreased concentrations of several nutrients for use in cell culture experiments to determine if the reformulated media could reduce color intensity of the isolated monoclonal IgG1 antibody (anti-Beta7) produced by the CHO cell line. Briefly, basal CDM (Media 3) and feed CDM (Media 4) solutions were prepared by combining the components into a single custom formulated blended powder that was dissolved in water and adjusted to a final pH and osmolality that ensured optimal cell growth. Basal Media 3 and feed Media 4 each had an excess of 20 components with components of interest listed in Table B. Some media components such as glucose were not combined into the blended powder but added separately during media preparation. Similarly, media components that were varied for this study were not included in the blended powders but added separately at appropriate levels during media preparation (Table B). Ferric citrate was added from a 5 g/L ferric citrate stock solution, vitamin B2 was provided as riboflavin powder, vitamin B6 was provided as pyridoxine HCl or as pyridoxal HCl, vitamin B9 was provided as folic acid powder, vitamin B12 was provided as cyanocobalamin powder, cysteine was provided as L-Cysteine monohydrochloride monohydrate powder, cystine was provided as disodium salt monohydrate powder, and hydrocortisone was added from a 150 µM stock solution.

**Table B. Components of Interest**

| **Media Components** | **Media 1 (Basal)** | **Media 2 (Feed)** | **Media 3 (Basal)** | **Media 4 (Feed)** |
|---|---|---|---|---|
| Iron (µM) | 75^{a} | 0 | 18^{b} | 0 |
| Vitamin B2 (mg/L) | 1.41 | 10 | 0.25 | 0 |
| Vitamin B6/ Pyridoxine (mg/L) | 15.42 | 7 | 5.35 | 0 |
| Vitamin B6/ Pyridoxal (mg/L) | 0 | 60 | 0 | 0 |
| Vitamin B9 (mg/L) | 9.93 | 197 | 8.61 | 0 |
| Vitamin B12 (mg/L) | 3.05 | 48 | 1.76 | 0 |
| Cysteine (mg/L) | 525 | 1500 | 0 | 1500 |
| Cystine (mg/L) | 0 | 0 | 480 | 0 |
| Hydrocortisone (nM) | 150 | 0 | 150 | 0 |

| | | | | |
|---|---|---|---|---|
| ^{a} Iron source is ferrous sulfate ^{b} Iron source is ferric citrate | | | | |

For initiating the growth phase of the cell culture, CHO cells were inoculated at approximately 1.0 x 10⁶ cells/mL in 2-liter stirred bioreactors (Applikon, Foster City, CA) containing 1L of basal Media 3. The cells were cultured in fed-batch mode with addition of 100 mL of feed Media 4 per liter of cell culture fluid at days 3, 6 and 9 for initiation of the production phase. The concentration of glucose was analyzed every day and if the glucose concentration fell below 3 g/L, it was replenished from a 500 g/L stock solution of glucose for prevention of glucose depletion. Reactors were equipped with calibrated dissolved oxygen, pH and temperature probes. Dissolved oxygen was controlled on-line through sparging with air and/or oxygen. pH was controlled through addition of CO₂ or Na₂CO₃ and antifoam was added to the cultures as needed. The cell cultures were maintained at pH 7.0 and a temperature of 37°C from days 0 through 3, and then at 35°C after day 3. The cell cultures were agitated at 275 rpm and the dissolved oxygen level was at 30% of air saturation. Osmolality was monitored using an osmometer from Advanced Instruments (Norwood, MA). In addition, offline pH and metabolite concentrations were also determined daily using a Nova Bioprofile 400 (Nova Biomedical, Waltham, MA). Viable cell density (VCC) and cell viability was measured daily using a ViCell® automated cell counter (Beckman Coulter, Fullerton, CA). Graduated centrifuge tubes (Kimble Science Products, Fullerton, CA) were used to measure packed cell volume (PCV) after centrifugation of cell suspension for 10 min at 700 x g or at 836 x g. PCV was expressed as percent of the total culture volume. At the end of the cell culture duration on day 14, when the amount of protein in the culture was approximately 2-10 g/L, the cell culture fluid was harvested by centrifugation. The monoclonal antibody in the harvested cell culture fluid was purified using protein A affinity chromatography. After purification, concentration of protein in the eluted protein A pool was approximately 5-10 g/L. The protein A pool was concentrated to 150 g/L using Amicon Centricon centrifugal filter devices (Millipore Corporation, Billerica, MA). Color was measured in the concentrated protein A pool using the standard COC assay. Harvested cell culture fluid was also purified in parallel using a standard antibody purification process, which included affinity purification through protein A affinity chromatography, further purification through anion and cation exchange chromatography, filtration for removal of virus, and a final ultrafiltration and diafiltration step for final formulation and concentration of the antibody before measurement of color with the standard COC assay. See Kelley, B. mAbs., 2009, 1(5):443-452. Even though slightly higher, measurement of color in the concentrated protein A pool was broadly indicative of the expected color in the final antibody formulation produced by the described standard antibody purification process. The cell culture fluid was collected daily by centrifuging 1 mL of cell culture fluid for determination of antibody titer using high performance liquid chromatography. The COC assay was performed by using identical tubes of colorless, transparent, neutral glass with a flat base and an internal diameter of 15 mm to 25 mm. A tube was filled up to a depth of 40 mm with a 150 g/L protein solution prepared from purified and concentrated cell culture fluid containing the secreted IgG1 monoclonal antibody. The tube containing the antibody solution was compared to seven reference vials, each filled with a reference solution ranging from BY1 (darkest) to BY7 (lightest), in diffused daylight, viewed vertically against a white background. The IgG1 monoclonal antibody solution was measured at a COC value of ≤ BY5 (Fig. 1; Formulation VII). Packed cell volume (PCV) was slightly reduced in cell culture grown in basal Media 3 and feed Media 4 as compared to cell culture grown in basal Media 1 and feed Media 2 (Fig. 2A). This reduction correlated with slightly reduced antibody production (Fig. 2B). An additional experiment to assess the effect of this media on PCV and antibody titer confirmed that PCV was reduced when cells were cultured with basal Media 3 and feed Media 4 as compared to cells cultured with basal Media 1 and feed Media 2 (Fig. 2C). As before, reduction of PCV correlated with reduced antibody production (Fig. 2D).

These results demonstrate that color intensity of the monoclonal IgG1 antibody produced by culturing CHO cells with basal Media 3 and feed Media 4 is reduced as compared to the color intensity of monoclonal IgG1 antibody produced by culturing the cells in basal Media 1 and feed Media 2.

### Example 3: Color intensity of antibodies isolated from antibody-producing cell lines is reduced by alteration of vitamin B levels in cell culture media.

To determine the influence of components that were varied in basal Media 3 and feed Media 4 on color intensity of isolated antibody, the levels of vitamin B2, B6, B9, and B12 were varied while the other media component levels were kept constant. The media was prepared as described in Example 2. The media components that were varied for this study were not included in the blended powders but added separately at appropriate levels during media preparation. Basal Media 5 was formulated to have reduced vitamin levels similar to basal Media 3 and all other components similar to basal Media 1 (Table C). For production of monoclonal IgG1 antibody (anti-Beta7) from CHO cells, the cell culture was fed with basal Media 5 for the initial growth phase and at day 3, 6, and 9 cultured with feed Media 2 or feed Media 4. Cell viability measurements and isolation of the monoclonal IgG1 antibody was performed as described in Example 2. After antibody purification, color intensity and antibody titers were also determined as described in Example 2. The IgG1 monoclonal antibody solution obtained from cells cultured with basal Media 5 and feed Media 2 was measured at a COC value of ≤ B4 (Fig. 1; Formulation V). The IgG1 monoclonal antibody solution obtained from cells cultured with basal Media 5 and feed Media 4 was measured at a COC value of ≤ B4 (Fig. 1; Formulation IV).

**Table C. Media with reduced vitamin B2, B6, B9, and B12 levels**

| **Media Components** | **Media 5 (Basal)** |
|---|---|
| Ferric Sulfate (µM) | 75 |
| Vitamin B2 (mg/L) | 0.25 |
| Vitamin B6/ Pyridoxine (mg/L) | 5.35 |
| Vitamin B6/ Pyridoxal (mg/L) | 0 |
| Vitamin B9 (mg/L) | 8.61 |
| Vitamin B12 (mg/L) | 1.76 |
| Cysteine (mg/L) | 525 |
| Cystine (mg/L) | 0 |
| Hydrocortisone (nM) | 150 |

A full factorial study was performed to assess the contribution of individual vitamin B components (vitamin B2, B6, B9, and B12), on color intensity of isolated antibody. While the level of vitamin B2 was treated as a separate factor, the level of pyroxidine and pyridoxal were combined together in a single factor as vitamin B6 such that the concentration of these two nutrients was varied simultaneously. Similarly, the levels of vitamins B9 and B12 were varied together as a single factor such that the concentration of these two nutrients was varied simultaneously. Several media formulations were prepared including media listed in Tables D, E, and F. For production of monoclonal IgG1 antibody (anti-Beta7) from CHO cells, the cell culture was fed with basal Media 5 and feed Media 4, basal Media 6 and feed Media 7, basal Media 8 and feed Media 9, or basal Media 10 and feed Media 11 in fed-batch mode. Additional cell cultures were fed with basal media containing 1.41 mg/L vitamin B2 and/or 15.42 mg/L vitamin B6 (supplied as pyridoxine) and feed media containing 10 mg/L vitamin B2 and/or 76 mg/L vitamin B6 (7mg/L pyridoxine and 60 mg/L pyridoxal). Cell viability measurements and isolation of the monoclonal IgG1 antibody was performed as described in Example 2. After antibody purification, antibody titers were determined as described in Example 2. Color intensity was determined with a color assay wherein higher numerical values indicate higher color intensity and lower numerical values indicate lower color intensity. For this color assay, referred to herein as the normalized fluorescence intensity (NIFTY) assay, about 50 to 125 µg of monoclonal antibody samples were analyzed by size exclusion chromatography (SEC) using a G3000SWXL column (TOSOH), with an isocratic flow rate of 0.5mL/min. Mobile phase for SEC was 0.2M potassium phosphate, 0.25M potassium chloride, pH 6.2. Column temperature was controlled at 15°C. The SEC eluent was monitored for UV absorption at 280nm and for fluorescence with excitation wavelength at 350nm and emission wavelength at 425nm. The SEC peaks of monoclonal antibody species were integrated using Agilent Chemstation software on the UV absorbance and the fluorescence emission chromatograms. For each monoclonal antibody sample, the normalized fluorescence was determined by dividing the fluorescence peak area of the main peak by the UV absorbance peak area of the main peak, which corrected the fluorescence response by the antibody mass contribution. The color intensity value was subsequently determined by calculating the ratio of the normalized fluorescence of the test monoclonal antibody sample to that of a reference monoclonal antibody sample containing a COC reading of ≤ B5. Analysis of packed cell volume over time (IVPCV) and antibody titer levels with JMP 8.0.2 statistical software demonstrated that although cell viability and antibody titer levels were only slightly impacted by alteration of vitamin B2 and vitamin B6 levels in both basal and feed media (Fig. 3A and B; middle trend line), increased levels of these two vitamins in the cell culture media significantly increased color intensity in the isolated antibody (Fig. 4; middle trend line). In contrast, increased levels of vitamin B9 and vitamin B12 did not have a significant impact on cell viability, antibody titer levels, or color intensity (Fig. 3 and 4; middle trend line). Additionally, low levels of color intensity were observed in antibodies isolated from cell lines cultured with basal media containing 0.25 mg/L vitamin B2 and 5.35 mg/L vitamin B6 (supplied as pyridoxine) and feed media free of vitamin B2 and vitamin B6.

The effect of vitamins B1, B3, B5, and B7 was also investigated; however, the effect of these nutrients on color intensity of antibody-containing solutions was not significant.

**Table D. Media with vitamin B2 variation**

| **Media Components** | **Media 6 (Basal)** | **Media 7 (Feed)** |
|---|---|---|
| Ferrous Sulfate (µM) | 75 | 0 |
| Vitamin B2 (mg/L) | 1.41 | 10 |
| Vitamin B6/ Pyridoxine (mg/L) | 5.35 | 0 |
| Vitamin B6/ Pyridoxal (mg/L) | 0 | 0 |
| Vitamin B9 (mg/L) | 8.61 | 0 |
| Vitamin B12 (mg/L) | 1.76 | 0 |
| Cysteine (mg/L) | 525 | 1500 |
| Cystine (mg/L) | 0 | 0 |
| Hydrocortisone (nM) | 150 | 0 |

**Table E. Media with vitamin B6 variation**

| **Media Components** | **Media 8 (Basal)** | **Media 9 (Feed)** |
|---|---|---|
| Iron (µM) | 75 | 0 |
| Vitamin B2 (mg/L) | 0.25 | 0 |
| Vitamin B6/ Pyridoxine (mg/L) | 15.42 | 7 |
| Vitamin B6/ Pyridoxal (mg/L) | 0 | 60 |
| Vitamin B9 (mg/L) | 8.61 | 0 |
| Vitamin B12 (mg/L) | 1.76 | 0 |
| Cysteine (mg/L) | 525 | 1500 |
| Cystine (mg/L) | 0 | 0 |
| Hydrocortisone (nM) | 150 | 0 |

**Table F. Media with vitamin B9 and B12 variation**

| **Media Components** | **Media 10 (Basal)** | **Media 11 (Feed)** |
|---|---|---|
| Iron (µM) | 75 | 0 |
| Vitamin B2 (mg/L) | 0.25 | 0 |
| Vitamin B6/ Pyridoxine (mg/L) | 5.35 | 0 |
| Vitamin B6/ Pyridoxal (mg/L) | 0 | 0 |
| Vitamin B9 (mg/L) | 9.93 | 197 |
| Vitamin B12 (mg/L) | 3.05 | 48 |
| Cysteine (mg/L) | 525 | 1500 |
| Cystine (mg/L) | 0 | 0 |
| Hydrocortisone (nM) | 150 | 0 |

### Example 4: Color intensity of antibodies isolated from antibody-producing cell lines is reduced by alteration of iron source and levels in cell culture media

To assess the contribution of iron levels on color intensity of isolated antibody, the level of iron was varied while the other media component levels were kept constant. The media was prepared as described in Example 2. Media components that were varied for this study were not included in the blended powders but added separately at appropriate levels during media preparation. Basal Media 1 was reformulated to have reduced levels of ferrous sulfate in order to produce basal Media 12 containing 18 µM ferrous sulfate and basal Media 13 containing 10 µM ferrous sulfate. For production of monoclonal IgG1 antibody (anti-Beta7) from CHO cells, the cell culture was fed with basal Media 1 and feed Media 2, basal Media 12 and feed Media 2, or basal Media 13 and feed Media 2 in fed-batch mode. Cell viability measurements and isolation of the monoclonal IgG1 antibody was performed as described in Example 2. After antibody purification, antibody titers were determined as described in Example 2. Color intensity was determined with a color assay wherein higher numerical values indicate higher color intensity and lower numerical values indicate lower color intensity. This color assay, also referred to as the NIFTY assay, was implemented as described in Example 3. Analysis of packed cell volume over time (IVPCV) and antibody titer levels with JMP 8.0.2 statistical software demonstrated that although cell viability and antibody titer levels were reduced with lower iron concentration levels as compared to iron at a concentration of 75 µM (Fig. 5A and B), lower iron concentration in the cell culture media significantly decreased color intensity in the isolated antibody (Fig. 5C).

To investigate if the observed effect of cell culture conditions on color was due to intracellular or extracellular phenomena, a series of *in vitro* incubation experiments were conducted. A 1 g/L quantity of the monoclonal antibody was spiked in freshly prepared cell culture medium and incubated at 33°C for up to six days. The cell culture media used for *in vitro* incubation had a ferrous sulfate concentration of 10, 18 and 75 µM. Vitamin B concentrations were kept constant at the reduced levels of 0.25 mg/L vitamin B2, 5.35 mg/L vitamin B6 (5.35 mg/L pyridoxine + 0 mg/L pyridoxal), 8.61 mg/L vitamin B9, and 1.76 mg/L vitamin B12. The incubated mixture was sampled on days 0, 3 and 6 and the antibody was purified via protein A chromatography. The isolated antibody-containing solution was measured for color intensity with the NIFTY assay. An increase in color intensity was observed from 1.0 units on day 0 to 1.8, 1.44 or 1.27 units on day 6 in antibody-containing solutions isolated from cells grown in media containing 75, 18 or 10 µM ferrous sulfate, respectively (Fig. 5D). The increased color formation at higher iron concentrations mirrored the results from the cell culture experiments indicating that extracellular mechanisms play a role in coloration of the antibody.

To further assess the contribution of the iron on color intensity of isolated antibody, the source and level of iron was varied while the other media component levels were kept constant. The media was prepared as described in Example 2. Media components that were varied for this study were not included in the blended powders but added separately at appropriate levels during media preparation. Basal Media 1 was reformulated to have reduced levels of ferrous sulfate in order to produce basal Media 12 containing 18 µM ferrous sulfate and basal Media 13 containing 10 µM ferrous sulfate. Additionally, basal Media 1 was reformulated to have reduced levels of iron as well as different iron sources in order to produce basal Media 14 containing 18 µM ferric nitrate, basal Media 15 containing 18 µM ferric citrate and basal Media 16 containing 10 µM ferric citrate. For production of monoclonal IgG1 antibody (anti-Beta7) from CHO cells, the cell culture was fed with basal Media 1 and feed Media 2, basal Media 12 and feed Media 2, basal Media 13 and feed Media 2, basal Media 14 and feed Media 2, basal Media 15 and feed Media 2, or basal Media 16 and feed Media 2 in fed-batch mode. Cell viability measurements and isolation of the monoclonal IgG1 antibody was performed as described in Example 2. After antibody purification and antibody titers were also determined as described in Example 2. Color intensity was determined with a color assay wherein higher numerical values indicate higher color intensity and lower numerical values indicate lower color intensity. This color assay, also referred to as the NIFTY assay, was implemented as described in Example 3. Analysis of packed cell volume over time (IVPCV) and antibody titer levels with JMP 8.0.2 statistical software demonstrated that cell viability and antibody titer levels were reduced with lower ferrous sulfate concentration levels as compared to ferrous sulfate at a concentration of 75 µM (Fig. 6A and B). Cell viability and antibody titer levels from cell culture using media containing 18 µM ferric citrate was comparable to media containing 75 µM ferrous sulfate (Fig. 6A and B). However, use of 18 µM ferric citrate in the cell culture media significantly decreased color intensity in the isolated antibody as compared to ferrous sulfate at all concentrations tested (Fig. 6C). Cell viability, antibody titers, and color of antibody isolated from cell culture grown in media containing 18 µM ferric nitrate was comparable to that observed with use of media containing 10µM or 18 µM ferrous sulfate (Fig. 6A-C).

Reduced vitamin B levels and reduced iron concentrations were combined to test whether the beneficial effects on color due to vitamins and iron were additive. Basal Media 13, Media 14, Media 15, and Media 16 were reformulated to have reduced levels of vitamin B levels in order to produce basal Media 21, Media 22, Media 23, and Media 25, respectively. Additionally, basal Media 1 was reformulated to have reduced vitamin B levels and 10 µM ferric nitrate as the iron source in order to produce basal Media 24. Reduced vitamin levels for Media 21 through 25 were as follows: 0.25 mg/L vitamin B2, 5.35 mg/L vitamin B6 (5.35 mg/L pyridoxine + 0 mg/L pyridoxal), 8.61 mg/L vitamin B9, and 1.76 mg/L vitamin B12. For production of monoclonal IgG1 antibody from CHO cells, the cell culture was fed with basal Media 21, 22, 23, 24, or 25 and with feed Media 2 in fed-batch mode. Isolation of the monoclonal IgG1 antibody and measurement of antibody titers was performed as described in Example 2. Color intensity was measured with the NIFTY assay. Analysis of antibody titer levels and color intensity demonstrated that when the ferrous sulfate concentration was lowered from the 75µM to 18µM and combined with lower vitamin concentrations, the resulting color and titer were lower than when reducing one of the factors alone (Fig. 6D and E, plus signs). However, there appeared to be no further benefit in reducing iron concentration from 18 µM to 10 µM for either ferric nitrate or ferrous sulfate (Fig. 6D and E, plus signs).

To investigate the contribution of reactive oxygen species (ROS) to antibody color intensity, *in vitro* experiments were performed by spiking a 2 g/L sample of monoclonal IgG1 antibody into a vial containing cell culture media supplemented with 75 µM or 18 µM ferrous sulfate. Additional *in vitro* experiments were performed by spiking a 2 g/L sample of monoclonal IgG1 antibody (anti-Beta7) into a vial containing cell culture media supplemented with 1.41 mg/L vitamin B2 or 0.25 mg/L vitamin B2. The samples were incubated at 37°C for 5 days without any cells in the absence or presence of 203 U/ml catalase. Color intensity was determined with a color assay wherein higher numerical values indicate higher color intensity and lower numerical values indicate lower color intensity. This color assay, also referred to as the NIFTY assay, was implemented as described in Example 3. Analysis of color levels with JMP 8.0.2 statistical software demonstrated that while increased levels of iron increased color intensity of the antibody solution (Fig. 7A) this increase in color was reduced by addition of catalase (Fig. 7B; middle trend line). In contrast, although increased levels of vitamin B2 increased color intensity of the antibody solution, the color intensity was not reduced by addition of catalase (Fig. 7A and B; middle trend line).

### Example 5: Formation of acidic charge variants of antibodies isolated from antibody-producing cell lines is reduced by alteration of specific components in cell culture media.

For production of monoclonal IgG1 antibody (anti-Beta7) from CHO cells, the cells were cultured with one of three reformulated basal Media 1 solutions, each containing either 10 µM, 18 µM or 75 µM ferrous sulfate. Additional cell cultures were fed (*i.e*., were cultured) with one of two reformulated basal Media 3 solutions, each containing either 10 µM or 18 µM ferric citrate. All cell cultures were fed iron-free feed media in fed-batch mode. Isolation of the monoclonal IgG1 antibody and antibody titer determination was performed as described in Example 2. Color intensity was determined with a color assay wherein higher numerical values indicate higher color intensity and lower numerical values indicate lower color intensity. This color assay, also referred to as the NIFTY assay, was implemented as described in Example 3. For detection of antibody acidic charge variants in the purified solution, charge heterogeneity of monoclonal antibodies was analyzed by ion exchange chromatography (IEC) using a Dionex ProPac WCX-10 (4x250 mm) column. The analysis was performed on an Agilent 1100 HPLC system with the effluent monitored at 280 nm. Mobile phase A was 25 mM sodium phosphate (pH 6.6) and mobile phase B was 150 mM sodium sulfate in mobile phase A. A linear gradient of 0 to 37% B in 45 minutes with a flow rate of 0.5 mL/min was employed. The column temperature was controlled at 40°C. The C-terminal lysine residues on the heavy chains of monoclonal antibodies were removed by Carboxypeptidase B before the IEC analysis to reduce the complexity of charge heterogeneity in the basic region. Analysis of color intensity as a relationship to presence of acidic charge variants using JMP 8.0.2 statistical software demonstrated a strong correlation between high color intensity and increased levels of acidic charge variants in the antibody solution (Fig. 8A). Furthermore, the presence of acidic charge variants was significantly reduced in antibodies isolated from cell lines cultured with modified Media 3 and Media 4 as compared to cell lines cultured with modified Media 1 and Media 2 with the greatest reduction observed at the lowest concentrations of iron (Fig. 8A).

The correlation between color intensity and formation of acidic charge variants was examined in antibody-containing solutions obtained from cell lines cultured with media containing varying iron or vitamin B levels. Monoclonal IgG1 antibodies were produced from CHO cells cultured in basal media containing 18 µM or 75 µM ferrous sulfate or in basal media containing low, medium or high levels vitamin B levels while maintaining the concentration of iron at 18 µM. Low vitamin levels were as follows: 0.25 mg/L vitamin B2, 5.35 mg/L vitamin B6 (5.35 mg/L pyridoxine + 0 mg/L pyridoxal), 8.61 mg/L vitamin B9, and 1.76 mg/L vitamin B12. Medium vitamin levels were as follows: 0.70 mg/L vitamin B2, 7.7 mg/L vitamin B6 (7.7 mg/L pyridoxine + 0 mg/L pyridoxal), 4.9 mg/L vitamin B9, and 1.5 mg/L vitamin B12. High vitamin levels were as follows: 1.41 mg/L vitamin B2, 15.42 mg/L vitamin B6 (15.42 mg/L pyridoxine + 0 mg/L pyridoxal), 9.93 mg/L vitamin B9, and 3.05 mg/L vitamin B12. Isolation of monoclonal IgG1 antibodies and antibody titer determination was performed as described in Example 2. Color intensity was determined using the Total Color assay. For the Total Color assay, a quantitative value of the relative color of samples was derived by using the CIE System of color measurement as described in Berns et al., Billmeyer and Saltzman's Principles of Color Technology, 3rd Edition. New York, NY, John Wiley & Sons, Inc., (2000). Briefly, after blanking with water, the absorption spectrum of a neat test sample was measured in the visible region (380-780nm) using a HP8453A spectrophotometer (1cm pathlength cuvette). The absorption spectrum was then converted to the CIE L*a*b* color scale as previously described in Standard Practice for Calculation of Color Tolerances and Color Differences from Instrumentally Measured Color Coordinates, Annual Book of ASTM Standards, Vol. 06.01, (2011). For these calculations an artificial flat spectrum in the visible region was used as the illuminant. The "Total Color" represented the Delta E which corresponded to the Euclidian distance between the test sample and water in the three dimensional CIE L*a*b* color space. In addition, the "Total Color" represented the overall color of the test monoclonal antibody sample without differentiating between differing hues. The color intensity value was subsequently determined by calculating the ratio of the "Total Color" measurement of the test monoclonal antibody sample to that of a reference monoclonal antibody sample containing a COC reading of ≤ B5. A positive correlation was seen between increased iron concentration and increased color intensity (Fig. 8B). Furthermore, increased iron concentrations resulted in increased levels of acidic charge variants. In comparison, simultaneously varying the levels of vitamin B2, B6, B9, and B12 in media containing a constant concentration of iron showed no correlation between acidic charge variants and color intensity (Fig. 8C).

The correlation of color intensity and formation of acidic charge variants in antibodies isolated from cell lines cultured with cell media containing varying levels of B2 and B6 was examined. For production of monoclonal IgG1 antibody (anti-Beta7) from CHO cells, the cell culture was fed with basal and feed media containing varying levels of vitamin B2 and B6 in fed-batch mode. The antibodies were subsequently isolated and measured for color intensity using the NIFTY assay as described in Example 3, in addition to the presence of acidic charge variants. Analysis of color intensity as a relationship to presence of acidic charge variants using JMP 8.0.2 statistical software demonstrated a strong correlation between high color intensity and increased levels of acidic charge variants in antibodies isolated from cell lines cultured with basal media containing 1.41 mg/L vitamin B2 and/or 15.42 mg/L vitamin B6 (supplied as pyridoxine) and feed media containing 10 mg/L vitamin B2 and/or 76 mg/L vitamin B6 (7mg/L pyridoxine and 60 mg/L pyridoxal)(Fig. 9A and B). Lowest levels of color intensity and acidic charge variants was observed in antibodies isolated from cell lines cultured with basal media containing 0.25 mg/L vitamin B2 and 5.35 mg/L vitamin B6 (supplied as pyridoxine) and feed media free of vitamin B2 and vitamin B6 (Fig. 9A and B).

A full factorial study was performed to determine the correlation of color intensity and formation of acidic charge variants in antibodies isolated from cell lines cultured with cell media containing varying levels of pyridoxal in the presence of different iron sources. For production of monoclonal IgG1 antibody (anti-Beta7) from CHO cells, the cells were cultured with basal and feed media containing varying levels of pyridoxal. Ferric citrate or ferrous sulfate was provided in the basal media and the cell culture process was performed in fed-batch mode. The antibodies were subsequently isolated and measured for color intensity using the Total Color assay, in addition to the presence of acidic charge variants. Analysis of color intensity as a relationship to presence of acidic charge variants using JMP 8.0.2 statistical software demonstrated a strong correlation between higher color intensity and increased levels of acidic charge variants in antibodies isolated from cell lines cultured with basal media containing 0 mg/L pyridoxal with 18 µM ferrous sulfate and feed media containing 0 mg/L or 60 mg/L pyridoxal as compared to antibodies isolated from cell lines cultured with basal media containing 0 mg/L pyridoxal with 18µM ferric citrate and feed media containing 0 mg/L or 60 mg/L pyridoxal (Fig. 10A and B).

The correlation of color intensity and formation of acidic charge variants in antibodies isolated from cell lines cultured with cell media containing varying levels of vitamin B2, B6, B9 and B12 in the presence of different iron sources was examined. For production of monoclonal IgG1 antibody (anti-Beta7) from CHO cells, the cells were cultured with a basal media containing 1.41 mg/L vitamin B2, 15.42 mg/L pyridoxine, 0 mg/L pyridoxal, 9.93 mg/L vitamin B9, and 3.05 mg/L vitamin B12, and one of three different feed media, each containing varying levels of vitamins B2, B6, B9 and B12. Ferric citrate or ferrous sulfate was provided in the basal media and the cell culture process was performed in fed-batch mode. The antibodies were subsequently isolated and measured for color intensity, using the Total Color assay, in addition to the presence of acidic charge variants. Analysis of color intensity as a relationship to presence of acidic charge variants using JMP 8.0.2 statistical software demonstrated reduced color intensity in antibodies isolated from cell lines cultured with feed media containing 5 mg/L vitamin B2, 3.5 mg/L pyridoxine, 30 mg/L pyridoxal, 98.5 mg/L vitamin B9, and 24 mg/L vitamin B12 in the presence of either 18 µM ferrous sulfate or 18 µM ferric citrate as compared to feed media containing 10 mg/L vitamin B2, 7 mg/L pyridoxine, 60 mg/L pyridoxal, 197 mg/L vitamin B9, and 48 mg/L vitamin B12 (Fig. 11A; vitamin level 2 and vitamin level 3, respectively). The greatest reduction in color intensity was seen in antibodies isolated from cells cultured with feed media that lacked vitamin B2, B6, B9, and B12 (Fig. 11A; vitamin level 1). Although, there was significant reduction in antibody color intensity with decreased concentrations of vitamin B2, B6, B9, and B12, preliminary results indicated there was no significant change in the presence of acidic charge variants (Fig. 11B).

The correlation of color intensity and formation of acidic charge variants in antibodies isolated from cell lines cultured with cell media containing increasing concentrations of iron from different sources was examined. For production of monoclonal IgG1 antibody (anti-Beta7) from CHO cells, the cells were cultured with basal Media 1 containing 75 µM ferrous sulfate, basal Media 12 containing 18 µM ferrous sulfate or basal Media 13 containing 10 µM ferrous sulfate in fed-batch mode. Additional cell cultures were fed basal Media 15 containing 18 µM ferric citrate or basal Media 16 containing 10 µM ferric citrate. All cell cultures were fed iron-free feed media in fed-batch mode. Color intensity was determined using the NIFTY assay as described in Example 3. The greatest reduction in color intensity was seen in antibodies isolated from cells cultured with basal media containing reduced levels of iron (Fig. 12A). Reduced color intensity correlated with a reduced presence of acidic charge variants in the isolated antibody solution (Fig. 12B).

The correlation of color intensity and formation of acidic charge variants in antibodies isolated from cell lines cultured with cell media containing increasing concentration of ferric citrate was examined. For production of monoclonal IgG1 antibody (anti-Beta7) from CHO cells, cells were cultured in modified basal Media 1 or modified basal Media 3 that each contained 18 µM ferric citrate or 36 µM ferric citrate. All cell cultures were fed iron-free feed media in fed-batch mode. Monoclonal IgG1 antibody was isolated from cell cultures incubated at 33°C or 37°C. Color intensity was determined with a color assay, specifically the Total Color assay, wherein higher numerical values indicate higher color intensity and lower numerical values indicate lower color intensity. The greatest reduction in color intensity was seen in antibodies isolated from cells cultured with basal media containing reduced levels of ferric citrate (Fig. 13A). Reduced color intensity correlated with a reduced presence of acidic charge variants in the isolated antibody solution. Antibodies isolated from cells cultured at 33°C demonstrated the greatest reduction of acidic charge variants (Fig. 13B). Increasing the temperature from 33°C to 37°C resulted in a significant increase in antibody production of approximately 2500 mg/L to 4250 mg/L by antibody-producing cells cultured in either modified basal Media 1 or modified basal Media 3.

Basal Media 3 was reformulated to contain 525 mg/L of cysteine instead of 480 mg/L of cystine for use in cell culture experiments to determine the contribution of cysteine to color intensity of the isolated monoclonal IgG1 antibody (anti-Beta7) produced by the CHO cell line. For production of monoclonal IgG1 antibody from CHO cells, cells were cultured in the modified basal Media 3 containing 525 mg/L cysteine and feed Media 4 in fed-batch mode. The antibodies were isolated and color was measured using the standard COC assay. The COC assay was performed by using identical tubes of colorless, transparent, neutral glass with a flat base and an internal diameter of 15 mm to 25 mm. Two tubes were each filled up to a depth of 40 mm with a 150 g/L protein solution prepared from purified and concentrated cell culture fluid containing the secreted IgG1 monoclonal antibody. Each tube that contained the antibody solution was either compared to seven reference vials, each filled with a reference solution ranging from BY1 (darkest) to BY7 (lightest) or to nine reference vials, each filled with a reference solution ranging from B1 (darkest) to B9 (lightest), in diffused daylight viewed vertically against a white background. Color analysis of the antibody-containing solutions demonstrated a COC value of ≤ B5 or ≤ BY5 (Fig. 1; Formulation VI and VIII, respectively). Antibody Formulation VI was further assayed for color intensity using the Total Color assay as described above and the NIFTY assay as described in Example 3. Color analysis of the antibody formulation demonstrated a color intensity value of 0.71 and 1.00 when measured with the NIFTY assay and Total Color assay, respectively. This antibody formulation was lighter in color as compared to color intensity in antibody Formulation III (described in Example 1) and antibody Formulation IV (described in Example 3). Antibody Formulation III demonstrated a color intensity value of 1.59 and 2.61 when measured with the NIFTY assay and Total Color assay, respectively. Antibody Formulation IV demonstrated a color intensity value of 1.47 and 2.04 when measured with the NIFTY assay and Total Color assay, respectively.

A multivariate study was performed to determine the correlation of color intensity and formation of acidic charge variants in antibodies isolated from cell lines cultured with cell media containing varying concentrations of vitamins B2, B6, B9, and B12 in the presence or absence of cystine or cysteine, and in the presence or absence of hydrocortisone. For production of monoclonal IgG1 antibody (anti-Beta7) from CHO cells, cells were cultured in basal media containing 1.41 mg/L vitamin B2, 15.42 mg/L pyridoxine, 0 mg/mL pyridoxal, 9.93 mg/L vitamin B9, and 3.05 mg/L vitamin B12 or in basal media containing 0.7 mg/L vitamin B2, 7.7 mg/L pyridoxine, 0 mg/L pyridoxal, 4.9 mg/L vitamin B9, and 1.5 mg/L vitamin B12. In addition, the basal media contained either 525 mg/L cysteine or 480 mg/L cystine (Table G). Additional basal media was prepared as in Table G and supplemented with 150 nM hydrocortisone. All cell cultures were fed iron-free feed media in fed-batch mode.

**Table G. Multivariate component experiment**

| **Media Components** | **Media 17 (Basal)** | **Media 18 (Basal)** | **Media 19 (Basal)** | **Media 20 (Basal)** |
|---|---|---|---|---|
| Vitamin B2 (mg/L) | 1.41 | 1.41 | 0.7 | 0.7 |
| Vitamin B6/ Pyridoxine (mg/L) | 15.42 | 15.42 | 7.7 | 7.7 |
| Vitamin B6/ Pyridoxal (mg/L) | 0 | 0 | 0 | 0 |
| Vitamin B9 (mg/L) | 9.93 | 9.93 | 4.9 | 4.9 |
| Vitamin B12 (mg/L) | 3.05 | 3.05 | 1.5 | 1.5 |
| Cysteine (mg/L) | 525 | 0 | 525 | 0 |
| Cystine (mg/L) | 0 | 480 | 0 | 480 |

Monoclonal IgG1 antibody was isolated from cell cultures and measured for color intensity as well as presence of acidic charge variants. Color intensity was determined using the Total Color assay as described above. Analysis of color intensity as a relationship to presence of acidic charge variants using JMP 8.0.2 statistical software demonstrated a correlation between reduced color intensity and decreased levels of acidic charge variants in antibodies isolated from cell lines cultured with basal media containing cystine as compared to cysteine (Fig. 14A and B). Furthermore, reduction of vitamin B levels also resulted in decreased color intensity and acidic charge variant formation with addition of hydrocortisone enhancing the reduction (Fig. 14A and B).

### Example 6: Color intensity of monoclonal IgG1 antibody isolated from a cell line was reduced by alteration of specific components in cell culture media.

The color intensity reducing effect of cystine when used in basal cell culture medium was further investigated with a CHO cell line that produced a different monoclonal IgG1 antibody (mAb10). This cell line was cultured in undefined basal media with either the amino acid cysteine in the monomer form at a concentration of 2.6 mM (cysteine) or in the dimer form at a concentration of 1.3 mM (cystine). Production of the mAb10 was initiated in cell culture by inoculating cells in the undefined basal medium and a batch feed medium was added on day 3 over a 14 day cell culture cycle in a bioreactor. The cells were cultured at 37°C on day 1 and a temperature shift was initiated over the course of the cell culture cycle. The media was harvested and mAb10 was recovered as a composition before assessment of color intensity with the COC assay. Compositions comprising mAb10 recovered from cells cultured in basal media containing cysteine appeared as a colorless or slightly colored liquid with a color intensity value of B7 as determined by the COC assay. A composition comprising mAb10 recovered from cells cultured in basal media where cysteine was replaced by cystine appeared as a colorless or slightly colored liquid with an improved color intensity COC value of B8.

In a multivariate study, four different protocols for the production of mAb10 from CHO cells were used to assess the effect of certain media components on color intensity of a composition comprising the antibody recovered from the cell culture (Table H). The levels of iron as well as the iron source in the undefined cell culture basal media differed between the protocols. Vitamin B levels and the amino acid cysteine in the monomer form (cysteine) or in the dimer form (cystine) in the undefined basal media also differed between the protocols. Production of mAb10 was initiated in cell culture by inoculating cells in undefined basal medium and a batch feed medium was added on day 3 over a 14 day cell culture cycle in a bioreactor. The media was harvested and mAb10 was recovered as a composition before assessment of color intensity with the COC assay. Analysis of the color intensity of compositions comprising recovered mAb demonstrated that Protocols 1, 2, and 3 resulted in an antibody composition with reduced color intensity (COC value of B7) as compared to the color intensity of the antibody composition obtained with Protocol 4 (COC value of B6). These results showed that use of cystine instead of cysteine in the basal medium, reduced vitamin B levels, and/or reduced iron as well as a change in the iron source resulted in the reduction of color intensity in the mAb10 compositions (Table H). For example, in Protocol 3 as compared to Protocol 4, reduction of vitamin B levels and substitution of ferric citrate for ferrous sulfate at a lower concentration reduced the color intensity of the antibody composition without the need to change the use of cysteine for cystine. The same effect on reduction of color intensity was observed in Protocol 2 versus Protocol 4, when ferric citrate was substituted for ferrous sulfate at a lower concentration and cystine was substituted for cysteine without alteration in vitamin B levels. Reduction of vitamin B levels, reduction of the iron level and change of iron source as well as use of cystine instead of cysteine as seen in Protocol 1 also reduced the color intensity of a mAb10 composition as compared to a mAb10 composition obtained from Protocol 4 (Table H).

**Table H. Summary of basal media components**

| Component | Protocol 1 | Protocol 2 | Protocol 3 | Protocol 4 |
|---|---|---|---|---|
| Iron (µM) | 25%^{a} | 50%^{a} | 50%^{a} | 100%^{b} |
| Vitamin B1 (µM) | 25% | 100% | 50% | 100% |
| Vitamin B2 (µM) | 25% | 100% | 50% | 100% |
| Vitamin B3 (µM) | 25% | 100% | 50% | 100% |
| vitamin B5 (µM) | 25% | 100% | 50% | 100% |
| vitamin B6 (µM) | 25% | 100% | 50% | 100% |
| vitamin B7 (µM) | 25% | 100% | 50% | 100% |
| vitamin B9 (µM) | 25% | 100% | 50% | 100% |
| vitamin B12 (µM) | 25% | 100% | 50% | 100% |
| Cysteine | 0 mM | 0 mM | 2.6 mM | 2.6 mM |
| Cystine | 1.3 mM | 1.3 mM | 0 mM | 0 mM |
| COC value | B7 | B7 | B7 | B6 |

| | | | | |
|---|---|---|---|---|
| ^{a} indicates ferric citrate as iron source; ^{b} indicates ferrous sulfate as iron source | | | | |

### Example 7: The NIFTY assay and Total Color assay are comparable to the standard COC assay for measurement of color intensity in an antibody-containing solutions.

The recent emphasis on high concentration formulations has generally increased the color intensity of monoclonal antibody liquid formulations. Color is considered a product quality attribute and accordingly, it is important that the color of the drug substance be closely monitored for consistency while developing and implementing process changes. One of the challenges faced for measuring color is the use of appropriate assays. Even though it is the industry standard, the COC assay is not fully quantitative and is vulnerable to the subjective judgment of the person performing the assay. To overcome this problem, two different methods for color measurement, the Total Color assay and the NIFTY assay, were developed and used as described in the Examples to measure color intensity.

The correlation between these three color measurement assays was determined by plotting Total Color values on the abscissa, NIFTY values on the ordinate and with the data points based on the actual COC measurements performed on antibody-containing solutions measured by Total Color and NIFTY assays (Fig. 15A). There was a good correlation between quantitative measurements from Total Color assays and NIFTY assays (R²=0.75). In addition, it could be seen that these assays correlated reasonably well with actual COC measurements indicating that results from Total Color and NIFTY assays were useful predictors of the color intensity of the samples.

Antibodies were harvested and purified from cell culture by two different methods before measurement of color intensity. In one method, the monoclonal antibody in the harvested cell culture fluid was purified using protein A affinity chromatography. After purification, the concentration of protein in the eluted protein A pool was approximately 5-10 g/L. The protein A pool was further concentrated to 150 g/L using Amicon Centricon centrifugal filter devices. Color was measured in the concentrated protein A pool using the standard COC assay, the Total Color assay or the NIFTY assay. In the other method, harvested cell culture fluid was purified using a standard antibody purification process, which included affinity purification through protein A affinity chromatography, further purification through anion and cation exchange chromatography, filtration for removal of virus, and a final ultrafiltration and diafiltration step for final formulation and concentration of the antibody before measurement of color with the standard COC assay, the Total Color assay or the NIFTY assay.

Due to practical considerations, it was decided to use the color of the protein A pool as a proxy for the color of the final drug substance in several of the experiments described in the Examples. The predictive value of the color intensity measured in antibody formulations prepared from the protein A pool for the color intensity that would be obtained from a final fully purified antibody formulation was assessed. Comparison of the color intensity measured by the NIFTY assay (Fig. 15B) or the Total Color assay (Fig. 15C) in antibody-containing solutions obtained from the protein A pool versus the final fully purified antibody formulation demonstrated a reasonably good correlation for Total Color measurements (R²=0.73) and NIFTY measurements (R²=0.98). Total Color is derived from the absorbance spectrum of the pool and hence higher color intensity can be expected in earlier in-process pools due to the presence of non-antibody impurities or due to increased scattering of light. In contrast, NIFTY values are measured from the main peak of the size exclusion chromatogram and hence could be expected to remain constant through the purification process if colored or uncolored protein molecules were not preferentially purified. Overall, reasonable correlations were seen between color measurements between the protein A pools and the formulated drug substance.

## Claims

1. A method of producing a polypeptide comprising the step of culturing in a cell culture medium a cell comprising an isolated nucleic acid encoding the polypeptide, wherein:
a) the cell culture medium comprises:
from about 200 mg/L to about 1200 mg/L cystine;
from about 0.05 mg/L to about 1.0 mg/L vitamin B2;
from about 0.05 mg/L to about 10.0 mg/L vitamin B6;
from about 0.05 mg/L to about 12.0 mg/L vitamin B9;
from about 0.05 mg/L to about 2.5 mg/L vitamin B12; and
ferric citrate or ferrous sulfate;
b) the cell expresses the polypeptide; and
c) the polypeptide is isolated from the cell culture medium, wherein the isolated polypeptide is concentrated to provide a composition comprising a polypeptide at a concentration of at least 100 mg/ml and wherein the composition has a color reference standard value selected from any one of B4-B9, BY4-BY7, Y4-Y7, GY4-GY7 and R4-R7 according to the European Pharmacopoeia color standards, European Pharmacopoeia, 2008, 7^{th} Edition, Page 22.

2. The method of claim 1, wherein a) the cell culture medium comprises from about 300 mg/L to about 1200mg/L cystine.

3. The method of claim 1, wherein the cell culture medium comprises:
from about 0.8 mM to about 2.5 mM cystine;
from about 0.11 µM to about 0.72 µM vitamin B2;
from about 4.5 µM to about 30.0 µM vitamin B6;
from about 3.4 µM to about 22.0 µM vitamin B9; and
from about 0.2 µM to about 1.5 µM vitamin B12.

4. The method of claim 1 to 3, wherein the cell culture medium further comprises any one or more of vitamin B1, vitamin B3, vitamin B5 and vitamin B7.

5. The method of claim 4, wherein the cell culture medium further comprises any one or more of:
from about 2.0 µM to about 14.0 µM vitamin B1;
from about 11.0 µM to about 72.0 µM vitamin B3;
from about 6.8 µM to about 44.0 µM vitamin B5; and
from about 0.02 µM to about 0.14 µM vitamin B7.

6. The method of any one of claims 1-5, wherein the cell culture medium comprises ferric citrate or ferrous sulfate at a concentration of from about 2 µM to about 80 µM.

7. The method of any one of claims 1-6, wherein the cell culture medium further comprises hydrocortisone, optionally at a concentration of from about 0.05 µM to about 0.25 µM.

8. The method of any one of claims 1-7, wherein the polypeptide is an antibody, optionally wherein the antibody is an IgG1 antibody.

9. The method of claim 8, wherein the antibody is an anti-VEGF, anti-mesothelin, anti-PCSK9 or anti-Beta7 antibody.

10. The method of claim 1, wherein the isolated polypeptide is concentrated to provide a composition comprising a polypeptide at a concentration of at least 125 mg/ml and wherein the composition has a color reference standard value selected from any one of B4-B9, BY4-BY7, Y4-Y7, GY4-GY7 and R4-R7 according to the European Pharmacopoeia color standards, European Pharmacopoeia, 2008, 7^{th} Edition, Page 22.

11. The method of claim 1, wherein the isolated polypeptide is concentrated to provide a composition comprising a polypeptide at a concentration of at least 150 mg/ml and wherein the composition has a color reference standard value selected from any one of B4-B9, BY4-BY7, Y4-Y7, GY4-GY7 and R4-R7 according to the European Pharmacopoeia color standards, European Pharmacopoeia, 2008, 7^{th} Edition, Page 22.

12. The method of claim 1, wherein the color of the isolated polypeptide is determined by an assay selected from the group consisting of the COC assay, the Total Color assay, and the NIFTY assay.

## Patentansprüche

1. Verfahren zum Herstellen eines Polypeptids, umfassend den Schritt des Kultivierens einer Zelle, umfassend eine isolierte, für das Polypeptid codierende Nukleinsäure, in einem Zellkulturmedium, wobei:
a) das Zellkulturmedium Folgendes umfasst:
von etwa 200 mg/l bis etwa 1200 mg/l Cystin;
von etwa 0,05 mg/l bis etwa 1,0 mg/l Vitamin B2;
von etwa 0,05 mg/l bis etwa 10,0 mg/l Vitamin B6;
von etwa 0,05 mg/l bis etwa 12,0 mg/l Vitamin B9;
von etwa 0,05 mg/l bis etwa 2,5 mg/l Vitamin B12; und
Ferricitrat und Ferrosulfat;
b) die Zelle das Polypeptid exprimiert; und
c) das Polypeptid aus dem Zellkulturmedium isoliert wird, wobei das isolierte Polypeptid konzentriert wird, um eine Zusammensetzung zu ergeben, umfassend ein Polypeptid mit einer Konzentration von mindestens 100 mg/ml, und wobei die Zusammensetzung einen Farbreferenzstandardwert aufweist, ausgewählt aus einem von B4-B9, BY4-BY7, Y4-Y7, GY4-GY7 und R4-R7 gemäß den Farbstandards der Europäischen Pharmakopöe, Europäische Pharmakopöe, 2008, 7. Auflage, Seite 22.

2. Verfahren nach Anspruch 1, wobei a) das Zellkulturmedium von etwa 300 mg/l bis etwa 1200 mg/l Cystin umfasst.

3. Verfahren nach Anspruch 1, wobei das Zellkulturmedium Folgendes umfasst:
von etwa 0,8 mM bis etwa 2,5 mM Cystin;
von etwa 0,11 µM bis etwa 0,72 µM Vitamin B2;
von etwa 4,5 µM bis etwa 30,0 µM Vitamin B6;
von etwa 3,4 µM bis etwa 22,0 µM Vitamin B9; und
von etwa 0,2 µM bis etwa 1,5 µM Vitamin B12.

4. Verfahren nach Anspruch 1 bis 3, wobei das Zellkulturmedium ferner eines oder mehrere von Vitamin B1, Vitamin B3, Vitamin B5 und Vitamin B7 umfasst.

5. Verfahren nach Anspruch 4, wobei das Zellkulturmedium ferner eines oder mehrere der Folgenden umfasst:
von etwa 2,0 µM bis etwa 14,0 µM Vitamin B1;
von etwa 11,0 µM bis etwa 72,0 µM Vitamin B3;
von etwa 6,8 µM bis etwa 44,0 µM Vitamin B5; und
von etwa 0,02 µM bis etwa 0,14 µM Vitamin B7.

6. Verfahren nach einem der Ansprüche 1-5, wobei das Zellkulturmedium Ferricitrat oder Ferrosulfat in einer Konzentration von etwa 2 µM bis etwa 80 µM umfasst.

7. Verfahren nach einem der Ansprüche 1-6, wobei das Zellkulturmedium ferner Hydrocortison umfasst, gegebenenfalls in einer Konzentration von etwa 0,05 µM bis etwa 0,25 µM,

8. Verfahren nach einem der Ansprüche 1-7, wobei das Polypeptid ein Antikörper ist, gegebenenfalls wobei der Antikörper ein IgG1-Antikörper ist.

9. Verfahren nach Anspruch 8, wobei der Antikörper ein Anti-VEGF-, Anti-Mesothelin-, Anti-PCSK9- oder Anti-Beta7-Antikörper ist.

10. Verfahren nach Anspruch 1, wobei das isolierte Polypeptid konzentriert wird, um eine Zusammensetzung zu ergeben, umfassend ein Polypeptid mit einer Konzentration von mindestens 125 mg/ml, und wobei die Zusammensetzung einen Farbreferenzstandardwert aufweist, ausgewählt aus einem von B4-B9, BY4-BY7, Y4-Y7, GY4-GY7 und R4-R7 gemäß den Farbstandards der Europäischen Pharmakopöe, Europäische Pharmakopöe, 2008, 7. Auflage, Seite 22.

11. Verfahren nach Anspruch 1, wobei das isolierte Polypeptid eingeengt wird, um eine Zusammensetzung zu ergeben, umfassend ein Polypeptid mit einer Konzentration von mindestens 150 mg/ml, und wobei die Zusammensetzung einen Farbreferenzstandardwert aufweist, ausgewählt aus einem von B4-B9, BY4-BY7, Y4-Y7, GY4-GY7 und R4-R7 gemäß den Farbstandards der Europäischen Pharmakopöe, Europäische Pharmakopöe, 2008, 7. Auflage, Seite 22.

12. Verfahren nach Anspruch 1, wobei die Farbe des isolierten Polypeptids mittels eines Assays bestimmt wird, ausgewählt aus der Gruppe bestehend aus dem COC-Assay, dem Total-Color-Assay und dem NIFTY-Assay.

## Revendications

1. Procédé de production d'un polypeptide comprenant l'étape de mise en culture, dans un milieu de culture cellulaire, d'une cellule comprenant un acide nucléique isolé codant pour le polypeptide, dans lequel :
a) le milieu de culture cellulaire comprend :
d'environ 200 mg/L à environ 1 200 mg/L de cystine ;
d'environ 0,05 mg/L à environ 1,0 mg/L de vitamine B2 ;
d'environ 0,05 mg/L à environ 10,0 mg/L de vitamine B6 ;
d'environ 0,05 mg/L à environ 12,0 mg/L de vitamine B9 ;
d'environ 0,05 mg/L à environ 2,5 mg/L de vitamine B12 ; et
du citrate ferrique ou du sulfate ferreux ;
b) la cellule exprime le polypeptide ; et
c) le polypeptide est isolé du milieu de culture cellulaire, dans lequel le polypeptide isolé est concentré pour fournir une composition comprenant un polypeptide à une concentration d'au moins 100 mg/ml et dans lequel la composition a une valeur standard de référence de couleur sélectionnée parmi l'une quelconque de B4-B9, BY4-BY7, Y4-Y7, GY4-GY7 et R4-R7 selon les standards de couleurs de la pharmacopée européenne, Pharmacopée européenne, 2008, 7^{e} édition, page 22.

2. Procédé selon la revendication 1, dans lequel a) le milieu de culture cellulaire comprend d'environ 300 mg/L à environ 1 200 mg/L de cystine.

3. Procédé selon la revendication 1, dans lequel le milieu de culture cellulaire comprend :
d'environ 0,8 mM à environ 2,5 mM de cystine ;
d'environ 0,11 µM à environ 0,72 µM de vitamine B2 ;
d'environ 4,5 µM à environ 30,0 µM de vitamine B6 ;
d'environ 3,4 µM à environ 22,0 µM de vitamine B9 ; et
d'environ 0,2 µM à environ 1,5 µM de vitamine B12.

4. Procédé selon les revendications 1 à 3, dans lequel le milieu de culture cellulaire comprend en outre un ou plusieurs parmi la vitamine B1, la vitamine B3, la vitamine B5 et la vitamine B7.

5. Procédé selon la revendication 4, dans lequel le milieu de culture cellulaire comprend en outre un ou plusieurs des suivants :
d'environ 2,0 µM à environ 14,0 µM de vitamine B1 ;
d'environ 11,0 µM à environ 72,0 µM de vitamine B3 ;
d'environ 6,8 µM à environ 44,0 µM de vitamine B5 ; et
d'environ 0,02 µM à environ 0,14 µM de vitamine B7.

6. Procédé selon l'une quelconque des revendications 1 à 5, dans lequel le milieu de culture cellulaire comprend du citrate ferrique ou du sulfate ferreux en une concentration d'environ 2 µM à environ 80 µM.

7. Procédé selon l'une quelconque des revendications 1 à 6, dans lequel le milieu de culture cellulaire comprend en outre de l'hydrocortisone, facultativement à une concentration d'environ 0,05 µM à environ 0,25 µM.

8. Procédé selon l'une quelconque des revendications 1 à 7, dans lequel le polypeptide est un anticorps, facultativement dans lequel l'anticorps est un anticorps IgG1.

9. Procédé selon la revendication 8, dans lequel l'anticorps est un anticorps anti-VEGF, anti-mésothéline, anti-PCSK9 ou anti-bêta7.

10. Procédé selon la revendication 1, dans lequel le polypeptide isolé est concentré pour fournir une composition comprenant un polypeptide à une concentration d'au moins 125 mg/ml et dans lequel la composition a une valeur standard de référence de couleur sélectionnée parmi l'une quelconque de B4-B9, BY4-BY7, Y4-Y7, GY4-GY7 et R4-R7 selon les standards de couleurs de la pharmacopée européenne, Pharmacopée européenne, 2008, 7^{e} édition, page 22.

11. Procédé selon la revendication 1, dans lequel le polypeptide isolé est concentré pour fournir une composition comprenant un polypeptide à une concentration d'au moins 150 mg/ml et dans lequel la composition a une valeur standard de référence de couleur sélectionnée parmi l'une quelconque de B4-B9, BY4-BY7, Y4-Y7, GY4-GY7 et R4-R7 selon les standards de couleurs de la pharmacopée européenne, Pharmacopée européenne, 2008, 7^{e} édition, page 22.

12. Procédé selon la revendication 1, dans lequel la couleur du polypeptide isolé est déterminée par un test sélectionné dans le groupe constitué par le test COC, le test de couleur totale et le test NIFTY.
